(19) Europäisches Patentamt
European Patent Office
Office européen des brevets

(11) EP 4 375 273 A1

(12) EUROPEAN PATENT APPLICATION
published in accordance with Art. 153(4) EPC

(43) Date of publication:
**29.05.2024 Bulletin 2024/22**

(21) Application number: **22845947.5**

(22) Date of filing: **20.07.2022**

(51) International Patent Classification (IPC):
***C07D 213/76*** *(2006.01)* ***A61K 31/44*** *(2006.01)*
***A61K 31/4436*** *(2006.01)* ***A61P 35/00*** *(2006.01)*
***B01J 31/22*** *(2006.01)* ***B01J 31/24*** *(2006.01)*
***C07B 61/00*** *(2006.01)* ***C07D 213/73*** *(2006.01)*
***C07D 333/66*** *(2006.01)* ***C07D 409/12*** *(2006.01)*

(52) Cooperative Patent Classification (CPC):
**A61K 31/44; A61K 31/4436; A61P 35/00; B01J 31/22; B01J 31/24; C07B 61/00; C07D 213/73; C07D 213/76; C07D 333/66; C07D 409/12**

(86) International application number:
**PCT/JP2022/028187**

(87) International publication number:
**WO 2023/003014 (26.01.2023 Gazette 2023/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.07.2021 JP 2021120977**

(71) Applicant: **CHUGAI SEIYAKU KABUSHIKI KAISHA**
**Tokyo 115-8543 (JP)**

(72) Inventors:
- **KUWATA Kazuaki**
  **Tokyo 115-8543 (JP)**
- **SOMEYA Hidehisa**
  **Tokyo 115-8543 (JP)**

(74) Representative: **Vossius & Partner Patentanwälte Rechtsanwälte mbB**
**Siebertstrasse 3**
**81675 München (DE)**

(54) **METHOD FOR PRODUCING ARYLAMIDE DERIVATIVE**

(57) The present disclosure provides, for example, a method for producing 2-(4-cyclopropyl-2-fluoroanilino)-3,4-difluoro-5-[[3-fluoro-2-(methylsu lfamoylamino)pyridin-4-yl]methyl]benzamide or a pharmaceutically acceptable salt thereof, or a pharmaceutically acceptable solvate of the compound or salt, the method comprising reacting (2-amino-3-fluoropyridin-4-yl)methanol with methyl chloroformate and N,N-dimethylaminopyridine in acetonitrile to give (2-amino-3-fluoropyridin-4-yl)methyl methyl carbonate. According to the present disclosure, there is provided a method for producing a specific aryl amide derivative that has RAF/MEK complex-stabilizing activity and/or MEK-inhibiting activity and is useful for the treatment or prevention of a cell proliferative disorder, particularly a cancer, the method enabling the aryl amide derivative to be produced with a small number of steps.

EP 4 375 273 A1

## Description

### Technical Field

The present disclosure relates to a method for producing an aryl amide derivative. Also, the present disclosure relates to a compound that can be used for producing an aryl amide derivative and a method for the production thereof.

### Background Art

MEK (mitogen-activated protein kinase kinase) is a serine/threonine kinase in the MAPK signaling pathway and is known to transmit signals intracellularly and to be closely involved in cell proliferation (see non patent document 1). MEK inhibitors that have been reported include PD0325901, CH4987655, trametinib, cobimetinib and selumetinib (see patent document 1 and non patent document 2), and their use either alone or in combination with RAF inhibitors has been reported to exhibit clinical effects against RAF-mutant cancers, such as BRAF-mutant malignant melanoma (see non patent documents 3 and 4).

It is also known that the clinical effects of some MEK inhibitors against RAS-mutant cancers such as RAS-mutant non-small cell lung cancer are less than satisfactory. In fact, it has been reported that selumetinib and trametinib showed poor effects in clinical trials for KRAS-mutant non-small cell lung cancer (see non patent documents 5 and 6).

CH5126766 (see patent document 2 and non patent documents 7 and 8), which is known not only as a MEK inhibitor but also as a RAF/MEK complex stabilizer, has been reported to exhibit a clinical effect against RAS-mutant non-small cell lung cancer (see non patent document 9). CH5126766 also reportedly stabilizes the RAF/MEK complex and inhibits increased MEK phosphorylation (feedback activation of the MAPK signaling pathway) (see non patent documents 7, 8 and 10). This feedback activation is thought to be one reason for the less than satisfactory clinical effects of some MEK inhibitors against RAS-mutant cancers (see non patent document 10).

### Citation List

#### Patent Literature

Patent document 1: WO 2006/011466
Patent document 2: WO 2007/091736

#### Non Patent Literature

Non patent document 1: Nat. Rev. Clin. Oncol. 2018, vol. 15, p. 709-720
Non patent document 2: Molecules. 2017, vol. 22, e1551
Non patent document 3: N. Engl. J. Med. 2012, vol. 367, p. 107-114
Non patent document 4: N. Engl. J. Med. 2012, vol. 367, p. 1694-1703
Non patent document 5: JAMA. 2017, vol. 317, no. 18, p. 1844-1853
Non patent document 6: Ann. Oncol. 2015, vol. 26, no. 5, p. 894-901
Non patent document 7: Cancer Res. 2013, vol. 73, no. 13, p. 4050-4060
Non patent document 8: Cancer Cell. 2014, vol. 25, no. 5, p. 697-710
Non patent document 9: J. Clin. Oncol. 2017, vol. 35, no. 15, suppl., 2506
Non patent document 10: Nat. Rev. Clin. Oncol. 2014, vol. 11, p. 385-400

### Summary of Invention

#### Technical Problem

Several RAF/MEK complex stabilizers or MEK inhibitors that are useful for the treatment or prevention of cell proliferative disorders (particularly cancers) are known, but at the current time the options available are still not sufficient to satisfy the varied needs of consumers.

In this situation, it has been found that a specific aryl amide derivative (a compound represented by general formula (1) below) has RAF/MEK complex-stabilizing activity and/or MEK-inhibiting activity and is useful for the treatment

or prevention of a cell proliferative disorder, particularly a cancer.

It is an object of the present disclosure to provide a method for producing such an aryl amide derivative, the method enabling the aryl amide derivative to be produced with a small number of steps.

## Solution to Problem

The present disclosure provides the methods according to (A1) to (A31) below.

(A1) A method for producing a compound represented by general formula (1) below or a pharmaceutically acceptable salt thereof, or a pharmaceutically acceptable solvate of said compound or salt, the method comprising:

(I) reacting a compound represented by general formula (2) below with a compound represented by $X_1$-$R_9$ and a base in a solvent to give a compound represented by general formula (4) below:

[Chemical Formula 1]

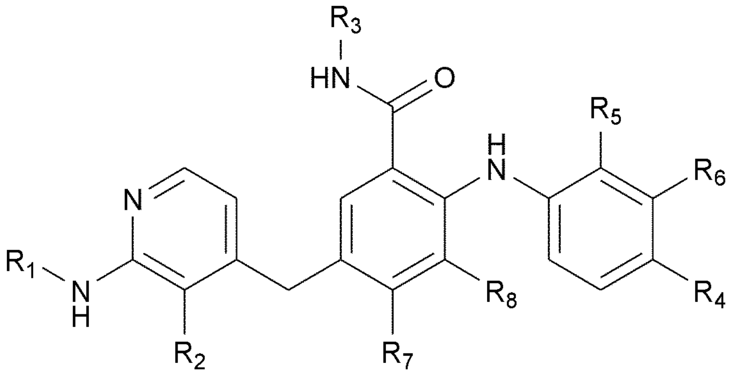

(1)

[Chemical Formula 2]

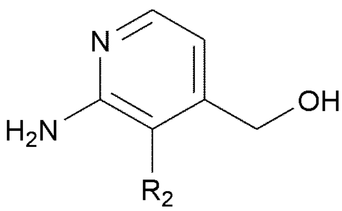

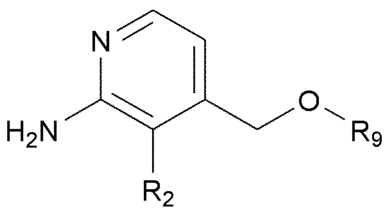

(2) (4)

wherein:

$R_1$ is -S(=O)$_2$-NH-$R_{11}$ or -S(=O)$_2$-$R_{11}$;

$R_{11}$ is a hydrogen atom, a C1-6 alkyl group (the C1-6 alkyl group being optionally substituted with a halogen atom, a hydroxy group, or a C1-6 alkoxy group), or a C3-6 cycloalkyl group (the C3-6 cycloalkyl group being optionally substituted with a C1-6 alkyl group);

$R_2$ is a hydrogen atom, a halogen atom, or a C1-6 alkyl group;

$R_3$ is a hydrogen atom, a C1-6 alkyl group (the C1-6 alkyl group being optionally substituted with a halogen atom, a hydroxy group, or a C1-6 alkoxy group), a C3-6 cycloalkyl group (the C3-6 cycloalkyl group being optionally substituted with a halogen atom or a C1-6 alkyl group), or a C1-6 alkoxy group (the C1-6 alkoxy group being optionally substituted with a halogen atom, a hydroxy group, or a C1-6 alkoxy group);

$R_4$ is a hydrogen atom, a halogen atom, a C1-6 alkyl group, a C2-7 alkenyl group, a C2-7 alkynyl group, a C3-6 cycloalkyl group, or a C1-6 alkylthio group;

$R_5$ is a halogen atom or a C1-6 alkyl group;

$R_6$ is a hydrogen atom, a halogen atom, or a C1-6 alkyl group;
$R_7$ is a hydrogen atom, a halogen atom, or a C1-6 alkyl group;
$R_8$ is a hydrogen atom, a halogen atom, or a C1-6 alkyl group;
$X_1$ is a halogen atom or -O-$R_9$;
$R_9$ is -C(=O)-$R_{12}$, -C(=O)-O-$R_{12}$, or -P(=O)(-O-$R_{12}$)$_2$; and
$R_{12}$ is a C1-6 alkyl group or an aryl group.

(A2) The method according to (A1), wherein the base used in step (I) is at least one selected from the group consisting of N,N-dimethylaminopyridine and 1-methylimidazole.
(A3) The method according to (A2), wherein the base used in step (I) is N,N-dimethylaminopyridine.
(A4) The method according to any one of (A1) to (A3), wherein the solvent used in step (I) is at least one selected from the group consisting of acetonitrile, tetrahydrofuran, 2-methyltetrahydrofuran, 4-methyltetrahydropyran, cyclopentyl methyl ether, and tert-butyl methyl ether.
(A5) The method according to (A4), wherein the solvent used in step (I) is acetonitrile.
(A6) The method according to any one of (A1) to (A5), further comprising:
(II) reacting the compound represented by general formula (4) with a compound represented by general formula (10) below in a solvent in the presence of a catalyst to give a compound represented by general formula (5) below:

[Chemical Formula 3]

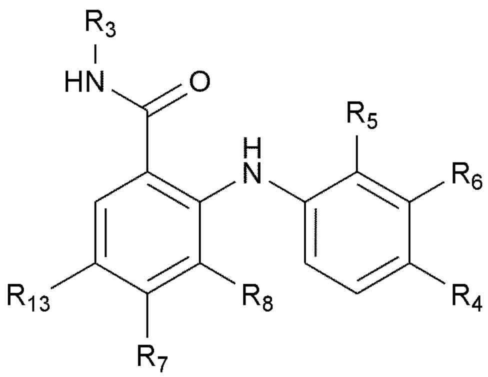

(10)

[Chemical Formula 4]

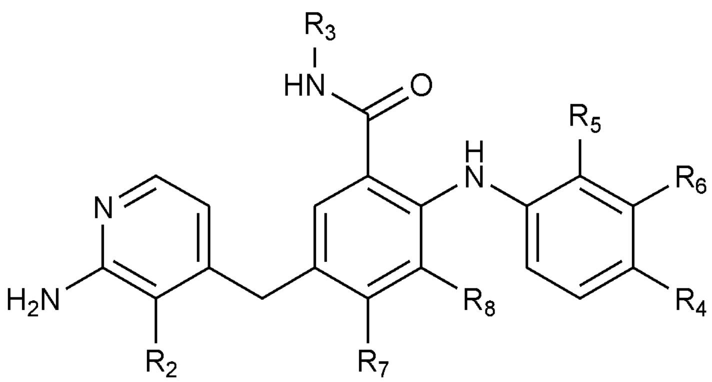

(5)

wherein:

$R_{13}$ is -B(-$OR_{14}$)(-$OR_{15}$) or -$BF_3K$;

$R_{14}$ and $R_{15}$ are each independently a hydrogen atom or a C1-6 alkyl group (the C1-6 alkyl group being optionally substituted with a C1-6 alkoxy group or an aryl group), or $R_{14}$ and $R_{15}$ form a 5- to 8-membered saturated or unsaturated ring together with the intervening oxygen and boron atoms (the ring being optionally substituted with a C1-6 alkyl group, a C1-6 alkoxy group, or an aryl group and being optionally fused with a benzene ring); and $R_2$ to $R_9$ have the same definitions as above.

(A7) The method according to (A6), wherein the catalyst used in step (II) is a palladium catalyst or a nickel catalyst.
(A8) The method according to (A7), wherein the catalyst used in step (II) is a palladium catalyst.
(A9) The method according to (A8), wherein said palladium catalyst is a combination of:

at least one selected from the group consisting of bis(allylchloropalladium), tetrakis(triphenylphosphine)palladium, tris(dibenzylideneacetone)dipalladium, and palladium(II) acetate; and
a compound represented by general formula (L1) below:

[Chemical Formula 5]

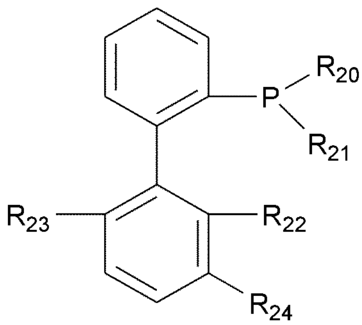

(L1)

wherein:

$R_{20}$ and $R_{21}$ are each independently a C3-6 cycloalkyl group;
$R_{22}$ is a C1-6 alkoxy group or an amino group (the amino group being optionally substituted with a C1-6 alkyl group or an aryl group);
$R_{23}$ is a hydrogen atom or a C1-6 alkoxy group; and
$R_{24}$ is a hydrogen atom or $-S(=O)_2-O-Na$.

(A10) The method according to (A9), wherein:

$R_{20}$ and $R_{21}$ are a cyclohexyl group;
$R_{22}$ is a methoxy group, an isopropoxy group, or an N,N-dimethylamino group;
$R_{23}$ is a hydrogen atom, a methoxy group, or an isopropoxy group; and
$R_{24}$ is a hydrogen atom.

(A11) The method according to (A10), wherein said palladium catalyst is a combination of:

at least one selected from the group consisting of bis(allylchloropalladium), tetrakis(triphenylphosphine)palladium, tris(dibenzylideneacetone)dipalladium, and palladium(II) acetate; and
at least one selected from the group consisting of 2',6'-dimethoxy-2-(dicyclohexylphosphino)biphenyl, 2-dicyclohexylphosphino-2',6' -diisopropoxybiphenyl, and 2-dicyclohexylphosphino-2'-(N,N-dimethylamino)biphenyl.

(A12) The method according to (A11), wherein said palladium catalyst is a combination of bis(allylchloropalladium) and at least one selected from the group consisting of 2',6'-dimethoxy-2-(dicyclohexylphosphino)biphenyl, 2-dicyclohexylphosphino-2',6' -diisopropoxybiphenyl, and 2-dicyclohexylphosphino-2'-(N,N-dimethylamino)biphenyl.
(A13) The method according to (A8), wherein said palladium catalyst is a compound represented by general formula (L2) below:

[Chemical Formula 6]

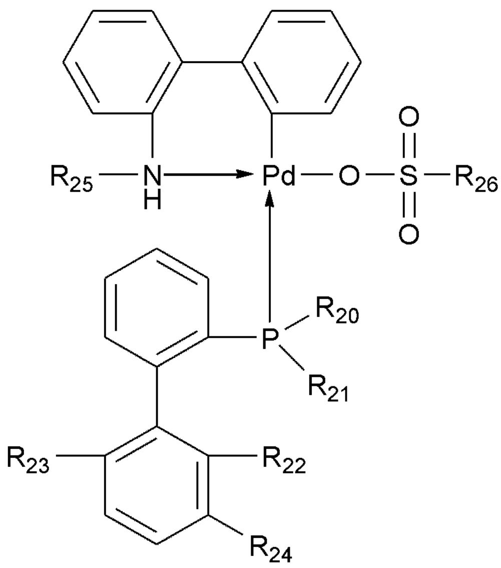

(L2)

wherein:

$R_{20}$ and $R_{21}$ are each independently a C3-6 cycloalkyl group;
$R_{22}$ is a C1-6 alkoxy group or an amino group (the amino group being optionally substituted with a C1-6 alkyl group or an aryl group);
$R_{23}$ is a hydrogen atom or a C1-6 alkoxy group;
$R_{24}$ is a hydrogen atom;
$R_{25}$ is a hydrogen atom or a C1-6 alkyl group;
$R_{26}$ is a C1-6 alkyl group; and
each arrow represents a coordinate bond.

(A14) The method according to (A13), wherein:

$R_{20}$ and $R_{21}$ are a cyclohexyl group;
$R_{22}$ is a methoxy group, an isopropoxy group, or an N,N-dimethylamino group;
$R_{23}$ is a hydrogen atom, a methoxy group, or an isopropoxy group;
$R_{25}$ is a hydrogen atom or a methyl group; and
$R_{26}$ is a methyl group.

(A15) The method according to (A14), wherein said palladium catalyst is at least one selected from the group consisting of:

(2-dicyclohexylphosphino-2' ,6' -dimethoxybiphenyl) [2-(2'-amin o-1,1'-biphenyl)]palladium(II) methanesulfonate,
2-dicyclohexylphosphino-2-(N,N-dimethylamino)biphenyl(2'-a mino-1,1'-biphenyl-2-yl)palladium(II) methanesulfonate, and
(2-dicyclohexylphosphino-2',6'-diisopropoxy-1,1'-biphenyl)[2-( 2'-amino-1,1'-biphenyl)]palladium(II) methanesulfonate.

(A16) The method according to (A15), wherein said palladium catalyst is (2-dicyclohexylphosphino-2',6'-diisopropoxy-1,1'-biphenyl)[2-(2'-ami no-1,1 '-biphenyl)]palladium(II) methanesulfonate.
(A17) The method according to (A7), wherein the catalyst used in step (II) is a nickel catalyst.
(A18) The method according to (A17), wherein said nickel catalyst is a combination of at least one selected from the group consisting of bis(1,5-cyclooctadiene)nickel and nickel(II) chloride and at least one selected from the group consisting of tricyclohexylphosphine, 1,1'-bis(diphenylphosphino)ferrocene, and 1,3-bis(diphenylphosphino)propane.

(A19) The method according to (A17), wherein said nickel catalyst is at least one selected from the group consisting of:

dichlorobis(tricyclohexylphosphine)nickel(II),
dichloro[1,1'-bis(diphenylphosphino)ferrocene]nickel(II), and
dichloro[1,3-bis(diphenylphosphino)propane]nickel(II).

(A20) The method according to (A6), wherein the catalyst used in step (II) is at least one selected from the group consisting of:

a combination of bis(allylchloropalladium) and 2',6'-dimethoxy-2-(dicyclohexylphosphino)biphenyl;
a combination of bis(allylchloropalladium) and 2-dicyclohexylphosphino-2',6' -diisopropoxybiphenyl;
a combination of bis(allylchloropalladium) and 2-dicyclohexylphosphino-2'-(N,N-dimethylamino)biphenyl;
(2-dicyclohexylphosphino-2' ,6' -dimethoxybiphenyl) [2-(2'-amin o-1,1'-biphenyl)]palladium(II) methanesulfonate;
2-dicyclohexylphosphino-2-(N,N-dimethylamino)biphenyl(2'-a mino-1,1'-biphenyl-2-yl)palladium(II) methanesulfonate; and
(2-dicyclohexylphosphino-2',6'-diisopropoxy-1,1'-biphenyl)[2-( 2'-amino-1,1'-biphenyl)]palladium(II) methanesulfonate.

(A21) The method according to (A20), wherein the catalyst used in step (II) is (2-dicyclohexylphosphino-2',6'-diisopropoxy-1,1'-biphenyl)[2-(2'-ami no-1,1'-biphenyl)]palladium(II) methanesulfonate.

(A22) The method according to any one of (A6) to (A21), wherein the solvent used in step (II) comprises a C1-6 alcohol.

(A23) The method according to (A22), wherein the solvent used in step (II) comprises a C2-3 alcohol.

(A24) The method according to (A23), wherein the solvent used in step (II) comprises ethanol.

(A25) The method according to any one of (A6) to (A24), further comprising:

(III) reacting the compound represented by general formula (5) with a compound represented by $X_2$-S(=O)$_2$-NH-R$1_1$ or $X_2$-S(=O)$_2$-$R_{11}$ to give a compound represented by general formula (1) or a salt thereof or a solvate of said compound or salt;
wherein $X_2$ is a halogen atom and $R_{11}$ has the same definition as above.

(A26) The method according to any one of (A1) to (A25), wherein:

$R_9$ is -C(=O)-O-$R_{12}$; and
$R_{12}$ is a C1-6 alkyl group or an aryl group.

(A27) The method according to (A26), wherein $R_2$ is a halogen atom.

(A28) The method according to (A27), wherein:

$R_2$ is a fluorine atom;
$R_9$ is -C(=O)-O-$CH_3$; and
$X_1$ is a chlorine atom.

(A29) The method according to any one of (A1) to (A28), wherein:

$R_2$ is a fluorine atom;
$R_1$ is -S(=O)$_2$-NH-$R_{11}$;
$R_{11}$ is a C1-4 alkyl group;
$R_3$ is a hydrogen atom or a cyclopropyl group;
$R_5$ is a fluorine atom;
$R_6$ is a hydrogen atom;
$R_4$ is an iodine atom or a cyclopropyl group;
$R_7$ is a fluorine atom;
$R_8$ is a fluorine atom; and
$X_1$ is a chlorine atom.

(A30) The method according to any one of (A1) to (A29), wherein the compound represented by general formula

(1) is 2-(4-cyclopropyl-2-fluoroanilino)-3,4-difluoro-5-[[3-fluoro-2-(methylsu lfamoylamino)pyridin-4-yl]methyl]benzamide.

(A31) The method according to any one of (A1) to (A30), being a method for producing a sodium salt of the compound represented by general formula (1).

The present disclosure provides the methods according to (B1) to (B26) below.

(B1) A method for producing a compound represented by general formula (1) below or a pharmaceutically acceptable salt thereof, or a pharmaceutically acceptable solvate of said compound or salt, the method comprising:

(II) reacting a compound represented by general formula (4) below with a compound represented by general formula (10) below in a solvent in the presence of a catalyst to give a compound represented by general formula (5) below:

[Chemical Formula 7]

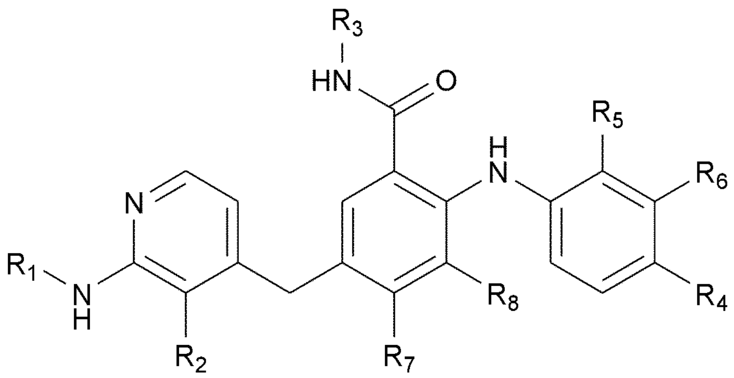

(1)

[Chemical Formula 8]

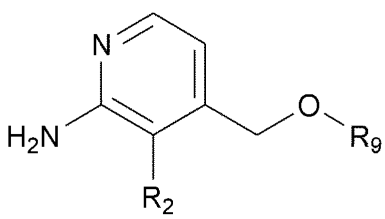

(4)

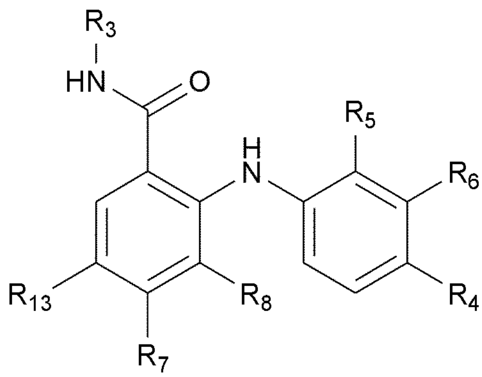

(10)

[Chemical Formula 9]

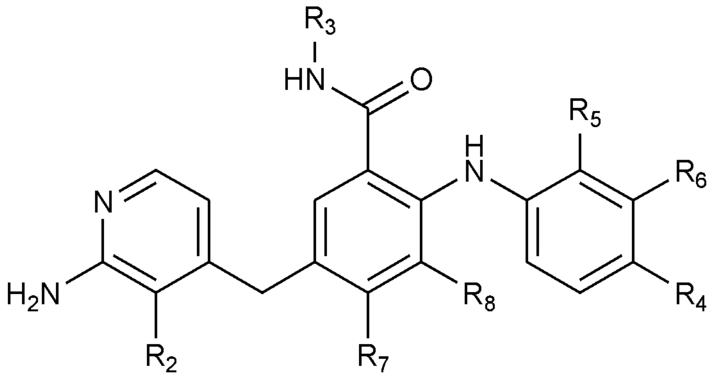

(5)

wherein:

$R_1$ is -S(=O)$_2$-NH-$R_{11}$ or -S(=O)$_2$-$R_{11}$;
$R_{11}$ is a hydrogen atom, a C1-6 alkyl group (the C1-6 alkyl group being optionally substituted with a halogen atom, a hydroxy group, or a C1-6 alkoxy group), or a C3-6 cycloalkyl group (the C3-6 cycloalkyl group being optionally substituted with a C1-6 alkyl group);
$R_2$ is a hydrogen atom, a halogen atom, or a C1-6 alkyl group;
$R_3$ is a hydrogen atom, a C1-6 alkyl group (the C1-6 alkyl group being optionally substituted with a halogen atom, a hydroxy group, or a C1-6 alkoxy group), a C3-6 cycloalkyl group (the C3-6 cycloalkyl group being optionally substituted with a halogen atom or a C1-6 alkyl group), or a C1-6 alkoxy group (the C1-6 alkoxy group being optionally substituted with a halogen atom, a hydroxy group, or a C1-6 alkoxy group);
$R_4$ is a hydrogen atom, a halogen atom, a C1-6 alkyl group, a C2-7 alkenyl group, a C2-7 alkynyl group, a C3-6 cycloalkyl group, or a C1-6 alkylthio group;
$R_5$ is a halogen atom or a C1-6 alkyl group;
$R_6$ is a hydrogen atom, a halogen atom, or a C1-6 alkyl group;
$R_7$ is a hydrogen atom, a halogen atom, or a C1-6 alkyl group;
$R_8$ is a hydrogen atom, a halogen atom, or a C1-6 alkyl group;
$R_9$ is -C(=O)-$R_{12}$, -C(=O)-O-$R_{12}$, or -P(=O)(-O-$R_{12}$)$_2$;
$R_{12}$ is a C1-6 alkyl group or an aryl group;
$R_{13}$ is -B(-O$R_{14}$)(-O$R_{15}$) or -$BF_3K$; and
$R_{14}$ and $R_{15}$ are each independently a hydrogen atom or a C1-6 alkyl group (the C1-6 alkyl group being optionally substituted with a C1-6 alkoxy group or an aryl group), or $R_{14}$ and $R_{15}$ form a 5- to 8-membered saturated or unsaturated ring together with the intervening oxygen and boron atoms (the ring being optionally substituted with a C1-6 alkyl group, a C1-6 alkoxy group, or an aryl group and being optionally fused with a benzene ring).

(B2) The method according to (B1), wherein the catalyst used in step (II) is a palladium catalyst or a nickel catalyst.
(B3) The method according to (B2), wherein the catalyst used in step (II) is a palladium catalyst.
(B4) The method according to (B3), wherein said palladium catalyst is a combination of:

at least one selected from the group consisting of bis(allylchloropalladium), tetrakis(triphenylphosphine)palladium, tris(dibenzylideneacetone)dipalladium, and palladium(II) acetate; and
a compound represented by general formula (L1) below:

[Chemical Formula 10]

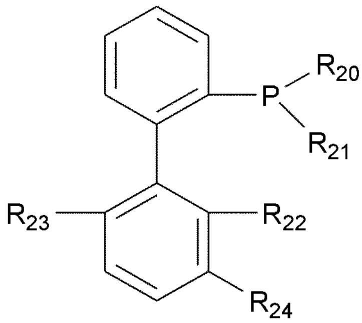

(L1)

wherein:

$R_{20}$ and $R_{21}$ are each independently a C3-6 cycloalkyl group;
$R_{22}$ is a C1-6 alkoxy group or an amino group (the amino group being optionally substituted with a C1-6 alkyl group or an aryl group);
$R_{23}$ is a hydrogen atom or a C1-6 alkoxy group; and
$R_{24}$ is a hydrogen atom or $-S(=O)_2-O-Na$.

(B5) The method according to (B4), wherein:

$R_{20}$ and $R_{21}$ are a cyclohexyl group;
$R_{22}$ is a methoxy group, an isopropoxy group, or an N,N-dimethylamino group;
$R_{23}$ is a hydrogen atom, a methoxy group, or an isopropoxy group; and
$R_{24}$ is a hydrogen atom.

(B6) The method according to (B5), wherein said palladium catalyst is a combination of:

at least one selected from the group consisting of bis(allylchloropalladium), tetrakis(triphenylphosphine)palladium, tris(dibenzylideneacetone)dipalladium, and palladium(II) acetate; and
at least one selected from the group consisting of 2',6'-dimethoxy-2-(dicyclohexylphosphino)biphenyl, 2-dicyclohexylphosphino-2',6' -diisopropoxybiphenyl, and 2-dicyclohexylphosphino-2'-(N,N-dimethylamino)biphenyl.

(B7) The method according to (B6), wherein said palladium catalyst is a combination of bis(allylchloropalladium) and at least one selected from the group consisting of 2',6'-dimethoxy-2-(dicyclohexylphosphino)biphenyl, 2-dicyclohexylphosphino-2',6' -diisopropoxybiphenyl, and 2-dicyclohexylphosphino-2'-(N,N-dimethylamino)biphenyl.
(B8) The method according to (B3), wherein said palladium catalyst is a compound represented by general formula (L2) below:

[Chemical Formula 11]

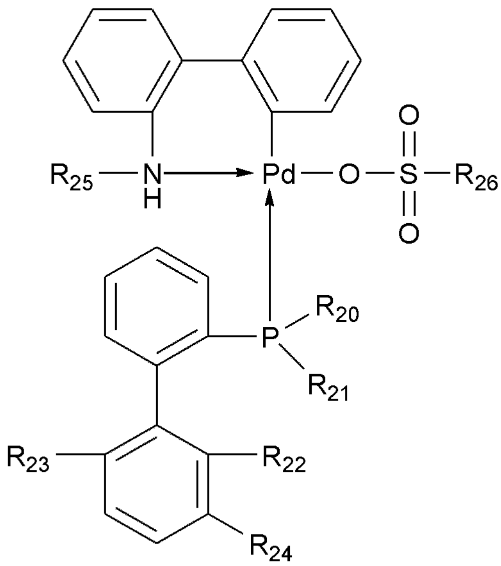

(L2)

wherein:

$R_{20}$ and $R_{21}$ are each independently a C3-6 cycloalkyl group;
$R_{22}$ is a C1-6 alkoxy group or an amino group (the amino group being optionally substituted with a C1-6 alkyl group or an aryl group);
$R_{23}$ is a hydrogen atom or a C1-6 alkoxy group;
$R_{24}$ is a hydrogen atom;
$R_{25}$ is a hydrogen atom or a C1-6 alkyl group;
$R_{26}$ is a C1-6 alkyl group; and
each arrow represents a coordinate bond.

(B9) The method according to (B8), wherein:

$R_{20}$ and $R_{21}$ are a cyclohexyl group;
$R_{22}$ is a methoxy group, an isopropoxy group, or an N,N-dimethylamino group;
$R_{23}$ is a hydrogen atom, a methoxy group, or an isopropoxy group;
$R_{25}$ is a hydrogen atom or a methyl group; and
$R_{26}$ is a methyl group.

(B10) The method according to (B9), wherein said palladium catalyst is at least one selected from the group consisting of:

(2-dicyclohexylphosphino-2' ,6' -dimethoxybiphenyl) [2-(2'-amin o-1,1'-biphenyl)]palladium(II) methanesulfonate,
2-dicyclohexylphosphino-2-(N,N-dimethylamino)biphenyl(2'-a mino-1,1'-biphenyl-2-yl)palladium(II) methanesulfonate, and
(2-dicyclohexylphosphino-2',6'-diisopropoxy-1,1'-biphenyl)[2-( 2'-amino-1,1'-biphenyl)]palladium(II) methanesulfonate.

(B11) The method according to (B10), wherein said palladium catalyst is (2-dicyclohexylphosphino-2',6'-diisopropoxy-1,1'-biphenyl)[2-(2'-ami no-1,1'-biphenyl)]palladium(II) methanesulfonate.
(B12) The method according to (B2), wherein the catalyst used in step (II) is a nickel catalyst.
(B13) The method according to (B12), wherein said nickel catalyst is a combination of at least one selected from the group consisting of bis(1,5-cyclooctadiene)nickel and nickel (II) chloride and at least one selected from the group consisting of tricyclohexylphosphine, 1,1'-bis(diphenylphosphino)ferrocene, and 1,3-bis(diphenylphosphino)propane.
(B14) The method according to (B12), wherein said nickel catalyst is at least one selected from the group consisting of:

dichlorobis(tricyclohexylphosphine)nickel (II),
dichloro[1,1'-bis(diphenylphosphino)ferrocene]nickel (II), and
dichloro[1,3-bis(diphenylphosphino)propane]nickel (II).

(B15) The method according to (B1), wherein the catalyst used in step (II) is at least one selected from the group consisting of:

a combination of bis(allylchloropalladium) and 2',6'-dimethoxy-2-(dicyclohexylphosphino)biphenyl;
a combination of bis(allylchloropalladium) and 2-dicyclohexylphosphino-2',6' -diisopropoxybiphenyl;
a combination of bis(allylchloropalladium) and 2-dicyclohexylphosphino-2'-(N,N-dimethylamino)biphenyl;
(2-dicyclohexylphosphino-2' ,6' -dimethoxybiphenyl) [2-(2'-amin o-1,1'-biphenyl)]palladium(II) methanesulfonate;
2-dicyclohexylphosphino-2-(N,N-dimethylamino)biphenyl(2'-a mino-1,1'-biphenyl-2-yl)palladium(II) methanesulfonate; and
(2-dicyclohexylphosphino-2',6'-diisopropoxy-1,1'-biphenyl)[2-( 2'-amino-1,1'-biphenyl)]palladium(II) methanesulfonate.

(B16) The method according to (B15), wherein the catalyst used in step (II) is (2-dicyclohexylphosphino-2',6'-diisopropoxy-1,1'-biphenyl)[2-(2'-ami no-1,1'-biphenyl)]palladium(II) methanesulfonate.
(B17) The method according to any one of (B 1) to (B 16), wherein the solvent used in step (II) comprises a C1-6 alcohol.
(B18) The method according to (B17), wherein the solvent used in step (II) comprises a C2-3 alcohol.
(B19) The method according to (B18), wherein the solvent used in step (II) comprises ethanol.
(B20) The method according to any one of (B1) to (B19), further comprising:

(III) reacting the compound represented by general formula (5) with a compound represented by $X_2$-S(=O)$_2$-NH-R1$_1$ or $X_2$-S(=O)$_2$-$R_{11}$ to give a compound represented by general formula (1) or a salt thereof or a solvate of said compound or salt;
wherein $X_2$ is a halogen atom and $R_{11}$ has the same definition as above.

(B21) The method according to any one of (B1) to (B20), wherein:

$R_9$ is -C(=O)-O-$R_{12}$; and
$R_{12}$ is a C1-6 alkyl group or an aryl group.

(B22) The method according to (B21), wherein $R_2$ is a halogen atom.
(B23) The method according to (B22), wherein:

$R_2$ is a fluorine atom; and
$R_9$ is -C(=O)-O-$CH_3$.

(B24) The method according to any one of (B1) to (B23), wherein:

$R_2$ is a fluorine atom;
$R_1$ is -S(=O)$_2$-NH-$R_{11}$;
$R_{11}$ is a C1-4 alkyl group;
$R_3$ is a hydrogen atom or a cyclopropyl group;
$R_5$ is a fluorine atom;
$R_6$ is a hydrogen atom;
$R_4$ is an iodine atom or a cyclopropyl group;
$R_7$ is a fluorine atom; and
$R_8$ is a fluorine atom.

(B25) The method according to any one of (B1) to (B24), wherein the compound represented by general formula (1) is 2-(4-cyclopropyl-2-fluoroanilino)-3,4-difluoro-5-[[3-fluoro-2-(methylsu lfamoylamino)pyridin-4-yl]methyl]benzamide.
(B26) The method according to any one of (B1) to (B25), being a method for producing a sodium salt of the compound represented by general formula (1).

The compound of general formula (5) can be produced using an amino-protected compound as described in, for example, Synthetic Example 4 (4-2) below, but such a method requires deprotection of the amino group. In contrast, the methods according to (A1) to (A31) and (B1) to (B26) enable the compound of general formula (5) to be produced using an amino-unprotected compound without protecting the amino group, and thus enable the aryl amide derivative of general formula (1) to be produced with a smaller number of steps.

Inventions provided by the present disclosure include the method according to (C1) below, for example.

(C1) The method according to (A31) or (B26), the method being a method for producing a sodium salt of 2-(4-cyclopropyl-2-fluoroanilino)-3,4-difluoro-5-[[3-fluoro-2-(methylsu lfamoylamino)pyridin-4-yl]methyl]benzamide, wherein a compound represented by formula (X) below or a sodium salt thereof is generated in the method, and the amount of the compound of formula (X) or sodium salt thereof that is generated is 3.0% w/w or less, 2.0% w/w or less, 1.0% w/w or less, 0.8% w/w or less, 0.5% w/w or less, or 0.3% w/w or less with respect to the weight of the sodium salt of 2-(4-cyclopropyl-2-fluoroanilino)-3,4-difluoro-5-[[3-fluoro-2-(methylsu lfamoylamino)pyridin-4-yl]methyl]benzamide that is generated.

[Chemical Formula 12]

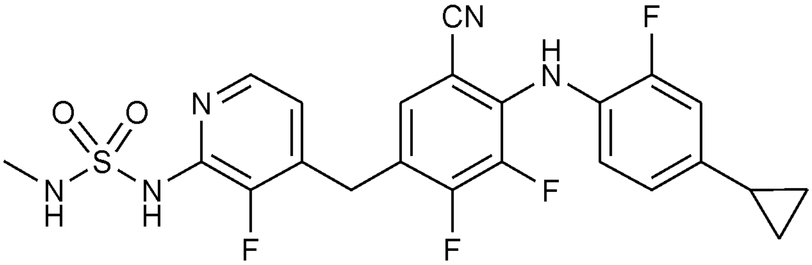

(X)

Inventions provided by the present disclosure also include the composition according to (D1) below, for example. (D1) A composition comprising a sodium salt of 2-(4-cyclopropyl-2-fluoroanilino)-3,4-difluoro-5-[[3-fluoro-2-(methylsu lfamoylamino)pyridin-4-yl]methyl]benzamide and a compound represented by formula (X) below or a sodium salt thereof, wherein the amount of the compound of formula (X) or sodium salt thereof that is contained in the composition is 3.0% w/w or less, 2.0% w/w or less, 1.0% w/w or less, 0.8% w/w or less, 0.5% w/w or less, or 0.3% w/w or less with respect to the weight of the sodium salt of 2-(4-cyclopropyl-2-fluoroanilino)-3,4-difluoro-5-[[3-fluoro-2-(methylsu lfamoylamino)pyridin-4-yl]methyl]benzamide that is contained in the composition.

[Chemical Formula 13]

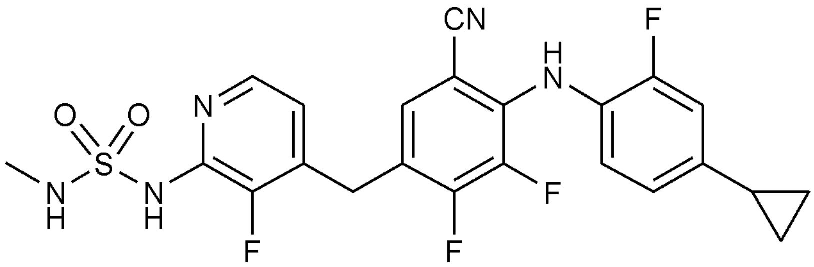

(X)

Inventions provided by the present disclosure also include the methods according to (E1) to (E7) below, for example.

(E1) A method for producing a compound represented by general formula (4) below, the method comprising:

(I) reacting a compound represented by general formula (2) below with a compound represented by $X_1$-$R_9$ and a base in a solvent to give a compound represented by general formula (4):

[Chemical Formula 14]

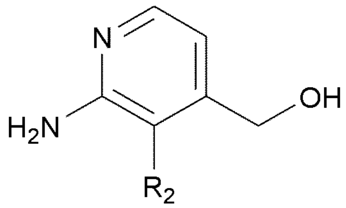

(2)

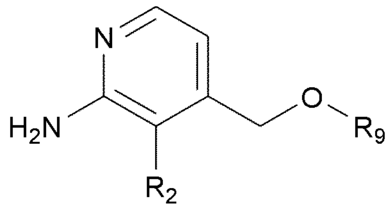

(4)

wherein:

$R_2$ is a hydrogen atom, a halogen atom, or a C1-6 alkyl group;
$X_1$ is a halogen atom or $-O-R_9$;
$R_9$ is $-C(=O)-R_{12}$, $-C(=O)-O-R_{12}$, or $-P(=O)(-O-R_{12})_2$; and
$R_{12}$ is a C1-6 alkyl group or an aryl group.

(E2) The method according to (E1), wherein the base used in step (I) is at least one selected from the group consisting of N,N-dimethylaminopyridine and 1-methylimidazole.
(E3) The method according to (E2), wherein the base used in step (I) is N,N-dimethylaminopyridine.
(E4) The method according to any one of (E1) to (E3), wherein the solvent used in step (I) is at least one selected from the group consisting of acetonitrile, tetrahydrofuran, 2-methyltetrahydrofuran, 4-methyltetrahydropyran, cyclopentyl methyl ether, and tert-butyl methyl ether.
(E5) The method according to (E4), wherein the solvent used in step (I) is acetonitrile.
(E6) The method according to any one of (E1) to (E5), wherein:

$R_9$ is $-C(=O)-O-R_{12}$; and
$R_{12}$ is a C1-6 alkyl group or an aryl group.

(E7) The method according to (E6), wherein $R_2$ is a halogen atom.
(E8) The method according to (E7), wherein:

$R_2$ is a fluorine atom;
$R_9$ is $-C(=O)-O-CH_3$; and
$X_1$ is a chlorine atom.

Inventions provided by the present disclosure also include the compound according to (F1) below, for example.
(F1) (2-Amino-3-fluoropyridin-4-yl)methyl methyl carbonate.

**Advantageous Effects of Invention**

According to the present disclosure, there is provided a method for producing a specific aryl amide derivative that has RAF/MEK complex-stabilizing activity and/or MEK-inhibiting activity and is useful for the treatment or prevention of a cell proliferative disorder, particularly a cancer, the method enabling the aryl amide derivative to be produced with a small number of steps.

**Brief Description of Drawings**

Fig. 1 shows a powder X-ray diffraction pattern of sample 1a (Form I).
Fig. 2 shows a powder X-ray diffraction pattern of sample 1b (Form I).
Fig. 3 shows a powder X-ray diffraction pattern of sample 1c.
Fig. 4 is sensorgrams showing change over time in the amount of binding of MEK1 added onto a RAF1-immobilized sensor chip surface together with a test compound (ref-2, ref-3, ref-4, A-1, ref-1, ref-5 or B-1).

Fig. 5 is sensorgrams showing change over time in the amount of binding of MEK1 added onto a RAF1-immobilized sensor chip surface together with a test compound (A-2, A-25, J-1, E-1, M-1, N-1 or H-3).
Fig. 6 is sensorgrams showing change over time in the amount of binding of MEK1 added onto a RAF1-immobilized sensor chip surface together with a test compound (I-1, H-4, L-1, P-1, E-7 or A-27).
Fig. 7 is sensorgrams showing change over time in the amount of binding of MEK1 added onto a RAF1-immobilized sensor chip surface together with a test compound (A-33, A-18, N-2, A-20, A-8, E-13 or H-1).
Fig. 8 is electrophoresis images showing the results of Western blotting of proteins (p-MEK, MEK, p-ERK, and ERK) extracted from A549 cells cultured in the presence of a test compound (ref-5 or compound A-1).
Fig. 9 is a graph showing change over time in tumor volume (mean $\pm$ SD) in nude mice subcutaneously transplanted with Calu-6 human lung cancer cells.

**Description of Embodiments**

Exemplary embodiments of the present invention are described below.

As used in the present disclosure, "halogen atom" refers to a fluorine atom, a chlorine atom, a bromine atom, or an iodine atom.

As used in the present disclosure, "C1-6 alkyl group" means a straight- or branched-chain alkyl group of 1 to 6 carbon atoms. Examples include methyl, ethyl, *n*-propyl, isopropyl, *n*-butyl, sec-butyl, *tert*-butyl, 1-methylpropyl, *n*-pentyl, 1-methylbutyl, 2-methylbutyl, 3-methylbutyl, 1,1-dimethylpropyl, 2,2-dimethylpropyl, 1,2-dimethylpropyl, 1-ethylpropyl, *n*-hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl groups.

As used in the present disclosure, "C2-7 alkenyl group" means a straight- or branched-chain alkenyl group of 2 to 7 carbon atoms. Examples include vinyl, allyl, 1-butenyl, 2-butenyl, 3-butenyl, pentenyl, pentadienyl, hexenyl, hexadienyl, heptenyl, heptadienyl, and heptatrienyl groups.

As used in the present disclosure, "C2-7 alkynyl group" means a straight- or branched-chain alkynyl group of 2 to 7 carbon atoms. Examples include ethynyl, 1-propynyl, 2-propynyl, 1-butynyl, 2-butynyl, 3-butynyl, pentynyl, pentadiynyl, hexynyl, hexadiynyl, heptynyl, heptadiynyl, and heptatriynyl groups.

As used in the present disclosure, "C1-6 alkoxy group" means an alkyloxy group having a straight- or branched-chain alkyl group of 1 to 6 carbon atoms. Examples include methoxy, ethoxy, *n*-propoxy, isopropoxy, *n*-butoxy, *sec*-butoxy, *tert*-butoxy, *n*-pentoxy, and *n*-hexoxy groups.

As used in the present disclosure, "C1-6 alkylthio group" means an alkylthio group having a straight- or branched-chain alkyl group of 1 to 6 carbon atoms. Examples include methylthio, ethylthio, *n*-propylthio, isopropylthio, *n*-butylthio, *sec*-butylthio, *tert*-butylthio, *n*-pentylthio, and *n*-hexylthio groups.

As used in the present disclosure, "C3-6 cycloalkyl group" means a monocyclic cyclic alkyl group of 3 to 6 atoms composing a ring. Examples include cyclopropyl, cyclobutyl, cyclopentyl, and cyclohexyl groups.

As used in the present disclosure, "aryl group" means an aromatic hydrocarbon group of 6 to 10 carbon atoms. Examples include phenyl, 1-naphthyl, and 2-naphthyl groups.

In the present disclosure, examples of pharmaceutically acceptable salts include: inorganic acid salts such as hydrochlorides, hydrobromides, hydroiodides, sulfates, and phosphates; sulfonates such as methanesulfonates, benzenesulfonates, and toluenesulfonates; carboxylates such as formates, acetates, oxalates, maleates, fumarates, citrates, malates, succinates, malonates, gluconates, mandelates, benzoates, salicylates, fluoroacetates, trifluoroacetates, tartrates, propionates, and glutarates; alkali metal salts such as lithium salts, sodium salts, potassium salts, cesium salts, and rubidium salts; alkaline earth metal salts such as magnesium salts and calcium salts; and ammonium salts such as ammonium salts, alkylammonium salts, dialkylammonium salts, trialkylammonium salts, and tetraalkylammonium salts. Among them, alkali metal salts such as lithium salts, sodium salts, potassium salts, cesium salts and rubidium salts are preferred, and sodium salts and potassium salts are more preferred.

In the present disclosure, a "pharmaceutically acceptable solvate" is a solvate with, for example, water, an alcohol (e.g., methanol, ethanol, 1-propanol, or 2-propanol), acetone, dimethylformamide, or dimethylacetamide. The solvate may be a solvate with a single solvent or may be a solvate with multiple solvents. Hydrates are examples of preferred solvates.

A first aspect of the present disclosure provides a method for producing a compound represented by general formula (1) below or a pharmaceutically acceptable salt thereof, or a pharmaceutically acceptable solvate of said compound or salt, the method comprising step (I) below.

A second aspect of the present disclosure provides a method for producing a compound represented by general formula (1) below or a pharmaceutically acceptable salt thereof, or a pharmaceutically acceptable solvate of said compound or salt, the method comprising step (II) below.

[Chemical Formula 15]

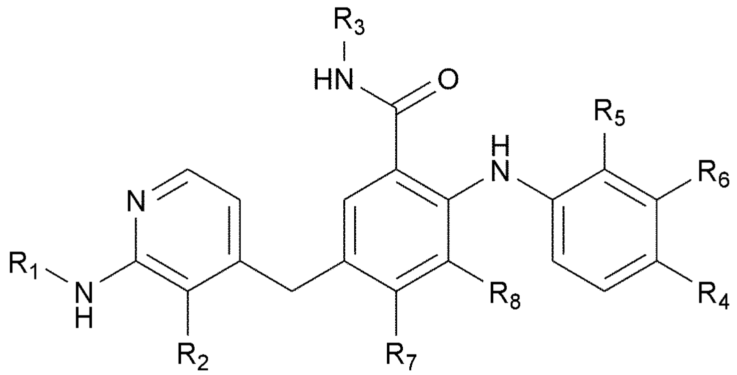

(1)

In the formula:

$R_1$ is $-S(=O)_2-NH-R_{11}$ or $-S(=O)_2-R_{11}$;
$R_{11}$ is a hydrogen atom, a C1-6 alkyl group (the C1-6 alkyl group being optionally substituted with a halogen atom, a hydroxy group, or a C1-6 alkoxy group), or a C3-6 cycloalkyl group (the C3-6 cycloalkyl group being optionally substituted with a C1-6 alkyl group);
$R_2$ is a hydrogen atom, a halogen atom, or a C1-6 alkyl group;
$R_3$ is a hydrogen atom, a C1-6 alkyl group (the C1-6 alkyl group being optionally substituted with a halogen atom, a hydroxy group, or a C1-6 alkoxy group), a C3-6 cycloalkyl group (the C3-6 cycloalkyl group being optionally substituted with a halogen atom or a C1-6 alkyl group), or a C1-6 alkoxy group (the C1-6 alkoxy group being optionally substituted with a halogen atom, a hydroxy group, or a C1-6 alkoxy group);
$R_4$ is a hydrogen atom, a halogen atom, a C1-6 alkyl group, a C2-7 alkenyl group, a C2-7 alkynyl group, a C3-6 cycloalkyl group, or a C1-6 alkylthio group;
$R_5$ is a halogen atom or a C1-6 alkyl group;
$R_6$ is a hydrogen atom, a halogen atom, or a C1-6 alkyl group;
$R_7$ is a hydrogen atom, a halogen atom, or a C1-6 alkyl group; and
$R_8$ is a hydrogen atom, a halogen atom, or a C1-6 alkyl group.

In one preferred embodiment, the method of the first aspect of the present disclosure further comprises step (II) below.

In one preferred embodiment, the method of the first aspect of the present disclosure further comprises steps (II) and (III) below.

In one preferred embodiment, the method of the second aspect of the present disclosure further comprises step (III) below.

A third aspect of the present disclosure provides a method for producing a compound represented by general formula (4) below, the method comprising step (I) below.

Step (I):

Reacting a compound represented by general formula (2) below with a compound represented by $X_1$-$R_9$ and a base in a solvent to give a compound represented by general formula (4) below:

[Chemical Formula 16]

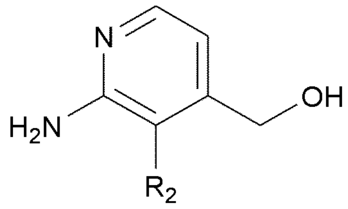

(2)

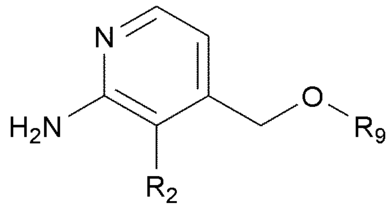

(4)

In the formulas:

$R_2$ has the same definition as above;
$X_1$ is a halogen atom or $-O-R_9$;
$R_9$ is $-C(=O)-R_{12}$, $-C(=O)-O-R_{12}$, or $-P(=O)(-O-R_{12})_2$; and
$R_{12}$ is a C1-6 alkyl group or an aryl group.

Step (II):

Reacting the compound represented by general formula (4) with a compound represented by general formula (10) below in a solvent in the presence of a catalyst to give a compound represented by general formula (5) below:

[Chemical Formula 17]

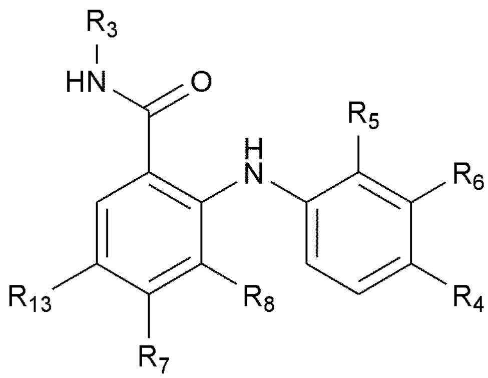

(10)

[Chemical Formula 18]

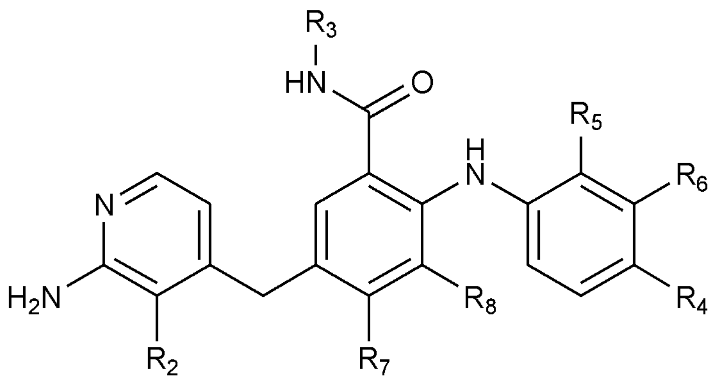

(5)

In the formulas:

$R_{13}$ is -B(-$OR_{14}$)(-$OR_{15}$) or -$BF_3K$;
$R_{14}$ and $R_{15}$ are each independently a hydrogen atom or a C1-6 alkyl group (the C1-6 alkyl group being optionally substituted with a C1-6 alkoxy group or an aryl group), or $R_{14}$ and $R_{15}$ form a 5- to 8-membered saturated or unsaturated ring together with the intervening oxygen and boron atoms (the ring being optionally substituted with a C1-6 alkyl group, a C1-6 alkoxy group, or an aryl group and being optionally fused with a benzene ring); and
$R_2$ to $R_9$ have the same definitions as above.

Step (III):

Reacting the compound represented by general formula (5) with a compound represented by $X_2$-S(=O)$_2$-NH-$R1_1$ or $X_2$-S(=O)$_2$-$R_{11}$ to give a compound represented by general formula (1) or a salt thereof or a solvate of said compound or salt.

In the formulas, $X_2$ is a halogen atom and Rn has the same definition as above.

$R_1$ is preferably -S(=O)$_2$-NH-$R_{11}$.
$R_{11}$ is preferably a C1-6 alkyl group (the C1-6 alkyl group being optionally substituted with a halogen atom or a C1-6 alkoxy group) or a C3-6 cycloalkyl group (the C3-6 cycloalkyl group being optionally substituted with a C1-6 alkyl group), more preferably a C1-4 alkyl group (the C1-4 alkyl group being optionally substituted with a fluorine atom or a C1-4 alkoxy group) or a cyclopropyl group (the cyclopropyl group being optionally substituted with a C1-4 alkyl group), and even more preferably a C1-4 alkyl group.
$R_2$ is preferably a hydrogen atom or a halogen atom, more preferably a halogen atom, and even more preferably a fluorine atom.
$R_3$ is preferably a hydrogen atom, a C1-6 alkyl group, a C3-6 cycloalkyl group or a C1-6 alkoxy group (the C1-6 alkoxy group being optionally substituted with a hydroxy group), more preferably a hydrogen atom, a C1-4 alkyl group, a cyclopropyl group or a C1-4 alkoxy group (the C1-4 alkoxy group being optionally substituted with a hydroxy group), and even more preferably a hydrogen atom or a cyclopropyl group.
$R_4$ is preferably a halogen atom or a cyclopropyl group, and more preferably an iodine atom or a cyclopropyl group.
$R_5$ is preferably a halogen atom and more preferably a fluorine atom.
$R_6$ is preferably a hydrogen atom.
$R_7$ is preferably a hydrogen atom or a halogen atom, more preferably a hydrogen atom or a fluorine atom, and even more preferably a fluorine atom.
$R_8$ is preferably a hydrogen atom or a halogen atom, more preferably a hydrogen atom or a fluorine atom, and even more preferably a fluorine atom.
$X_1$ is preferably a halogen atom and more preferably a chlorine atom.
$R_9$ is preferably -C(=O)-O-$R_{12}$ (where $R_{12}$ is a C1-6 alkyl group or an aryl group), and more preferably -C(=O)-O-$CH_3$.

The compound of general formula (1) is, for example, 2-(4-cyclopropyl-2-fluoroanilino)-3,4-difluoro-5-[[3-fluoro-2-(methylsu lfamoylamino)pyridin-4-yl]methyl]benzamide.

The compound of general formula (4) is, for example, (2-amino-3-fluoropyridin-4-yl)methyl methyl carbonate.

The compound of general formula (2) is, for example, (2-amino-3-fluoropyridin-4-yl)methanol. (2-Amino-3-fluoropyridin-4-yl)methanol, for example, is a commercially available reagent.

Examples of the compound of $X_1$-$R_9$ include methyl chloroformate, ethyl chloroformate, acetic anhydride, acetyl chloride, dimethyl chlorophosphate, diethyl chlorophosphate, and diphenyl chlorophosphate. Preferably, it is, for example, at least one selected from the group consisting of methyl chloroformate and ethyl chloroformate. More preferably, it is, for example, methyl chloroformate. Methyl chloroformate, for example, is a commercially available reagent.

Examples of the base used in step (I) include triethylamine, N,N-diisopropylethylamine, N-methylmorpholine, imidazole, pyridine, N,N-dimethylaminopyridine, 2,6-dimethylpyridine, 1-methylimidazole, and 1,8-diazabicyclo[5.4.0]undec-7-ene. Preferably, it is, for example, at least one selected from the group consisting of N,N-dimethylaminopyridine and 1-methylimidazole. More preferably, it is, for example, N,N-dimethylaminopyridine.

Examples of the solvent used in step (I) include acetone, methyl ethyl ketone, ethyl acetate, isopropyl acetate, acetonitrile, N,N-dimethylacetamide, N,N-dimethylformamide, N,N-dimethylimidazolidinone, dimethyl sulfoxide, tetrahydrofuran, 2-methyltetrahydrofuran, 4-methyltetrahydropyran, cyclopentyl methyl ether, *tert*-butyl methyl ether, toluene, xylene, heptane, and cyclohexane. Preferably, it is, for example, at least one selected from the group consisting of acetonitrile, tetrahydrofuran, 2-methyltetrahydrofuran, 4-methyltetrahydropyran, cyclopentyl methyl ether, and *tert*-butyl methyl ether. More preferably, it is, for example, acetonitrile.

The reaction of step (I) can be performed by stirring the reaction mixture at a suitable temperature (e.g., 0°C

to 40°C) for a certain period of time (e.g., 0.5 hours to 24 hours).

The mixture obtained upon completion of the reaction of step (I) may be directly supplied to the subsequent step, or may be subjected to isolation or purification before being supplied to the subsequent step.

$R_{13}$ is preferably the group of formula (a) below, the group of formula (b) below, $-B(-OH)_2$, or $-BF_3K$, and more preferably the group of formula (a) below.

[Chemical Formula 19]

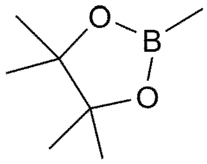

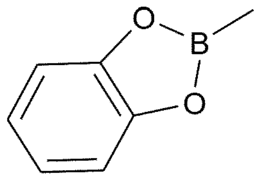

(a) (b)

The compound of general formula (10) is, for example, 2-(4-cyclopropyl-2-fluoroanilino)-3,4-difluoro-5-(4,4,5,5-tetramethyl-1, 3,2-dioxaboran-2-yl)benzamide.

The compound of general formula (5) is, for example, 5-[(2-amino-3-fluoropyridin-4-yl)methyl]-2-(4-cyclopropyl-2-fluoroanil ino)-3,4-difluorobenzamide.

The catalyst used in step (II) is, for example, a palladium catalyst or a nickel catalyst.

Examples of the palladium catalyst include a combination of:

at least one selected from the group consisting of bis(allylchloropalladium), tetrakis(triphenylphosphine)palladium, tris(dibenzylideneacetone)dipalladium, and palladium(II) acetate; and
a compound represented by general formula (L1) below:

[Chemical Formula 20]

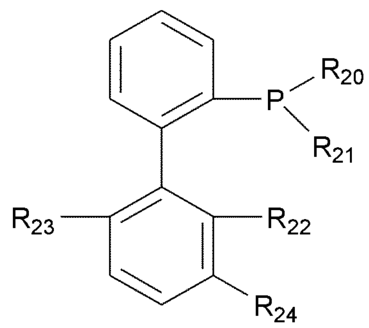

(L1)

wherein:

$R_{20}$ and $R_{21}$ are each independently a C3-6 cycloalkyl group;
$R_{22}$ is a C1-6 alkoxy group or an amino group (the amino group being optionally substituted with a C1-6 alkyl group or an aryl group);
$R_{23}$ is a hydrogen atom or a C1-6 alkoxy group; and
$R_{24}$ is a hydrogen atom or $-S(=O)_2-O-Na$.

Examples of the palladium catalyst also include a compound represented by general formula (L2) below:

[Chemical Formula 21]

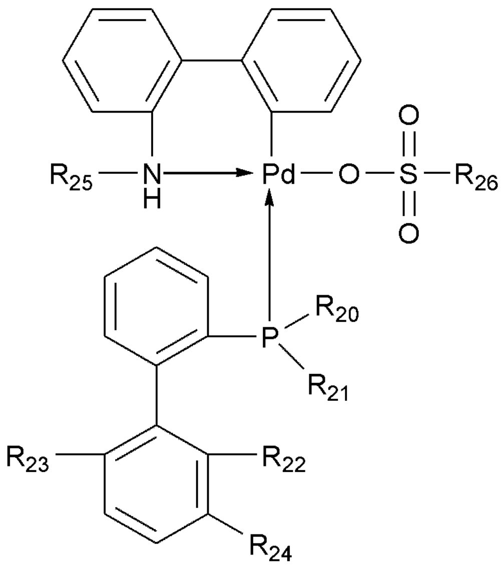

(L2)

wherein:

$R_{20}$ and $R_{21}$ are each independently a C3-6 cycloalkyl group;
$R_{22}$ is a C1-6 alkoxy group or an amino group (the amino group being optionally substituted with a C1-6 alkyl group or an aryl group);
$R_{23}$ is a hydrogen atom or a C1-6 alkoxy group;
$R_{24}$ is a hydrogen atom;
$R_{25}$ is a hydrogen atom or a C1-6 alkyl group;
$R_{26}$ is a C1-6 alkyl group; and
each arrow represents a coordinate bond.

$R_{20}$ and $R_{21}$ are preferably a cyclohexyl group.
$R_{22}$ is preferably a methoxy group, an isopropoxy group, or an N,N-dimethylamino group.
$R_{23}$ is preferably a hydrogen atom, a methoxy group, or an isopropoxy group.
$R_{24}$ is preferably a hydrogen atom.
$R_{25}$ is preferably a hydrogen atom or a methyl group.
$R_{26}$ is preferably a methyl group.
Preferably, the compound represented by general formula (L1) is, for example, at least one selected from the group consisting of 2',6'-dimethoxy-2-(dicyclohexylphosphino)biphenyl, 2-dicyclohexylphosphino-2',6' -diisopropoxybiphenyl, and 2-dicyclohexylphosphino-2'-(N,N-dimethylamino)biphenyl.
Preferably, the compound represented by general formula (L2) is, for example, at least one selected from the group consisting of:

(2-dicyclohexylphosphino-2' ,6' -dimethoxybiphenyl) [2-(2'-amin o-1,1'-biphenyl)]palladium(II) methanesulfonate,
2-dicyclohexylphosphino-2-(N,N-dimethylamino)biphenyl(2'-a mino-1,1'-biphenyl-2-yl)palladium(II) methanesulfonate, and
(2-dicyclohexylphosphino-2',6'-diisopropoxy-1,1'-biphenyl)[2-( 2'-amino-1,1'-biphenyl)]palladium(II) methanesulfonate.

More preferably, it is, for example, (2-dicyclohexylphosphino-2',6'-diisopropoxy-1,1'-biphenyl)[2-( 2'-amino-1,1'-biphenyl)]palladium(II) methanesulfonate.
Examples of the nickel catalyst include a combination of at least one selected from the group consisting of bis(1,5-cyclooctadiene)nickel and nickel(II) chloride and at least one selected from the group consisting of tricyclohexylphosphine, 1,1'-bis(diphenylphosphino)ferrocene, and 1,3-bis(diphenylphosphino)propane.
Examples of the nickel catalyst also include at least one selected from the group consisting of:

dichlorobis(tricyclohexylphosphine)nickel(II),

dichloro[1,1'-bis(diphenylphosphino)ferrocene]nickel(II), and
dichloro[1,3-bis(diphenylphosphino)propane]nickel(II).

Preferably, the catalyst used in step (II) is, for example, at least one selected from the group consisting of:

a combination of bis(allylchloropalladium) and 2',6'-dimethoxy-2-(dicyclohexylphosphino)biphenyl;
a combination of bis(allylchloropalladium) and 2-dicyclohexylphosphino-2',6' -diisopropoxybiphenyl;
a combination of bis(allylchloropalladium) and 2-dicyclohexylphosphino-2'-(N,N-dimethylamino)biphenyl;
(2-dicyclohexylphosphino-2' ,6' -dimethoxybiphenyl) [2-(2'-amin o-1,1'-biphenyl)]palladium(II) methanesulfonate;
2-dicyclohexylphosphino-2-(N,N-dimethylamino)biphenyl(2'-a mino-1,1'-biphenyl-2-yl)palladium(II) methanesulfonate; and
(2-dicyclohexylphosphino-2',6'-diisopropoxy-1,1'-biphenyl)[2-( 2'-amino-1,1'-biphenyl)]palladium(II) methanesulfonate.

More preferably, it is, for example, (2-dicyclohexylphosphino-2',6'-diisopropoxy-1,1'-biphenyl)[2-( 2'-amino-1,1'-biphenyl)]palladium(II) methanesulfonate.

When a combination of two or more compounds is used as a catalyst, they may form, for example, a complex in the solvent.

Examples of the solvent used in step (II) include C1-6 alcohols. C2-3 alcohols are preferred and ethanol is more preferred.

The reaction of step (II) can be performed by stirring the reaction mixture at a suitable temperature (e.g., 40°C to 90°C) for a certain period of time (e.g., 0.5 hours to 24 hours).

The mixture obtained upon completion of the reaction of step (II) may be directly supplied to the subsequent step, or may be subjected to isolation or purification before being supplied to the subsequent step.

The compound ($X_2$-S(=O)$_2$-NH-$R_{11}$ or $X_2$-S(=O)$_2$-$R_{11}$) that is reacted with the compound of general formula (5) in step (III) is preferably $X_2$-S(=O)$_2$-NH-R$1_1$ and more preferably N-methylsulfamoyl chloride.

Examples of the solvent used in step (III) include acetonitrile, N,N-dimethylacetamide, N,N-dimethylformamide, N,N-dimethylimidazolidinone, N,N-dimethylpropyleneurea, tetramethylurea, dimethyl sulfoxide, tetrahydrofuran, 2-methyltetrahydrofuran, 4-methyltetrahydropyran, pyridine, dichloromethane, and mixed solvents thereof. Preferably, it is, for example, N,N-dimethylacetamide, N,N-dimethylimidazolidinone, tetrahydrofuran, 2-methyltetrahydrofuran, or a mixed solvent thereof. More preferably, it is, for example, a mixed solvent of N,N-dimethylimidazolidinone and tetrahydrofuran.

Examples of the solvent used for producing, for example, a sodium salt in step (III) include acetone, acetonitrile, methanol, ethanol, tetrahydrofuran, 2-methyltetrahydrofuran, 4-methyltetrahydropyran, and mixed solvents thereof. Preferably, it is, for example, acetone, tetrahydrofuran, or a mixed solvent thereof.

With regard to, for example, a sodium salt produced in step (III), examples of the solvent used for precipitating the sodium salt as crystals include acetone, acetonitrile, methanol, ethanol, tetrahydrofuran, 2-methyltetrahydrofuran, 4-methyltetrahydropyran, cyclopentyl methyl ether, *tert*-butyl methyl ether, toluene, xylene, heptane, and mixed solvents thereof. Preferably, it is, for example, acetone, tetrahydrofuran, *tert*-butyl methyl ether, heptane, or a mixed solvent thereof. More preferably, it is, for example, a mixed solvent of acetone, tetrahydrofuran and *tert*-butyl methyl ether.

The reaction of step (III) can be performed by stirring the reaction mixture at a suitable temperature (e.g., -10°C to 30°C) for a certain period of time (e.g., 0.5 hours to 24 hours).

When a sodium salt of 2-(4-cyclopropyl-2-fluoroanilino)-3,4-difluoro-5-[[3-fluoro-2-(methylsu lfamoylamino)pyridin-4-yl]methyl]benzamide is produced by the method of the first or second aspect, a compound represented by formula (X) below or a sodium salt thereof can be generated.

[Chemical Formula 22]

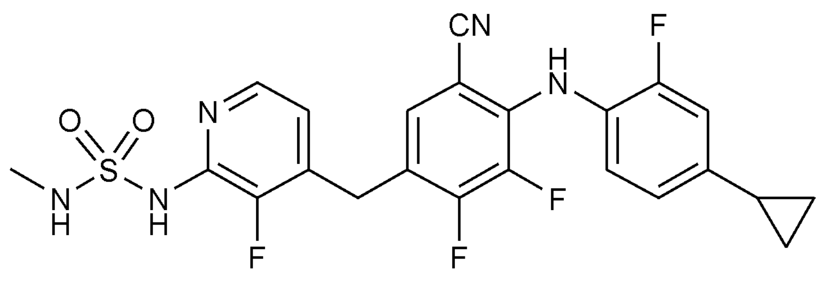

(X)

That is, in one aspect, the present disclosure provides a compound represented by formula (X) or a sodium salt thereof.

Also, in one aspect, the present disclosure provides a composition comprising a sodium salt of 2-(4-cyclopropyl-2-fluoroanilino)-3,4-difluoro-5-[[3-fluoro-2-(methylsu lfamoylamino)pyridin-4-yl]methyl]benzamide and a compound represented by formula (X) or a sodium salt thereof.

In one embodiment, the above composition is a pharmaceutical composition, preferably a pharmaceutical composition comprising a sodium salt of 2-(4-cyclopropyl-2-fluoroanilino)-3,4-difluoro-5-[[3-fluoro-2-(methylsu lfamoylamino)pyridin-4-yl]methyl]benzamide as an active ingredient.

In one embodiment, the above pharmaceutical composition is a pharmaceutical composition for the treatment or prevention of a cell proliferative disorder, particularly a cancer.

When the compound of formula (X) or a sodium salt thereof is generated, the amount of the compound of formula (X) or sodium salt thereof that is generated is small and is, for example, 3.0% w/w or less, 2.0% w/w or less, 1.0% w/w or less, 0.8% w/w or less, 0.5% w/w or less, or 0.3% w/w or less with respect to the weight of the sodium salt of 2-(4-cyclopropyl-2-fluoroanilino)-3,4-difluoro-5-[[3-fluoro-2-(methylsu lfamoylamino)pyridin-4-yl]methyl]benzamide that is generated.

That is, the amount of the compound of formula (X) or sodium salt thereof that is contained in the above composition is, for example, 3.0% w/w or less, 2.0% w/w or less, 1.0% w/w or less, 0.8% w/w or less, 0.5% w/w or less, or 0.3% w/w or less with respect to the weight of the sodium salt of 2-(4-cyclopropyl-2-fluoroanilino)-3,4-difluoro-5-[[3-fluoro-2-(methylsu lfamoylamino)pyridin-4-yl]methyl]benzamide that is contained in the composition.

The amount of the compound of formula (X) or sodium salt thereof that is contained in the above composition can be determined by, for example, HPLC analysis. Examples of the HPLC analysis conditions include the analysis conditions C listed in Table 1 below.

When the amount of the compound of formula (X) or sodium salt thereof that is contained in the above composition is determined by HPLC analysis, the peak area of the compound of formula (X) is, for example, 3.0% or less, 2.0% or less, 1.0% or less, 0.8% or less, 0.5% or less, or 0.3% or less with respect to the sum of the peak areas of 2-(4-cyclopropyl-2-fluoroanilino)-3,4-difluoro-5-[[3-fluoro-2-(methylsu lfamoylamino)pyridin-4-yl]methyl]benzamide, the compound of formula (X), and other degradation products of 2-(4-cyclopropyl-2-fluoroanilino)-3,4-difluoro-5-[[3-fluoro-2-(methylsu lfamoylamino)pyridin-4-yl]methyl]benzamide.

Seed crystals of a compound or a salt thereof may be used in connection with carrying out inventions of the present disclosure. Generally, seed crystals can be obtained by a method well known to those skilled in the art, such as by: cooling a solution of the compound or salt; adding a solvent with low solubility for the compound or salt (i.e., a poor solvent); rubbing with a spatula the wall of a vessel containing a solution of the compound or salt; or concentrating a solution of the compound or salt under reduced pressure after purification by silica gel column chromatography.

The following are examples of abbreviations used throughout the present specification and their meanings.

AA: Ammonium acetate
tAmOH: *tert*-Amyl alcohol
Boc: *tert*-Butoxycarbonyl
tBuOH: *tert*-Butanol
2-BuOH: 2-Butanol
tBuXPhos: 2-Di-*tert*-butylphosphino-2',4',6'-trilsopropylbiphenyl
tBuDavePhos: 2-Di-*tert*-butylphosphino-2'-(N,N-dimethylamino)biphenyl
COMU: (1-Cyano-2-ethoxy-2-oxoethylideneaminooxy)dimethylamino-morpholi no-carbenium hexafluorophosphate
CPME: Cyclopentyl methyl ether
CyJohnPhos: 2-(Dicyclohexylphosphino)biphenyl
DavePhos: 2-Dicyclohexylphosphino-2'-(N,N-dimethylamino)biphenyl
DBU: Diazabicycloundecene
DCC: N,N'-Dicyclohexylcarbodiimide
DCM: Dichloromethane
DIPEA: N,N-Diisopropylethylamine
DMA: N,N-Dimethylacetamide
DMAP: N,N-Dimethylaminopyridine
DMF: N,N-Dimethylformamide
DMI: 1,3-Dimethyl-2-imidazolidinone
DMSO: Dimethyl sulfoxide
EDC: 1-Ethyl-3 -(3 -dimethylaminopropyl)carbodiimide
EDC·HCl: 1-(3-Dimethylaminopropyl)-3-ethylcarbodiimide hydrochloride
EtOH: Ethanol

FA: Formic acid
HATU: O-(7-Azabenzotriazol-1-yl)-N,N,N',N'-tetramethyluronium hexafluorophosphate
HOAt: 1-Hydroxy-7-azabenzotriazole
HOOBt: 3,4-Dihydro-3-hydroxy-4-oxo-1,2,3-benzotriazine
IPA: Isopropanol
JohnPhos: (2-Biphenyl)di-*tert*-butylphosphine
LDA: Lithium diisopropylamide
2-MeTHF: 2-Methyltetrahydrofuran
MTHP: 4-Methyltetrahydropyran
MeCN: Acetonitrile
MeOH: Methanol
MePhos: 2-Dicyclohexylphosphino-2'-methylbiphenyl
NMP: N-Methyl-2-pyrrolidone
1-PrOH: 1-Propanol
$[PdCl(allyl)]_2$: Bis(allylchloropalladium)
RuPhos: 2-Dicyclohexylphosphino-2',6'-diisopropoxybiphenyl
RuPhos-Pd-G3 : (2-Dicyclohexylphosphino-2',6'-diisopropoxy-1,1'-biphenyl)[2-(2'-ami no-1,1'-biphenyl)]palladium(II) methanesulfonate
SPhos: 2',6 '-Dimethoxy-2-(dicyclohexylphosphino)biphenyl
TBME: *tert*-Butyl methyl ether
TBS: *tert*-Butyldimethylsilyl
TFA: Trifluoroacetic acid
THF: Tetrahydrofuran
Xantphos: 4,5-Bis(diphenylphosphino)-9,9-dimethylxanthene
XPhos-Pd-G3: (2-Dicyclohexylphosphino-2',4',6'-triisopropyl-1,1' -biphenyl) [2-(2' -am ino-1,1'-biphenyl)]palladium(II) methanesulfonate

As used herein, "room temperature" means a temperature of about 20°C to about 25°C.

## Examples

The present disclosure will now be explained in greater detail based on examples, with the understanding that the present disclosure is in no way limited to the examples.

[Synthetic Examples]

In the synthetic examples below, high-performance liquid chromatography (HPLC) analysis was conducted using one of the analysis conditions listed in Table 1 below. Each compound was detected using a photodiode array detector or a mass spectrometer, but another technique such as evaporative light scattering detection may be used.

[Table 1-1]

| Table 1 | | | | |
|---|---|---|---|---|
| Analysis conditions | Apparatus | Column | Column temperature | Detection wavelength (PDA) |
| A | Waters H-Class | InertSustain AQ-C18 HP 2.1 mmI.D. × 50 mm L, 3 μm | 35°C | 210-400 nm |
| B | Acquity SQD/SQD2 | Ascentis Express C18 2.1 mm I.D. × 50 mm L, 2.7 μm | 35°C | 210-400 nm |
| C | Waters H-Class | InertSustain AQ-C18 HP 2.1 mmI.D. × 50 mm L, 3 μm | 35°C | 210-400 nm |

(continued)

| Table 1 | | | | |
|---|---|---|---|---|
| Analysis conditions | Apparatus | Column | Column temperature | Detection wavelength (PDA) |
| D | Acquity SQD/SQD2 | Ascentis Express C18 2.1 mmI.D. × 50 mm L, 5 μm | 35°C | 210-400 nm |
| E | Nexera UC LCMS-2020 | Ascentis Express C18 2.1 mm I.D. × 50 mm L, 2.7 μm | 35°C | 210-400 nm |

[Table 1-2]

| Table 1 (cont.) | | | | |
|---|---|---|---|---|
| Analysis conditions | Mobile phase | Gradient | | Flow rate (mL/min) |
| | | Time after injection (min) | A/B | |
| A | A) 0.05% TFA/ $H_2O$<br>B) 0.05% TFA/ MeCN | 0-5.0<br>5.0-6.0 | 100/0 → 0/100<br>0/100 | 0.5 |
| B | A) 0.1% FA/MeCN<br>B) 0.1% FA/$H_2O$ | 0-1.0<br>1.0-1.4 | 5/95 → 100/0<br>100/0 | 1 |
| C | A) 0.05% TFA/ $H_2O$<br>B) 0.05% TFA/ MeCN | 0-10.0<br>10.0-15.0<br>15.0-16.0 | 65/35 → 55/45<br>55/45 → 0/100<br>0/100 | 0.5 |
| D | A) MeOH<br>B) 10 mMAA/ $H_2O$ | 0-1.0<br>1.0-1.4 | 5/95 → 100/0<br>100/0 | 0.9 |
| E | A) 0.05% TFA/ MeCN<br>B) 0.05% TFA/ $H_2O$ | 0-1.5<br>1.5-2.0 | 5/95 → 100/0<br>100/0 | 1 |

NMR was performed using a JEOL JNM-ECZ500R Nuclear Magnetic Resonance spectrometer. The NMR data were shown in ppm (parts per million) (δ) and the deuterium lock signal from the sample solvent was used as a reference.
Commercially available reagents were used directly without further purification.
All of the nonaqueous reactions were conducted in anhydrous solvents.
Concentration under reduced pressure and solvent distillation were carried out using a rotary evaporator.

(Synthetic Example 1)

Synthesis of (2-amino-3-fluoropyridin-4-yl)methyl methyl carbonate (compound 4A)

[Chemical Formula 23]

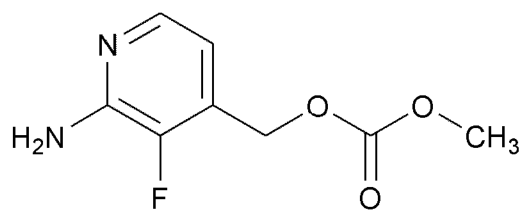

(1-1) Synthesis of (2-amino-3-fluoropyridin-4-yl)methyl methyl carbonate (compound 4A)

(2-Amino-3-fluoropyridin-4-yl)methanol (compound 2A) (30.0 g, 211 mmol) and DMAP (28.4 g, 232 mmol) were added to a reaction vessel, and then this was purged with nitrogen. MeCN (885 mL) was added, and after confirming that the reaction solution had been homogenized, methyl chloroformate (compound 3A) (16.2 mL, 211 mmol) was added dropwise over 1 hour. MeCN (15 mL) was added and the solution was stirred at 25°C for 2 hours. After distilling off the solvent at 40°C under reduced pressure to give a solution volume of 90 mL, isopropyl acetate (900 mL) was added. This solution was washed once with a 15% sodium chloride aqueous solution (300 mL), once with a 15% ammonium chloride aqueous solution (300 mL), and twice with water (150 mL), and then the solvent was distilled off at 40°C under reduced pressure to give a solution volume of 90 mL. Toluene (300 mL) was added to the resulting solution, and then the solvent was distilled off at 40°C under reduced pressure to give a solution volume of 90 mL. Toluene (300 mL) was again added, and the solvent was distilled off at 40°C under reduced pressure to give a solution volume of 90 mL. Toluene (30 mL) was further added and the internal temperature of the solution was increased to 60°C to dissolve the precipitated solid, and then the temperature was decreased to 40°C over 30 minutes. Seed crystals (75 mg) obtained in (1-2) below were added and the solution was stirred for 30 minutes. Then the internal temperature of the solution was decreased to 25°C over 30 minutes and the solution was stirred for 30 minutes. Heptane (60 mL) was added over 30 minutes and the solution was stirred for 30 minutes. Heptane (60 mL) was again added over 30 minutes and the solution was stirred for 1 hour and 30 minutes. Heptane (120 mL) was further added over 30 minutes and the solution was stirred for 30 minutes. The precipitated solid was filtered off and washed with a mixed solvent of heptane (45 mL) and toluene (15 mL), and then dried under reduced pressure to give compound 4A (35.1 g, 83% yield).

HPLC purity: 98.86% (analysis conditions A)

$^1$H-NMR (DMSO-$d_6$) $\delta$: 7.69 (1H, d, J = 5.2 Hz), 6.47 (1H, t, J = 4.9 Hz), 6.26 (2H, s), 5.15 (2H, s), 3.73 (3H, s).
MS (ESI$^+$) m/z: 201 $[M+H]^+$

(1-2) Synthesis of seed crystals of (2-amino-3-fluoropyridin-4-yl)methyl methyl carbonate (compound 4A)

(2-Amino-3-fluoropyridin-4-yl)methanol (compound 2A) (10.0 g, 70.4 mmol) and DMAP (12.9 g, 106 mmol) were added to a reaction vessel under a nitrogen atmosphere, and then purging with nitrogen was performed. MeCN (300 mL) was added, the mixture was stirred, and methyl chloroformate (compound 3A) (5.4 mL, 70 mmol) was added dropwise to the resulting homogeneous solution at an external temperature of 25°C over 1 hour. The resulting reaction solution was stirred for 3 hours and then concentrated under reduced pressure. 2-MeTHF (100 mL) was added to the concentrated residue and concentrated under reduced pressure, and 2-MeTHF (100 mL) was again added to the residue and concentrated. After adding 2-MeTHF (50 mL) to the concentrated residue, the precipitated solid was filtered off and the filtration residue was washed with 2-MeTHF (30 mL) (the resulting wash solution being referred to as the wash solution 1). The remaining filtration residue was again washed with 2-MeTHF (100 mL) (the resulting wash solution being referred to as the wash solution 2). The filtrate and the wash solution 1 were combined and the mixture was concentrated under reduced pressure to a total volume of 25 mL. Then 2-MeTHF (5 mL) was added, and the mixture was heated and stirred at an external temperature of 40°C to give a homogeneous solution. Heptane (30 mL) was added to this solution over 1 hour, and this was cooled to an external temperature of 35°C. Heptane (30 mL) was again added to the resulting mixture over 1 hour, and the mixture was cooled to an external temperature of 25°C. The precipitated solid was filtered off and the filtration residue was washed with a liquid mixture of heptane/2-MeTHF (3:1, 28 mL). The filtrate and the wash solution were combined and the mixture was concentrated under reduced pressure. The concentrated residue, the residue remaining in the reaction vessel after filtration, and the residue obtained by concentrating the wash solution 2 under reduced pressure were combined, and the mixture was purified by silica gel column chromatography using ethyl acetate and heptane as the mobile phase. The resulting solution of compound 4A in ethyl acetate/ heptane was concentrated under reduced pressure. The colorless solid obtained by the concentration was dried at an external temperature of 40°C under reduced pressure to give compound 4A (4.2 g, 30% yield) as crystals.
HPLC purity: 99.99% (analysis conditions A)

(1-3) Reaction selectivity and conversion in various solvents

The reaction selectivity and conversion in the solvents listed in Table 2 below were investigated as follows. (2-Amino-3-fluoropyridin-4-yl)methanol (compound 2A), DMAP, and the solvent were added to a reaction vessel, and the mixture was stirred at room temperature. After adding 1.0 equivalent of methyl chloroformate (compound 3A) relative to compound 2A, stirring was continued for a predetermined time (i.e., the time set forth in Table 2). The resulting reaction mixture was subjected to HPLC analysis under the analysis conditions A.

The results are shown in Table 2. Table 2 shows the peak area ratio of impurity A, impurity B or impurity C mentioned below to compound 4A and the conversion calculated according to the formula below.

Conversion = [peak area of compound 4A / (peak area of compound 2A + peak area of compound 4A)] × 100%

Compound 2A
HPLC retention time: about 0.77 min
Compound 4A
HPLC retention time: 1.63 min
Impurity A

LCMS m/z: 258
HPLC retention time: 2.24 min

Impurity B

LCMS m/z: 369

HPLC retention time: 2.47 min

Impurity C

LCMS m/z: 427

HPLC retention time: 3.11 min

[Table 2]

Table 2

| Solvent | Amount of compound 2A added | Equivalents of DMAP | Stirring time | Impurity A/ Compound 4A | Impurity B/ Compound 4A | Impurity C/ Compound 4A | Conversion |
|---|---|---|---|---|---|---|---|
| 2-MeTHF 4mL | 200 mg | 1.0 | 1 hr | 0.017 | 0.001 | 0.010 | 90% |
| MTHP 4mL | 200 mg | 1.0 | 1 hr | 0.028 | 0.017 | 0.026 | 73% |
| THF 4mL | 200 mg | 1.0 | 1 hr | 0.021 | 0.013 | 0.018 | 80% |
| CPME 4mL | 200 mg | 1.0 | 1 hr | 0.022 | 0.006 | 0.010 | 80% |
| TBME 4mL | 200 mg | 1.0 | 1 hr | 0.021 | 0.001 | 0.006 | 92% |
| MeCN 3mL | 100 mg | 1.0 | 1 hr | 0.003 | ND | ND | 81% |
| MeCN 3mL | 100 mg | 1.1 | 1 hr | 0.004 | ND | ND | 88% |

(continued)

| Solvent | Amount of compound 2A added | Equivalents of DMAP | Stirring time | Impurity A/ Compound 4A | Impurity B/ Compound 4A | Impurity C/ Compound 4A | Conversion |
|---|---|---|---|---|---|---|---|
| MeCN 3mL | 100 mg | 1.5 | 30 min | ND | ND | ND | 94% |
| ND: Not detected | | | | | | | |

As the above results show, MeCN is superior in terms of reaction selectivity and conversion.

(Synthetic Example 2)

Synthesis of 2-(4-cyclopropyl-2-fluoroanilino)-3,4-difluoro-5-(4,4,5,5-tetramethyl-1, 3,2-dioxaboran-2-yl)benzamide (compound 10A)

[Chemical Formula 24]

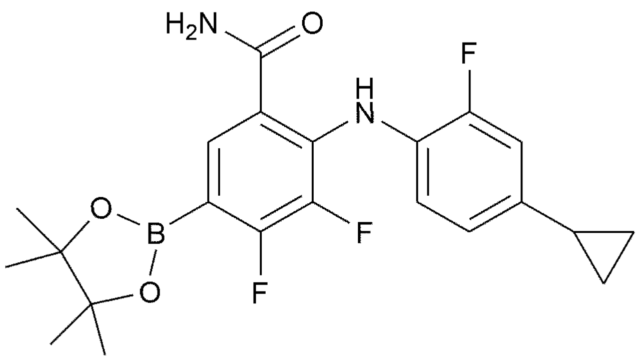

(2-1) Synthesis of 5-bromo-2-(4-cyclopropyl-2-fluoroanilino)-3,4-difluorobenzoic acid (compound 8A)

[Chemical Formula 25]

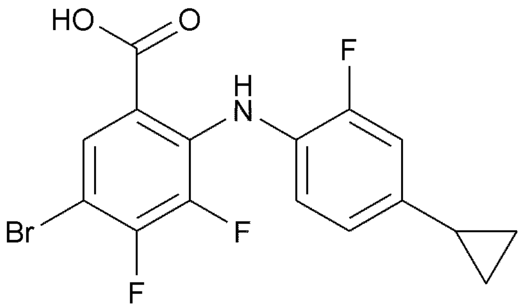

A reaction vessel in which a 1 M lithium bis(trimethylsilyl)amide THF solution (206 mL, 206 mmol) had been placed was cooled to an external temperature of -15°C, and a solution of 4-cyclopropyl-2-fluoroaniline (11.6 g, 76.5 mmol) in THF (30 mL) was added dropwise. Then a solution of 5-bromo-2,3,4-trifluorobenzoic acid (15.0 g, 58.8 mmol) in THF (120 mL) was added dropwise over 30 minutes and the mixture was stirred for 30 minutes. Then 5 M hydrochloric acid (118 mL) was added to the reaction mixture, the temperature was increased to room temperature, and extraction was performed with isopropyl acetate (75 mL). The organic layer was washed sequentially twice with water (75 mL) and

once with a 15% sodium chloride aqueous solution (75 mL), and concentrated under reduced pressure. Acetone (120 mL) was added to the resulting concentrated residue, and after heating to dissolution, water (45 mL) and seed crystals (150 mg) were added to precipitate crystals. Water (45 mL) was added to the resulting slurry, and the crystals were filtered off. After washing with a liquid mixture of acetone/water (1/2), they were dried at an external temperature of 40°C under reduced pressure to give compound 8A (19.4 g, 85% yield).
LCMS m/z: 386 $[M+H]^+$
HPLC retention time: 0.62 min (analysis conditions B)

(2-2) Synthesis of 5-bromo-2-(4-cyclopropyl-2-fluoroanilino)-3,4-difluorobenzamide (compound 9A)

[Chemical Formula 26]

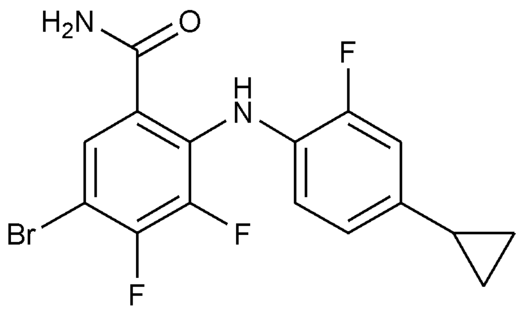

MeCN (104 mL), THF (26 mL), and 1,1'-carbonyldiimidazole (8.2 g, 50.5 mmol) were added to a reaction vessel in which 5-bromo-2-(4-cyclopropyl-2-fluoroanilino)-3,4-difluorobenzoic acid (compound 8A) (13.0 g, 33.7 mmol) had been placed, and the mixture was stirred for 2 hours at room temperature. After adding 28% ammonia water (13 mL) to the reaction mixture, this was stirred for 30 minutes at room temperature, and then water (117 mL) was added over 1 hour. Crystals were filtered off and washed with water, and then dried at an external temperature of 40°C under reduced pressure to give compound 9A (12.0 g, 93% yield).
LCMS m/z: 385 $[M+H]^+$
HPLC retention time: 0.52 min (analysis conditions B)

(2-3) Synthesis of 2-(4-cyclopropyl-2-fluoroanilino)-3,4-difluoro-5-(4,4,5,5-tetramethyl-1, 3,2-dioxaboran-2-yl)benzamide (compound 10A)

Potassium acetate (7.64 g, 77.8 mmol), 5-bromo-2-(4-cyclopropyl-2-fluoroanilino)-3,4-difluorobenzamide (compound 9A) (10.0 g, 26.0 mmol), and bispinacoldiboron (7.25 g, 28.6 mmol) were suspended in 2-MeTHF (150 mL) under a nitrogen atmosphere, and the internal area of the reaction vessel was purged with nitrogen. XPhos-Pd-G3 (440 mg, 0.519 mmol) was added to the resulting mixture, and the internal area of the reaction vessel was again purged with nitrogen. The mixture was heated to an external temperature of 80°C and stirred for 6 hours. The mixture was cooled to room temperature, and after filtration the filtrate was concentrated under reduced pressure. Cyclopentyl methyl ether (50 mL) was added to the residue, and the mixture was again concentrated under reduced pressure. Cyclopentyl methyl ether (50 mL) was added to the resulting concentrated residue, and the mixture was again concentrated under reduced pressure. Then cyclopentyl methyl ether (50 mL) was added to the residue, and the precipitated solid was filtered off. The filtration residue was washed with cyclopentyl methyl ether (30 mL) and then dried at 40°C under reduced pressure to give compound 10A (7.02 g, 63% yield).
HPLC purity: 97.60% (analysis conditions A)
$^1$H-NMR (DMSO-$d_6$) $\delta$: 9.83 (1H, brs), 8.40-8.32 (1H, brs), 7.77 (1H, d, J = 5.2 Hz), 7.72-7.63 (1H, brs), 6.96-6.90 (2H, m), 6.83 (1H, dd, J = 1.7, 8.0 Hz), 1.93-1.86 (1H, m), 1.30 (12H, s), 0.95-0.90 (2H, m), 0.67-0.63 (2H, m).
MS (ESI$^+$) m/z: 433 $[M+H]^+$
Some compounds 10A are hydrolyzed to boronic acid during HPLC analysis. Therefore, the purity of compound 10A was determined from the sum of the peak areas of compound 10A (retention time: 4.57 min; m/z: 433 $[M+H]^+$) and boronic acid (retention time: about 3.48 min; m/z: 351 $[M+H]^+$).

(Synthetic Example 3)

Synthesis of 5-[(2-amino-3-fluoropyridin-4-yl)methyl]-2-(4-cyclopropyl-2-fluoroanil ino)-3,4-difluorobenzamide (compound 5A)

[Chemical Formula 27]

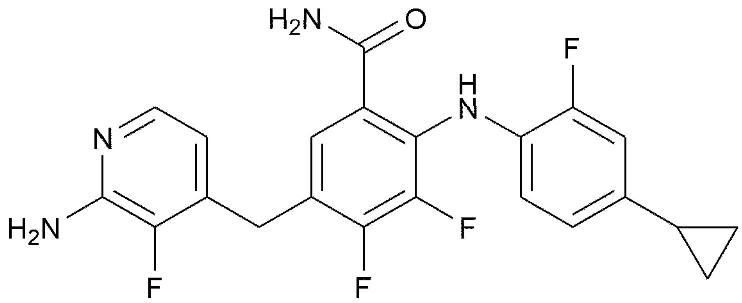

(3-1) Synthesis of 5-[(2-amino-3-fluoropyridin-4-yl)methyl]-2-(4-cyclopropyl-2-fluoroanil ino)-3,4-difluorobenzamide (compound 5A)

5-Bromo-2-(4-cyclopropyl-2-fluoroanilino)-3,4-difluorobenzami de (compound 9A) (15.00 g, 38.9 mmol), potassium acetate (11.47 g, 117 mmol), bispinacoldiboron (10.88 g, 42.8 mmol), and 2-MeTHF (113 mL) were added to a reaction vessel, and then the internal area of the reaction vessel was purged with nitrogen. XPhos-Pd-G3 (659 mg, 0.779 mmol) was added to the reaction solution, and then the internal area of the reaction vessel was purged with nitrogen. The internal temperature of the reaction solution was increased to 80°C and the solution was stirred for 4 hours. The internal temperature of the reaction solution was decreased to 25°C, and then (2-amino-3-fluoropyridin-4-yl)methyl methyl carbonate (compound 4A) (11.69 g, 58.4 mmol), potassium carbonate (16.15 g, 117 mmol), and RuPhos-Pd-G3 (1.629 g, 1.947 mmol) were added. The internal area of the reaction vessel was purged with nitrogen (the oxygen concentration in the reaction vessel ≤ 0.1%), and then the internal temperature of the reaction solution was increased to 75°C. EtOH (52 mL, 900 mmol) was slowly added dropwise (so that the internal temperature of the solution did not fall below 60°C), and then the solution was stirred for 4 hours and 30 minutes. After confirming degradation of compound 4A (less than 1%), 2-MeTHF (113 mL) and an N-acetyl-L-cysteine aqueous solution (1.271 g, 113 mL) were added and the mixture was stirred for 1 hour. The internal temperature of the reaction solution was decreased to 40°C, and then extraction was performed. The organic layer was washed sequentially with 0.1 M hydrochloric acid (113 mL), a 0.1 M potassium phosphate aqueous solution (113 mL), and a 2% sodium chloride aqueous solution (113 mL), and the resulting organic layer was filtered. The filtrate was concentrated under reduced pressure to a total volume of 75 mL, and toluene (225 mL) was added to the concentrated residue. The solvent was distilled off under reduced pressure to give a solution volume of 75 mL, and then toluene (225 mL) was again added. The solvent was distilled off under reduced pressure to give a solution volume of 75 mL, and then 1-butanol (12 mL) was added as an internal standard. $^1$H-NMR was used to determine the content of toluene, and toluene was added to give a solution containing 240 mL of toluene. The internal temperature of the reaction solution was increased to 110°C, and after confirming complete dissolution of solids, it was decreased to 90°C. Seed crystals (75 mg) obtained in (4-2) (5) below were added, and then the internal temperature of the reaction solution was decreased to 80°C and the mixture was stirred for 1 hour. The temperature of the solution was decreased to 60°C and the solution was stirred for 30 minutes. The temperature of the solution was decreased to 40°C and the solution was stirred for 30 minutes. The temperature of the solution was decreased to 25°C and the solution was stirred for 30 minutes. The temperature of the solution was decreased to 5°C and the solution was stirred for 30 minutes. The precipitated solid was filtered off, washed with toluene (45 mL), and filtered under reduced pressure to give compound 5A (12.6 g, 75% yield).

HPLC purity: 99.69% (analysis conditions A)

$^1$H-NMR (DMSO-$d_6$) δ: 9.38 (1H, brs), 8.19 (1H, brs), 7.70 (1H, brs), 7.65 (1H, d, J = 5.2 Hz), 7.58 (1H, d, J = 6.9 Hz), 6.90 (1H, d, J = 13.2), 6.81-6.76 (2H, m), 6.38 (1H, t, J = 5.2 Hz), 6.14 (2H, brs), 3.91 (2H, s), 1.90-1.84 (1H, m), 0.92-0.88 (2H, m), 0.64-0.61 (2H, m).

MS (ESI$^+$) m/z: 431 $[M+H]^+$

(3-2) Reaction selectivity and conversion in various solvents

The reaction selectivity and conversion in the solvents listed in Table 3 below were investigated as follows. 2-(4-Cyclopropyl-2-fluoroanilino)-3,4-difluoro-5-(4,4,5,5-tetramethyl-1 ,3,2-dioxaboran-2-yl)benzamide (compound 10A) (100 mg, 0.231 mmol), MeTHF (1.5 mL), (2-amino-3-fluoropyridin-4-yl)methyl methyl carbonate (compound 4A) (93 mg, 0.46 mmol), and potassium carbonate (96 mg, 0.69 mmol) were sequentially added to a reaction vessel, and the internal area of the reaction vessel was purged with nitrogen. Bis(allylchloropalladium) (2.1 mg, 5.8 μmol), 2',6'-dimethoxy-2-(dicyclohexylphosphino)biphenyl (4.8 mg, 12 μmol) and the solvent (1.5 mL) were added to the resulting mixture, and after purging the reaction vessel with nitrogen again, the temperature of the reaction solution was increased to 70°C while stirring. After adding an additive ($H_2O$) (30 μL) over about 30 minutes or without adding an additive ($H_2O$), stirring was continued for 1 hour. The resulting reaction mixture was subjected to HPLC analysis under the analysis conditions A.

The results are shown in Table 3. Table 3 shows the peak area ratio of compound 5A to impurity D mentioned below and the conversion calculated according to the formula below.

Conversion = [(peak area of compound 5A + peak area of impurity D + peak area of impurity E) / (peak area of compoud 10A + peak area of boronic acid + peak area of compound 5A + peak area of impurity D + peak area of impurity E)] × 100%

Compound 5A
HPLC retention time: about 3.31 min

Impurity D
HPLC retention time: about 3.98 min

Impurity E

LCMS m/z: 611

HPLC retention time: about 5.06 min

[Table 3]

Table 3

| Solvent | Additive | Compound 5A/ Impurity D | Conversion |
|---|---|---|---|
| DMI | $H_2O$ | 0.09 | 71% |
| Toluene | $H_2O$ | 0.33 | 56% |
| 2-BuOH | $H_2O$ | 0.59 | 100% |
| CPME | $H_2O$ | 0.39 | 57% |
| EtOH | $H_2O$ | 4.97 | 100% |
| 1-PrOH | $H_2O$ | 3.16 | 98% |
| IPA | $H_2O$ | 1.48 | 100% |
| tBuOH | $H_2O$ | 1.11 | 100% |
| tAmOH | $H_2O$ | 1.02 | 97% |
| EtOH | None | 4.84 | 98% |

As the above results show, EtOH is superior in terms of reaction selectivity and conversion.

The structural formula, NMR data, and m/z of impurity D are as follows.

[Chemical Formula 28]

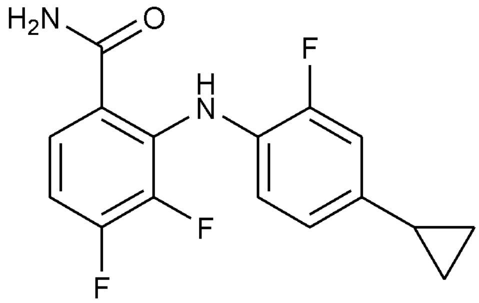

$^1$H-NMR (DMSO-$d_6$) δ: 9.63 (1H, brs), 8.25-8.20 (1H, brs), 7.75-7.70 (1H, brs), 7.61 (1H, ddd, J = 1.7, 5.7, 8.0 Hz), 7.09-7.02 (1H, m), 6.91 (1H, dd, J = 1.7, 12.0 Hz), 6.85-6.80 (2H, m), 1.91-1.85 (1H, m), 0.93-0.89 (2H, m), 0.65-0.62 (2H, m).

MS (ESI$^+$) m/z: 307 $[M+H]^+$

(3-3) Reaction selectivity and conversion in various catalysts

The reaction selectivity and conversion in the catalysts listed in Table 4 below were investigated as follows. 2-(4-Cyclopropyl-2-fluoroanilino)-3,4-difluoro-5-(4,4,5,5-tetramethyl-1 ,3,2-dioxaboran-2-yl)benzamide (compound 10A) (100 mg, 0.231 mmol), MeTHF (1.5 mL), (2-amino-3-fluoropyridin-4-yl)methyl methyl carbonate (compound 4A) (93 mg, 0.463 mmol), and potassium carbonate (96 mg, 0.694 mmol) were sequentially added to a reaction vessel, and the internal area of the reaction vessel was purged with nitrogen. The catalyst and EtOH (0.7 mL) were added to the resulting mixture, and the reaction vessel was again purged with nitrogen. The reaction solution was stirred at 70°C for 1.5 hours, and the resulting reaction mixture was subjected to HPLC analysis under the analysis conditions A.

The results are shown in Table 4. Table 4 shows the peak area ratio of compound 5A to impurity D mentioned in (3-2) above and the conversion calculated according to the formula shown in (3-2) above.

[Table 4]

Table 4

| Catalyst | Compound 5A/ Impurity D | Conversion |
|---|---|---|
| $[PdCl(allyl)]_2$ + SPhos | 6.68 | 100% |
| $[PdCl(allyl)]_2$ + tBuXPhos | 0.00 | 100% |
| $[PdCl(allyl)]_2$ + tBuDavePhos | 0.01 | 100% |
| $[PdCl(allyl)]_2$ + JohnPhos | 0.05 | 100% |
| $[PdCl(allyl)]_2$ + RuPhos | 9.44 | 100% |
| $[PdCl(allyl)]_2$ + CyJohnPhos | 0.29 | 55% |
| $[PdCl(allyl)]_2$ + DavePhos | 2.16 | 100% |
| $[PdCl(allyl)]_2$ + MePhos | 0.11 | 64% |
| RuPhos-Pd-G3 | 13.37 | 100% |

As the above results show, a combination of $[PdCl(allyl)]_2$ and Sphos, a combination of $[PdCl(allyl)]_2$ and RuPhos, a combination of $[PdCl(allyl)]_2$ and DavePhos, or RuPhos-Pd-G3 is superior in terms of reaction selectivity and conversion.

(Synthetic Example 4)

Synthesis of a sodium salt of 2-(4-cyclopropyl-2-fluoroanilino)-3,4-difluoro-5-[[3-fluoro-2-(methylsu lfamovlamino)pvridin-4-vl]methyl]benzamide (compound 1A)

[Chemical Formula 29]

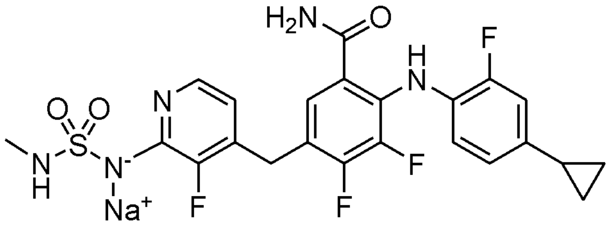

(4-1) Preparation of sample 1a (Form I)

5-[(2-Amino-3-fluoropyridin-4-yl)methyl]-2-(4-cyclopropyl-2-fl uoroanilino)-3,4-difluorobenzamide (compound 5A) (3.00 g, 6.97 mmol) was added to a reaction vessel, and then 1,3-dimethyl-2-imidazolidinone (12 mL) and THF (6 mL) were added to dissolve it. After purging the reaction vessel with nitrogen, pyridine (1.69 mL, 20.91 mmol) was added and the mixture was cooled to 0°C. N-Methylsulfamoyl chloride (0.67 mL, 7.67 mmol) was added and the mixture was stirred for 45 minutes, and then pyridine (0.10 mL, 1.26 mmol) and N-methylsulfamoyl chloride (0.30 mL, 3.42 mmol) were added and the mixture was stirred for 35 minutes. Subsequently, pyridine (0.24 mL, 2.93 mmol) and N-methylsulfamoyl chloride (0.13 mL, 1.46 mmol) were added and the mixture was stirred for 35 minutes, and then pyridine (0.09 mL, 1.12 mmol) and N-methylsulfamoyl chloride (0.05 mL, 0.56 mmol) were added and the mixture was stirred for 3 hours. The reaction solution was diluted with THF (18 mL) and TBME (24 mL), and then a 10% sodium chloride aqueous solution (15 g) was added to stop the reaction, after which the temperature was increased to 25°C. The reaction solution was separated into two layers, and the upper layer (organic layer) was washed with a 10% sodium chloride aqueous solution (24 g). The resulting organic layer was concentrated under reduced pressure to 15 mL and diluted with THF (45 mL). This procedure was repeated two more times, and then the precipitated inorganic salts were filtered off. The filtered off inorganic salts were washed with THF (15 mL) and this was combined with the filtrate. Then the mixture was concentrated under reduced pressure to 15 mL. The residue was diluted with acetone (11 mL), and then THF (9.3 mL) was added. After warming the resulting solution to 40°C, a 5M sodium hydroxide aqueous solution (1.32 mL, 6.62 mmol) and a suspension of seed crystals of a sodium salt of compound 1A (1.82 mg) (sample 1b mentioned below) in acetone (0.7 mL) were sequentially added and the mixture was stirred for 2 hours and 30 minutes. Acetone (6.6 mL) was added over 30 minutes and the mixture was stirred for 2 hours. Acetone (18.2 mL) was added over 20 minutes and the mixture was stirred for 45 minutes. TBME (24 mL) was added over 20 minutes and the mixture was stirred for 50 minutes. The resulting suspension was cooled to 25°C over 30 minutes, stirred for 1 hour, and allowed to stand at room temperature overnight. After standing overnight, the suspension was stirred at 25°C for 2 hours and 30 minutes. The precipitated solid was filtered off, washed with a mixed solvent of acetone (11.0 mL) and TBME (11.0 mL), and dried under reduced pressure to give a sodium salt of compound 1A (2.77 g, 73% yield) (sample 1a (Form I)).

HPLC purity: 99.49% (analysis conditions C)

HPLC retention time: 7.02 min (analysis conditions C)

$^1$H-NMR (DMSO-$d_6$) δ: 9.36 (1H, brs), 8.21 (1H, brs), 7.68 (1H, brs), 7.60-7.55 (2H, m), 6.89 (1H, brd, J = 13.5 Hz), 6.82-6.75 (2H, m), 6.17 (1H, t, J = 5.0 Hz), 5.50 (1H, q, J = 6.0 Hz), 3.85 (2H, s), 2.28 (3H, d, J = 6.0 Hz), 1.90-1.83 (1H, m), 0.92-0.87 (2H, m), 0.64-0.60 (2H, m).

MS (ESI$^+$) m/z: 524 $[M+2H-Na]^+$

In the HPLC analysis of the resulting sample 1a (Form I), a compound represented by formula (X) below was detected. The relative peak area of the compound of formula (X) was 0.27 if the sum of the peak areas of compound 1A, the compound of formula (X), and other degradation products of compound 1A is taken as 100. It can be said that the content of the compound of formula (X) or a sodium salt thereof in sample 1a (Form I) is sufficiently low.

[Chemical Formula 30]

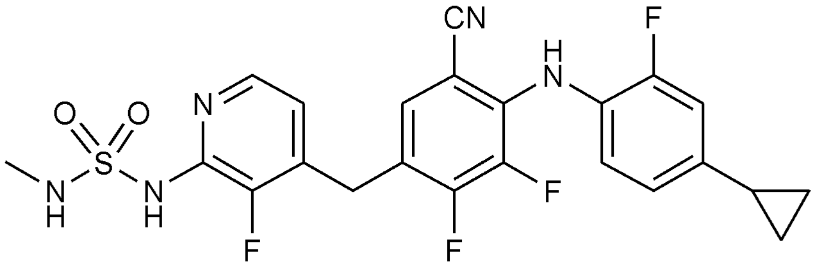

(X)

HPLC retention time: 8.81 min (analysis conditions C)
MS ($ESI^+$) m/z: 506 $[M+H]^+$

(4-2) Preparation of sample 1b (Form I)

(1) Synthesis of N-[4-[[*tert*-butyl(dimethyl)silyl]oxymethyl]-3-fluoropyridin-2-yl]aceta mide

tert-Butyl-[(2-chloro-3-fluoropyridin-4-yl)methoxy]-dimethylsil ane (180 g, 653 mmol), Xantphos (22.7 g, 39.2 mmol), potassium carbonate (135 g, 979 mmol), acetamide (77.1 g, 1.31 mol), and 2-methyl-2-butanol (540 mL) were added to a reaction vessel, and then this was deaerated under reduced pressure and purged with nitrogen. After then adding tris(dibenzylideneacetone)dipalladium(0) (14.9 g, 16.3 mmol) and toluene (540 mL), deaeration under reduced pressure and purging with nitrogen were further performed. The mixture was warmed to an external temperature of 120°C under a nitrogen atmosphere and stirred for 7 hours. The external temperature was cooled to room temperature, and the reaction mixture was filtered and washed with toluene (450 mL). Active carbon (9.00 g, 749 mmol) was added to the filtrate and the mixture was stirred for 1 hour at room temperature. The reaction mixture was then filtered and washed twice with toluene (270 mL the first time and 180 mL the second time) to give a crude product of N-[4-[[*tert*-butyl(dimethyl)silyl]oxymethyl]-3-fluoropyridin-2-yl]aceta mide as a toluene solution.
LCMS m/z: 299 $[M+H]^+$
HPLC retention time: 0.81 min (analysis conditions B)

(2) Synthesis of N-[3-fluoro-4-(hydroxymethyl)pyridin-2-yl] acetamide methanesulfonate (compound 13A)

[Chemical Formula 31]

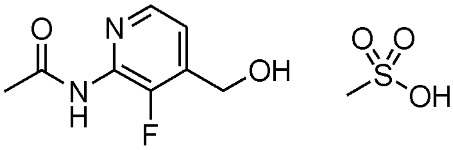

The resulting toluene solution of N-[4-[[*tert*-butyl(dimethyl)silyll]oxymethyl]-3-fluoropyridin-2-yl]aceta mide, toluene (175 mL), and MeOH (195 mL) were added to a reaction vessel, and this was deaerated under reduced pressure and purged with nitrogen. Methanesulfonic acid (188 g, 1.96 mol) was added dropwise at an external temperature of 10°C and the mixture was stirred for 2 hours at room temperature. The reaction mixture was cooled to an external temperature of 0°C and stirred for 3 hours. The precipitate was filtered off and washed with a cooled liquid mixture of toluene (312 mL) and MeOH (78 mL). The filtered off solid and a liquid mixture of toluene (1.1 L) and EtOH (492 mL) were added to a reaction vessel and stirred at an external temperature of 0°C for 1 hour. The solid was filtered off, washed with a liquid mixture of toluene (281 mL) and EtOH (117 mL), and dried at an external temperature of 40°C under reduced pressure to give compound 13A (149 g, 81% yield).
LCMS m/z: 185 $[M+H]^+$
HPLC retention time: 0.30 min (analysis conditions D)

(3) Synthesis of (2-acetamide-3-fluoropyridin-4-yl)methyl methyl carbonate (compound 14A)

[Chemical Formula 32]

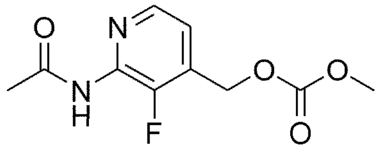

DMAP (52.3 g, 428 mmol) was added at room temperature to a reaction vessel in which N-[3-fluoro-4-(hydroxymethyl)pyridin-2-yl] acetamide methanesulfonate (compound 13A) (50.0 g, 178 mmol) and 2-MeTHF (750 mL) had been placed. The external temperature was cooled to 0°C and methyl chloroformate (21.9 g, 232 mmol) was added. Then the temperature was increased to room temperature and the mixture was stirred. The precipitated solid was filtered off and the filtrate was concentrated under reduced pressure at an external temperature of 40°C. Ethyl acetate (300 mL) was added to the concentrated residue to dissolve it at room temperature, and then DIPEA (31.2 mL, 178 mmol), heptane (150 mL) and seed crystals were added. After confirming precipitation of crystals, heptane (1 L) was added. The slurry was cooled to an external temperature of 0°C, and then the crystals were filtered off and washed with a liquid mixture of ethyl acetate/ heptane (2/7). They were dried at an external temperature of 40°C under reduced pressure to give compound 14A (31.3 g, 72% yield) as a colorless solid.

LCMS m/z: 243 $[M+H]^+$

HPLC retention time: 0.37 min (analysis conditions B)

(4) Synthesis of 5-[(2-acetamide-3-fluoropyridin-4-yl)methyl]-2-(4-cyclopropyl-2-fluoro anilino)-3,4-difluorobenzamide (compound 15A)

[Chemical Formula 33]

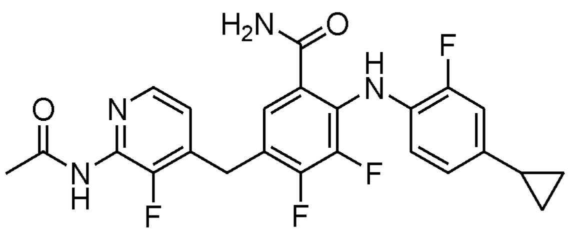

5-Bromo-2-(4-cyclopropyl-2-fluoroanilino)-3,4-difluorobenzami de (compound 9A) (10.0 g, 26.0 mmol), bis(pinacolato)diboron (7.3 g, 28.6 mmol), potassium acetate (7.6 g, 77.9 mmol), and 2-MeTHF (150 mL) were added to a reaction vessel, and this was deaerated under reduced pressure and purged with nitrogen. (2-Dicyclohexylphosphino-2',4',6'-triisopropyl-1,1'-biphenyl) [2-(2'-am ino-1,1'-biphenyl)]palladium(II) methanesulfonate (440 mg, 0.52 mmol) was added, and deaeration under reduced pressure and purging with nitrogen were further performed. The mixture was warmed to an external temperature of 80°C under a nitrogen atmosphere and stirred for 6 hours. The external temperature was cooled to room temperature, potassium carbonate (10.8 g, 77.9 mmol) was added, and deaeration under reduced pressure and purging with nitrogen were performed.

(2-Dicyclohexylphosphino-2',4',6'-triisopropyl-1,1' -biphenyl) [2-(2' -am ino-1,1'-biphenyl)]palladium(II) methanesulfonate (1.1 g, 1.3 mmol) was added, and after deaeration under reduced pressure and purging with nitrogen, a solution of (2-acetamide-3-fluoropyridin-4-yl)methyl methyl carbonate (compound 14A) (12.6 g, 51.9 mmol) in 2-MeTHF (150 mL) was added. The mixture was warmed to an external temperature of 70°C under a nitrogen atmosphere, and after adding water (935 μL, 51.9 mmol) three times at 20 minute intervals, the mixture was stirred for 20 minutes. Water (7.0 mL) was further added dropwise, the mixture was stirred for 2 hours, a solution prepared from N-acetylcysteine (847 mg, 5.2 mmol) and water (150 mL) was added, and the mixture was stirred for 1 hour. After cooling to an external temperature of 40°C, the aqueous layer was discharged. The organic layer was washed with a 15% sodium chloride

aqueous solution (150 mL), the insoluble portion was filtered, and concentration was carried out under reduced pressure. MeCN (500 mL) was added to the resulting concentrated residue and heated to an external temperature of 100°C to dissolve it, and then the temperature was cooled to room temperature. Crystals were filtered off, washed with MeCN (200 mL), and dried at an external temperature of 40°C under reduced pressure to give compound 15A (8.34 g, 68% yield).

LCMS m/z: 471 $[M-H]^-$
HPLC retention time: 0.74 min (analysis conditions B)

(5) Synthesis of 5-[(2-amino-3-fluoropyridin-4-yl)methyl]-2-(4-cyclopropyl-2-fluoroanil ino)-3,4-difluorobenzamide (compound 5A)

[Chemical Formula 34]

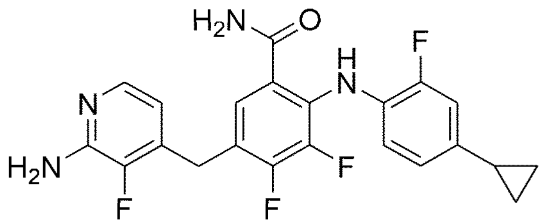

MeOH (3 mL) and 5M hydrochloric acid (0.42 mL, 2.1 mmol) were added to a reaction vessel in which 5-[(2-acetamide-3-fluoropyridin-4-yl)methyl]-2-(4-cyclopropyl-2-fluoro anilino)-3,4-difluorobenzamide (compound 15A) (100 mg, 0.21 mmol) had been placed, and the mixture was stirred at an external temperature of 50°C for 6 hours. The reaction mixture was cooled to room temperature, and a 2 M sodium hydroxide aqueous solution (1.1 mL, 2.1 mmol) was added. Water (0.5 mL) was added to the resulting slurry, and crystals were filtered off. They were washed with a liquid mixture of MeOH/water (3/2) and dried at an external temperature of 40°C under reduced pressure to give compound 5A (77.7 mg, 85% yield) as a colorless solid.

LCMS m/z: 431 $[M+H]^+$
HPLC retention time: 0.61 min (analysis conditions B)

(6) Synthesis of 2-(4-cyclopropyl-2-fluoroanilino)-3,4-difluoro-5-[[3-fluoro-2-(methylsu lfamoylamino)pyridin-4-yl]methyl]benzamide (compound 1A)

[Chemical Formula 35]

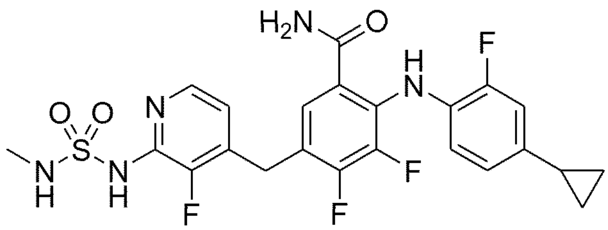

5-[(2-Amino-3-fluoropyridin-4-yl)methyl]-2-(4-cyclopropyl-2-fl uoroanilino)-3,4-difluorobenzamide (compound 5A) (100 mg, 0.232 mmol) was dissolved in anhydrous DMA (1 mL), and pyridine (56.4 μL, 0.697 mmol) was added. After then cooling to 0°C, methylsulfamoyl chloride (30.2 μL, 0.349 mmol) was added and the mixture was stirred for 1 hour. MeCN (0.6 mL), water (0.3 mL), and seed crystals (which had been obtained in Production Example A-1-1 below) (1 mg) were added to the reaction mixture, the temperature was increased to room temperature, water (0.7 mL) and MeCN (0.4 mL) were added, and the mixture was stirred for 20 hours. The precipitate was filtered off and washed with a liquid mixture of MeCN/water (1/1) to give compound 1A (93.1 mg, 77% yield) as a colorless solid.

LCMS m/z: 524 $[M+H]^+$
HPLC retention time: 1.13 min (analysis conditions E)

(7) Preparation of a sodium salt of 2-(4-cyclopropyl-2-fluoroanilino)-3,4-difluoro-5-[[3-fluoro-2-(methylsu lfamoylamino)pyridin-4-yl]methyl]benzamide (compound 1A)

(i) Preparation of sample 1b (Form I)

Acetone (10.6 mL) and DMSO (1.51 mL) were added to 2-(4-cyclopropyl-2-fluoroanilino)-3,4-difluoro-5-[[3-fluoro-2-(methylsu lfamoylamino)pyridin-4-yl]methyl]benzamide (compound 1A) (3.03 g), and this was dissolved at room temperature. A 20% sodium ethoxide EtOH solution (3.03 mL) and seed crystals of a sodium salt of compound 1A (sample 1c mentioned below) were added to the solution, and the mixture was stirred at room temperature for 1 hour. Then EtOH (15.1 mL) was added and the mixture was stirred at room temperature for 4 hours. Then EtOH (15.1 mL) was further added, and the mixture was stirred at room temperature for 4 hours to give a sodium salt of compound 1A (2.74 g) as powdered crystals (sample 1b (Form I)).

(ii) Preparation of sample 1c

A 20% sodium ethoxide EtOH solution (0.054 mL) and methyl isobutyl ketone (0.161 mL) were added to compound 1A (53.6 mg), and the mixture was stirred at room temperature for 30 minutes. Then methyl isobutyl ketone (0.161 mL) was added and the mixture was stirred at 60°C for 4 days. Then DMSO (0.054 mL) was added, and the mixture was stirred at 60°C for 5 hours to give a sodium salt of compound 1A (25.6 mg) as powdered crystals (sample 1c).

(4-3) Powder X-ray diffraction analysis

Sample 1a (Form I) was subjected to powder X-ray diffraction analysis under the following conditions.

Apparatus: Empyrean (PANalytical)
Anti-cathode: Cu
Tube voltage: 45 kV
Tube current: 40 mA
Scan mode: Continuous
Step width: 0.0262606°
Scan axis: 2θ
Sampling time per step: 5.100 seconds
Scan range: 3° to 25°

Sample 1b (Form I) and sample 1c were subjected to powder X-ray diffraction analysis under the following conditions.

Apparatus: SmartLab, D/Tex Ultra detector (Rigaku Corp.)
Anti-cathode: Cu
Tube voltage: 45 kV
Tube current: 200 mA
Sampling width: 0.02°

The results of powder X-ray diffraction analysis are shown in Figs. 1 to 3. Fig. 1 shows a powder X-ray diffraction pattern of sample 1a (Form I). Fig. 2 shows a powder X-ray diffraction pattern of sample 1b (Form I). Fig. 3 shows a powder X-ray diffraction pattern of sample 1c. In Figs. 1 to 3, the horizontal axis (X-axis) represents the diffraction angle 2θ (°) and the vertical axis (Y-axis) represents the diffraction intensity.

[Reference Examples]

With regard to specific aryl amide derivatives, reference examples (production examples and test examples) are presented below. Among the aryl amide derivatives described in the reference examples below (compounds A-1, A-2, A-8, A-18, A-20, A-25, A-27, A-33, B-1, E-1, E-7, E-13, H-1, H-3, H-4, I-1, J-1, K-10, L-1, M-1, N-1, N-2 and P-1), compounds A-1, A-2, A-8, A-18, A-20, A-25, A-27, A-33, B-1 and I-1 are compounds of general formula (1) above (compound A-1 being also referred to as compound 1A herein).

[Production Examples]

In the production examples below, NMR analysis was conducted using an AVANCE III HD400 (400 MHz) by Bruker Co. The NMR data were shown in ppm (parts per million) (δ) and the deuterium lock signal from the sample solvent was used as a reference.

The mass spectrum data were obtained using an ultra-high performance liquid chromatography (Nexera UC)-equipped single quadrupole mass spectrometer (LCMS-2020) by Shimadzu Corp. or an Acquity ultra-high performance liquid chromatography (UPLCor UPLC I-Class)-equipped single quadrupole mass spectrometer (SQD or SQD2) by Waters Co.

High-performance liquid chromatography (HPLC) analysis was conducted using one of the analysis conditions listed in Table 5 below.

[Table 5-1]

Table 5

| Analysis conditions | Apparatus | Column | Column temperature | Detection wavelength (PDA) |
|---|---|---|---|---|
| E | Nexera UC LCMS-2020 | Ascentis Express C18 2.1 mm I.D. × 50 mm L, 2.7 μm | 35°C | 210-400 nm |
| H | Nexera UC LCMS-2020 | XSelect CSH C18 2.1 mm I.D. × 50 mm L, 2.5 μm | 35°C | 210-400 nm |
| B | Acquity SQD/SQD2 | Ascentis Express C18 2.1 mm I.D. × 50 mm L, 2.7 μm | 35°C | 210-400 nm |
| I | Acquity SQD/SQD2 | Ascentis Express C18 2.1 mm I.D. × 50 mm L, 2.7 μm | 35°C | 210-400 nm |
| D | Acquity SQD/SQD2 | Ascentis Express C18 2.1 mm I.D. × 50 mm L, 5 μm | 35°C | 210-400 nm |
| F | Acquity SQD/SQD2 | Ascentis Express C18 2.1 mm I.D. × 50 mm L, 2.7 μm | 35°C | 210-400 nm |
| G | Acquity SQD/SQD2 | Ascentis Express C18 2.1 mm I.D. × 50 mm L, 2.7 μm | 35°C | 210-400 nm |

[Table 5-2]

Table 5 (cont.)

| Analysis conditions | Mobile phase | Gradient | | Flow rate (mL/min) |
|---|---|---|---|---|
| | | Time after injection (min) | A/B | |
| E | A) 0.05% TFA/ MeCN<br>B) 0.05% TFA/ $H_2O$ | 0-1.5<br>1.5-2.0 | 5/95 → 100/0<br>100/0 | 1 |
| H | A) 0.1% FA/MeCN<br>B) 0.1% FA/$H_2O$ | 0-1.75<br>1.75-2.00 | 5/95 → 100/0<br>100/0 | 1 |

(continued)

| Table 5 (cont.) | | | | |
|---|---|---|---|---|
| Analysis conditions | Mobile phase | Gradient | | Flow rate (mL/min) |
| | | Time after injection (min) | A/B | |
| B | A) 0.1% FA/MeCN<br>B) 0.1% FA/$H_2O$ | 0-1.0<br>1.0-1.4 | 5/95 → 100/0<br>100/0 | 1 |
| I | A) 0.1% FA/MeCN<br>B) 0.1% FA/$H_2O$ | 0-1.0<br>1.0-1.4 | 40/60 → 100/0<br>100/0 | 1 |
| D | A) MeOH<br>B) 10 mMAA/ $H_2O$ | 0-1.0<br>1.0-1.4 | 5/95 → 100/0<br>100/0 | 0.9 |
| F | A) 0.05% TFA/ MeCN<br>B) 0.05% TFA/ $H_2O$ | 0-1.0<br>1.0-1.4 | 5/95 → 100/0<br>100/0 | 1 |
| G | A) 0.05% FA/MeCN<br>B) 0.05% FA/ $H_2O$ | 0-1.0<br>1.0-1.4 | 5/95 → 100/0<br>100/0 | 1 |

Microwave reaction was conducted using an Initiator by Biotage Co. A snap cap reaction vial was used for the microwave reaction.
Commercially available reagents were used directly without further purification.
All of the nonaqueous reactions were conducted in anhydrous solvents.
Concentration under reduced pressure and solvent distillation were carried out using a rotary evaporator.
As used in the production examples below, "production example for compound A-1" means Production Example A-1-1 and "production example for compound a9" means Production Example a9-1.

Compound a1:

Methyl 3,4-difluoro-2-(2-fluoro-4-iodoanilino)-5-formylbenzoate

[Chemical Formula 36]

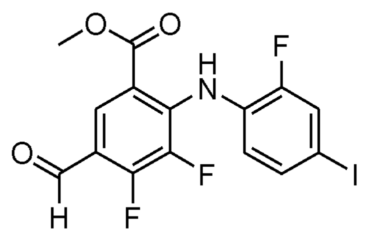

A mixed suspension of 3,4-difluoro-2-((2-fluoro-4-iodophenyl)amino)-5-formylbenzoic acid (5.50 g, 13.1 mmol) in toluene (44 mL) and MeOH (11 mL) was cooled to 0°C, a 10% diazomethyltrimethylsilane hexane solution (21.8 mL, 13.1 mmol) was added, and the mixture was stirred for 64 hours at room temperature. Acetic acid (0.748 mL) was added to the reaction mixture, which was then concentrated under reduced pressure. The resulting residue was purified by trituration (hexane/ethyl acetate) to give the title compound (5.01 g, 88%) as a colorless solid.
LCMS m/z: 436 $[M+H]^+$
HPLC retention time: 1.00 min (analysis conditions I)

Compound a2:

Methyl

3,4-difluoro-2-(2-fluoro-4-iodoanilino)-5-[(E)-[(4-methylphenyl)sulfon ylhydrazinylidene]methyl]benzoate

[Chemical Formula 37]

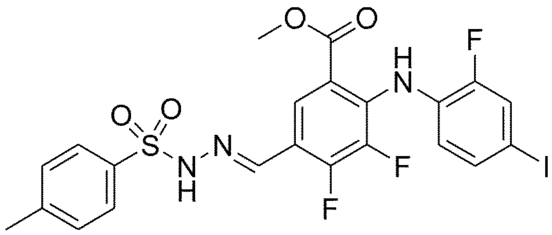

4-Methylbenzenesulfonyl hydrazide (2.14 g, 11.5 mmol) was added to a suspension of methyl 3,4-difluoro-2-(2-fluoro-4-iodoanilino)-5-formylbenzoate (compound a1, 5.00 g, 11.5 mmol) in EtOH (100 mL), and the mixture was stirred for 3 hours at room temperature. The reaction mixture was concentrated under reduced pressure, and then hexane (150 mL) was added. The mixture was cooled to 0°C and filtered, and then washed with hexane (30 mL) to give the title compound (7.05 g, quant.) as a solid.
LCMS m/z: 604 $[M+H]^+$
HPLC retention time: 1.06 min (analysis conditions I)

Compound a3:

N-(2,4-Dimethoxybenzyl)-3-fluoro-4-iodopyridine-2-amine

[Chemical Formula 38]

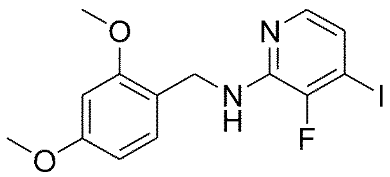

Triethylamine (3.63 mL, 26.0 mmol) and 1-(2,4-dimethoxyphenyl)methaneamine (3.26 mL, 21.7 mmol) were added to a solution of 2,3-difluoro-4-iodopyridine (2.09 g, 8.67 mmol) in NMP (32 mL), and the mixture was stirred for 1.5 hours at 100°C. Water was added to the reaction mixture, and extraction was performed with ethyl acetate. The organic layer was washed with 13% brine, dried over anhydrous sodium sulfate and, after filtering off the drying agent, concentrated under reduced pressure. The resulting residue was purified by silica gel column chromatography (hexane/ethyl acetate) to give the title compound (3.20 g, 95%) as an oil.
LCMS m/z: 389 $[M+H]^+$
HPLC retention time: 0.94 min (analysis conditions B)

Compound a4:

[2-[(2,4-Dimethoxyphenyl)methylamino]-3-fluoropyridin-4-yl]boronic acid

[Chemical Formula 39]

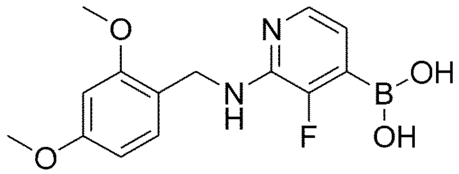

A 1,4-dioxane solution (27 mL) of N-(2,4-dimethoxybenzyl)-3-fluoro-4-iodopyridine-2-amine (compound a3, 2.70 g, 6.96 mmol), [1,1-bis(diphenylphosphino)ferrocene]dichloropalladium(II) dichloromethane addition product (568 mg, 0.696 mmol), potassium acetate (2.05 g, 20.9 mmol) and bis(pinacolato)diboron (2.65 g, 10.4 mmol) was stirred under a nitrogen atmosphere for 5 hours at 90°C and then for 19 hours at 110°C. The reaction mixture was concentrated under reduced pressure, and the resulting residue was purified by reversed-phase column chromatography (0.1% formic acid aqueous solution/ 0.1% formic acid acetonitrile solution) to give the title compound (2.07 g, 97%) as an oil.

LCMS m/z: 307 $[M+H]^+$

HPLC retention time: 0.44 min (analysis conditions B)

Compound a5:

Methyl

5-[[2-[(2,4-dimethoxyphenyl)methylaminol-3-fluoropyridin-4-yl]methy l]-3,4-difluoro-2-(2-fluoro-4-iodoanilino)benzoate

[Chemical Formula 40]

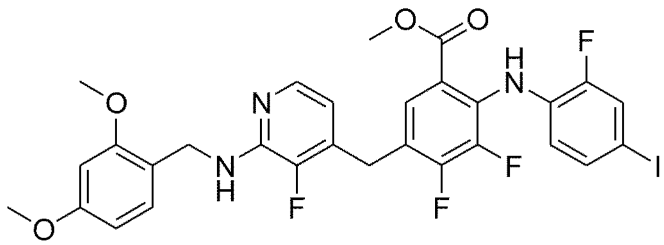

A 1,4-dioxane suspension (59 mL) of methyl 3,4-difluoro-2-(2-fluoro-4-iodoanilino)-5-[(E)-[(4-methylphenyl)sulfon ylhydrazinylidene]methyl]benzoate (compound a2, 1.30 g, 2.16 mmol), [2-[(2,4-dimethoxyphenyl)methylamino]-3-fluoropyridin-4-yl]boronic acid (compound a4, 1.98 g, 6.46 mmol) and potassium carbonate (357 mg, 2.59 mmol) was stirred under a nitrogen atmosphere for 2.5 hours at 100°C and then for 3 hours at 110°C. Ethyl acetate was added to the reaction mixture, which was then washed with water and 13% brine. The organic layer was dried over anhydrous sodium sulfate and, after filtering off the drying agent, concentrated under reduced pressure. The resulting residue was purified by silica gel column chromatography (hexane/ ethyl acetate) to give the title compound (524 mg, 36%) as a foam.

LCMS m/z: 682 $[M+H]^+$

HPLC retention time: 1.03 min (analysis conditions I)

Compound a6:

Methyl

5-(2-amino-3-fluoropyridin-4-yl)methyl]-3,4-difluoro-2-(2-fluoro-4-io doanilino)benzoate

[Chemical Formula 41]

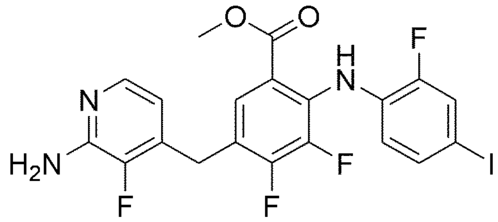

A DCM solution (16 mL) of methyl 5-[[2-[(2,4-dimethoxyphenyl)methylamino]-3-fluoropyridin-4-yl]methy l]-3,4-difluoro-2-(2-fluoro-4-iodoanilino)benzoate (compound a5, 523 mg, 0.768 mmol) was cooled to 0°C, trifluoroacetic acid (15.7 mL) was added, and the mixture was stirred for 1 hour at room temperature. The reaction mixture was concentrated under reduced pressure, and the resulting residue was purified by reversed-phase column chromatography (0.05% trifluoroacetic acid aqueous solution/ 0.05% trifluoroacetic acid acetonitrile solution) to give the title compound (321 mg, 79%) as an oil.

LCMS m/z: 532 $[M+H]^+$
HPLC retention time: 0.55 min (analysis conditions I)

Compound a7:

5-((2-Amino-3-fhioropyridin-4-yl)methyl)-3,4-difluoro-2-((2-fluoro-4-i odophenyl)amino)benzoic acid hydrochloride

[Chemical Formula 42]

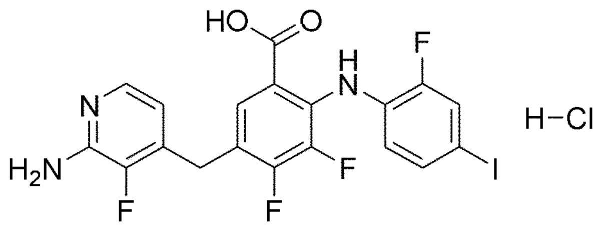

A mixed solution of methyl 5-[(2-amino-3-fluoropyridin-4-yl)methyl]-3,4-difluoro-2-(2-fluoro-4-io doanilino)benzoate (compound a6, 4.00 g, 7.53 mmol) in THF (64 mL) and water (32 mL) was cooled to 0°C, lithium hydroxide monohydrate (948 mg, 22.6 mmol) was added, and the mixture was stirred for 3.5 hours at room temperature. After cooling to 0°C, 5 M hydrochloric acid (15.1 mL) was added to the reaction mixture, which was then concentrated under reduced pressure. The resulting residue was washed with water and TBME to give the title compound (4.20 g, quant.) as a violet compound.

LCMS m/z: 518 $[M+H]^+$
HPLC retention time: 0.68 min (analysis conditions B)

Compound a8:

5-((2-Amino-3-fluoropyridin-4-yl)methyl)-3,4-difluoro-2-((2-fluoro-4-i odophenyl)amino)benzamide

[Chemical Formula 43]

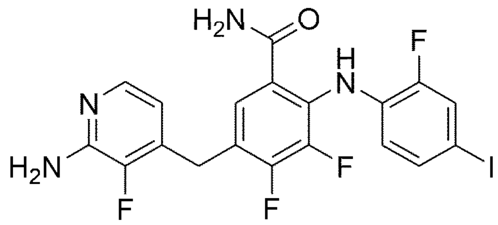

An anhydrous DMF solution (3.6 mL) of 5-((2-amino-3-fluoropyridin-4-yl)methyl)-3,4-difluoro-2-((2-fluoro-4-io dophenyl)amino)benzoic acid hydrochloride (compound a7, 200 mg, 0.361 mmol) was cooled to 0°C, HOOBt (67.8 mg, 0.415 mmol) and EDC·HCl (80.0 mg, 0.415 mmol) were added, and the mixture was stirred for 1.5 hours at room temperature. After further adding HOOBt (8.8 mg, 0.054 mmol) and EDC·HCl (10.4 mg, 0.054 mmol) and stirring at room temperature for 1 hour, a 7 M ammonia MeOH solution (0.103 mL, 0.722 mmol) and DIPEA (0.189 mL, 1.08 mmol) were added at 0°C and the mixture was stirred for 30 minutes at room temperature. Water and a saturated sodium hydrogen carbonate aqueous solution were added at 1:1 to the reaction mixture, and extraction was performed with ethyl acetate. The organic layer was dried over anhydrous sodium sulfate and, after filtering off the drying agent, concentrated under reduced pressure. The resulting residue was dissolved in ethyl acetate (1 mL), and hexane (10 mL) was added. The resulting solid was filtered off and washed with hexane to give the title compound (162 mg, 87%) as a colorless solid.

LCMS m/z: 517 $[M+H]^+$

HPLC retention time: 0.64 min (analysis conditions B)

Compound a9:

5-((2-Amino-3-fluoropyridin-4-yl)methyl)-2-((4-cyclopropyl-2-fluorop henyl)amino)-3,4-difluorobenzamide

[Chemical Formula 44]

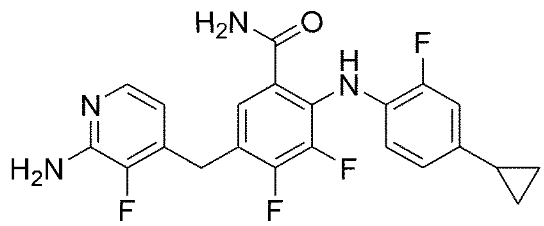

Production Example a9-1:

Tetrakis(triphenylphosphine)palladium(0) (11.2 mg, 9.68 μmol) and 0.5 M cyclopropylzinc bromide (1.94 mL, 0.969 mmol) were added to an anhydrous THF solution (1.9 mL) of 5-((2-amino-3-fluoropyridin-4-yl)methyl)-3,4-difluoro-2-((2-fluoro-4-io dophenyl)amino)benzamide (compound a8, 100 mg, 0.194 mmol), and the mixture was stirred for 2.5 hours at room temperature under a nitrogen atmosphere. Ethyl acetate (5 mL) was added to the reaction mixture, which was then filtered with Celite and washed with ethyl acetate (3 mL). The filtrate was washed with water and saturated brine, and the organic layer was dried over anhydrous sodium sulfate and, after filtering off the drying agent, concentrated under reduced pressure. Dichloromethane/hexane (1/10, 11 mL) was added to the resulting residue, and the solid was filtered off and washed with hexane (3 mL) to give compound a9 (63.4 mg, 76%) as a colorless solid.

LCMS m/z: 431 $[M+H]^+$

HPLC retention time: 0.61 min (analysis conditions B)

Compound r1:

4-Nitrophenyl methylsulfamate

[Chemical Formula 45]

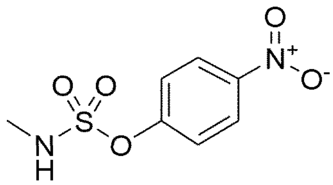

A dichloromethane solution (60 mL) of 4-nitrophenol (5.00 g, 35.9 mmol) and triethylamine (11.3 mL, 81.0 mmol) was cooled to -78°C, a dichloromethane solution (15 mL) of methylsulfamoyl chloride (5.82 g, 44.9 mmol) was added, and the mixture was stirred for 1.5 hours at -78°C. The reaction mixture was concentrated under reduced pressure, and the resulting residue was purified by silica gel column chromatography (hexane/ ethyl acetate) and reversed-phase column chromatography (0.1% formic acid aqueous solution/ 0.1% formic acid acetonitrile solution) to give the title compound (5.51 g, 66%) as a colorless solid.
HPLC retention time: 0.63 min (analysis conditions B)
$^1$H-NMR (400 MHz, $CDCl_3$) δ: 8.31 (2H, m), 7.46 (2H, m), 4.68 (1H, m), 3.00 (3H, d, J = 5.4 Hz).

Compound A-1:

2-(4-Cyclopropyl-2-fluoroanilino)-3,4-difluoro-5-[[3-fluoro-2-(methyls ulfamoylamino)pyridin-4-yl]methyl]benzamide

[Chemical Formula 46]

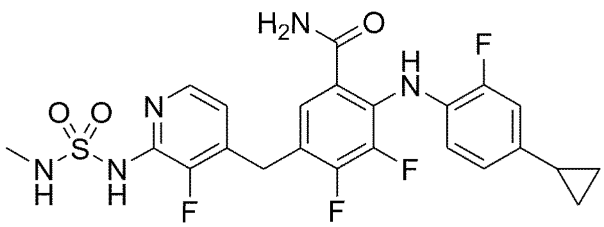

Production Example A-1-1:

After dissolving 5-((2-amino-3-fluoropyridin-4-yl)methyl)-2-((4-cyclopropyl-2-fluoroph enyl)amino)-3,4-difluorobenzamide (compound a9, 2.47 g, 5.74 mmol) in anhydrous DMF (28.7 mL), pyridine (2.78 mL, 34.4 mmol) and 4-nitrophenyl methylsulfamate (compound r1, 4.00 g, 17.2 mmol) were added and the mixture was stirred for 2.5 hours at 40°C. The reaction mixture was cooled to room temperature, and water (24.7 mL) was added. After further adding acetonitrile (3 mL) and water (19.8 mL) and stirring for 10 minutes, the solid was filtered off. The resulting solid was washed with water/acetonitrile (1/1, 49.4 mL) to give compound A-1 (2.56 g, 85%) as a colorless solid.
LCMS m/z: 524 $[M+H]^+$
HPLC retention time: 1.13 min (analysis conditions E)

Compound a10:

5-((2-Amino-3-fluoropyridin-4-yl)methyl)-N-cyclopropyl-3,4-difluoro-2-((2-fluoro-4-iodophenyl)amino)benzamide

[Chemical Formula 47]

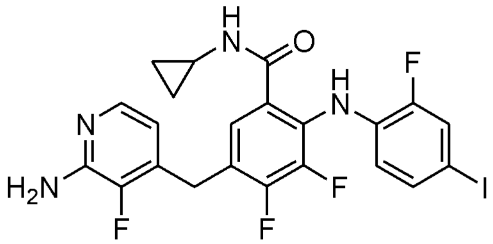

After dissolving 5-((2-amino-3-fluoropyridin-4-yl)methyl)-3,4-difluoro-2-((2-fluoro-4-io dophenyl)amino)benzoic acid hydrochloride (compound a7, 100 mg, 0.193 mmol) in anhydrous DMF (1 mL), HOOBt (63.1 mg, 0.387 mmol) and EDC·HCl (74.1 mg, 0.387 mmol) were added at room temperature. After stirring at room temperature for 3 hours, aminocyclopropane (33.1 mg, 0.580 mmol) and DIPEA (0.101 mL, 0.580 mmol) were added and the mixture was stirred for 1 hour at room temperature. The reaction mixture was purified by reversed-phase column chromatography (0.1% formic acid aqueous solution/ 0.1% formic acid acetonitrile solution) to give the title compound (103 mg, 96%) as a brown solid.

LCMS m/z: 557 $[M+H]^+$

HPLC retention time: 0.73 min (analysis conditions B)

Compound A-2:

N-Cyclopropyl-3,4-difluoro-2-(2-fluoro-4-iodoanilino)-5-[[3-fluoro-2-( methylsulfamoylamino)pyridin-4-yl]methyl]benzamide

[Chemical Formula 48]

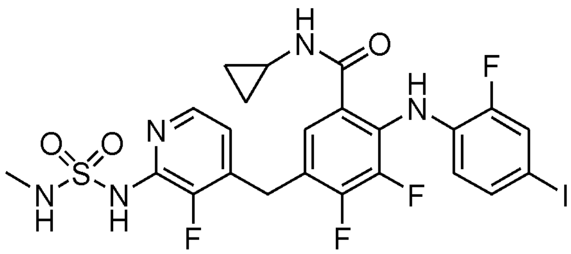

The title compound was synthesized from 5-((2-amino-3-fluoropyridin-4-yl)methyl)-N-cyclopropyl-3,4-difluoro-2 -((2-fluoro-4-iodophenyl)amino)benzamide (compound a10) under the same conditions as the production example for compound A-1.

LCMS m/z: 650 $[M+H]^+$

HPLC retention time: 1.65 min (analysis conditions H)

Compound a12:

5-((2-Amino-3-fluoropyridin-4-yl)methyl)-N-(*tert*-butoxy)-3,4-difluoro-2-((2-fluoro-4-iodophenyl)amino)benzamide

[Chemical Formula 49]

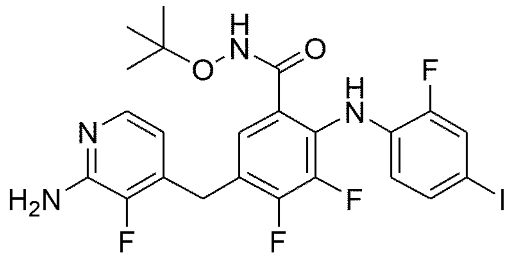

After dissolving 5-[(2-amino-3-fluoropyridin-4-yl)methyl]-3,4-difluoro-2-(2-fluoro-4-io doanilino)benzoic acid hydrochloride (compound a7, 100 mg, 0.181 mmol) in anhydrous DMF (0.9 mL), HOOBt (58.9 mg, 0.361 mmol) and EDC·HCl (69.2 mg, 0.361 mmol) were added and the mixture was stirred for 3.5 hours at room temperature. Next, *tert*-butoxyamine hydrochloride (68.1 mg, 0.542 mmol) and DIPEA (0.95 mL, 0.542 mmol) were added, and the mixture was stirred for 1.5 hours at room temperature. The reaction mixture was purified by reversed-phase column chromatography (0.1% formic acid aqueous solution/ 0.1% formic acid acetonitrile solution) to give the title compound (89 mg, 84%) as a colorless solid.

LCMS m/z: 589 $[M+H]^+$

HPLC retention time: 0.77 min (analysis conditions B)

Compound A-8:

3,4-Difluoro-2-(2-fluoro-4-iodoanilino)-5-[[3-fluoro-2-(methylsulfamoy lamino)pyridin-4-yl]methyl]-N-[(2-methylpropan-2-yl)oxy]benzamide

[Chemical Formula 50]

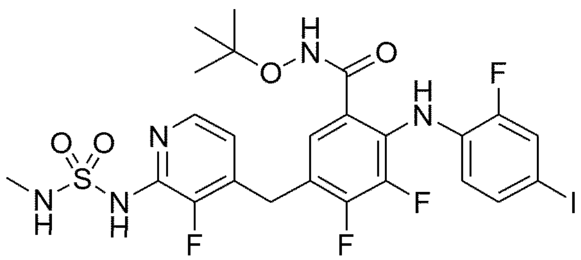

The title compound was synthesized from 5-((2-amino-3-fluoropyridin-4-yl)methyl)-N-(*tert*-butoxy)-3,4-difluoro-2-((2-fluoro-4-iodophenyl)amino)benzamide (compound a12) under the same conditions as the production example for compound A-1.

LCMS m/z: 682 $[M+H]^+$

HPLC retention time: 1.69 min (analysis conditions H)

Compound a16:

5-[(2-Amino-3-fluoropyridin-4-yl)methyl]-3,4-difluoro-2-(2-fluoro-4-m ethylsulfanylanilino)benzamide

[Chemical Formula 51]

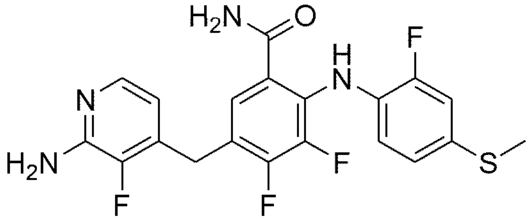

After dissolving 5-[(2-amino-3-fluoropyridin-4-yl)methyl]-3,4-difluoro-2-(2-fluoro-4-io doanilino)benzamide (compound a8, 30.0 mg, 0.058 mmol) in anhydrous 1,4-dioxane (0.3 mL), methylmercaptan sodium (12.2 mg, 0.174 mmol), DIPEA (30.4 μL, 0.174 mmol) and [(4,5-bis(diphenylphosphino)-9,9-dimethylxanthene)-2-(2'-amino-1,1'-biphenyl)]palladium(II) methanesulfonate (11.2 mg, 0.012 mmol) were added, and the mixture was stirred for 30 minutes at room temperature under a nitrogen atmosphere. The reaction mixture was purified by reversed-phase column chromatography (0.1% formic acid aqueous solution/ 0.1% formic acid acetonitrile solution) to give the title compound (15 mg, 59%) as a colorless solid.

LCMS m/z: 437 $[M+H]^+$

HPLC retention time: 0.60 min (analysis conditions B)

Compound A-18:

3,4-Difluoro-5-[[3-fluoro-2-(methylsulfamoylamino)pyridin-4-yl]methy l]-2-(2-fluoro-4-methylsulfanylanilino)benzamide

[Chemical Formula 52]

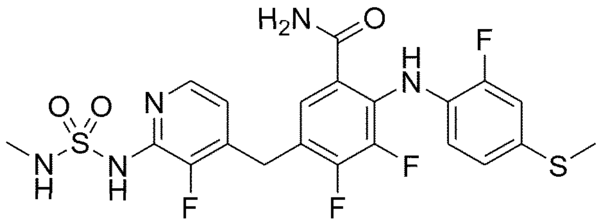

The title compound was synthesized from 5-[(2-amino-3-fluoropyridin-4-yl)methyl]-3,4-difluoro-2-(2-fluoro-4-me thylsulfanylanilino)benzamide (compound a16) under the same conditions as the production example for compound A-1.

LCMS m/z: 530 $[M+H]^+$

HPLC retention time: 1.09 min (analysis conditions E)

Compound a18:

5-[(2-Amino-3-fluoropyridin-4-yl)methyl]-3,4-difluoro-2-[2-fluoro-4-(2 -trimethylsilylethynyl)anilino]benzamide

[Chemical Formula 53]

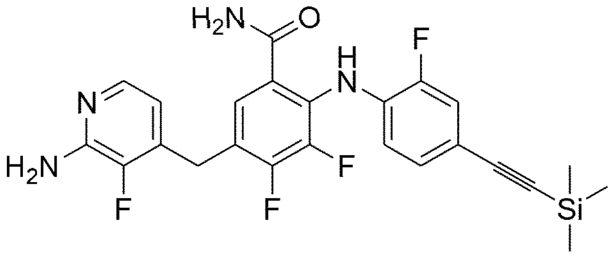

Triethylamine (31.7 mL, 228 mmol), trimethylsilylacetylene (1.43 mL, 10.3 mmol), bis(triphenylphosphine)palladium(II) dichloride (363 mg, 0.517 mmol) and copper(I) iodide (296 mg, 1.55 mmol) were added to an anhydrous THF solution (26 mL) of 5-[(2-amino-3-fluoropyridin-4-yl)methyl]-3,4-difluoro-2-(2-fluoro-4-io doanilino)benzamide (compound a8, 2.67 g, 5.17 mmol), and the mixture was stirred for 3 hours at room temperature. The reaction mixture was concentrated under reduced pressure, and the resulting residue was purified by reversed-phase column chromatography (0.1% formic acid aqueous solution/ 0.1% formic acid acetonitrile solution) to give the title compound (2.57 g, 83%) as a colorless solid.

LCMS m/z: 487 $[M+H]^+$

HPLC retention time: 0.84 min (analysis conditions G)

Compound a19:

5-[(2-Amino-3-fluoropyridin-4-yl)methyl]-2-(4-ethynyl-2-fluoroanilino) -3,4-difluorobenzamide

[Chemical Formula 54]

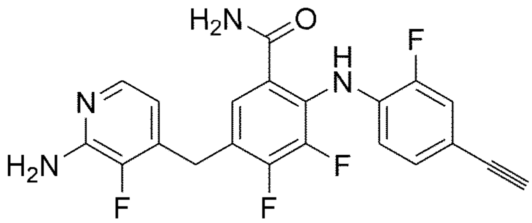

Potassium carbonate (17.0 mg, 0.123 mmol) was added to a MeOH solution (0.411 mL) of 5-[(2-amino-3-fluoropyridin-4-yl)methyl]-3,4-difluoro-2-[2-fluoro-4-(2-trimethylsilylethynyl)anilino]benzamide (compound a18, 20.0 mg, 0.041 mmol), and the mixture was stirred for 1.5 hours at room temperature. The reaction mixture was concentrated under reduced pressure, and the resulting residue was purified by reversed-phase column chromatography (0.1% formic acid aqueous solution/ 0.1% formic acid acetonitrile solution) to give the title compound (14 mg, 82%) as a colorless solid.

LCMS m/z: 415 $[M+H]^+$

HPLC retention time: 0.60 min (analysis conditions G)

Compound A-20:

2-(4-Ethynyl-2-fluoroanilino)-3,4-difluoro-5-[[3-fluoro-2-(propylsulfam oylamino)pyridin-4-yl]methyl]benzamide

[Chemical Formula 55]

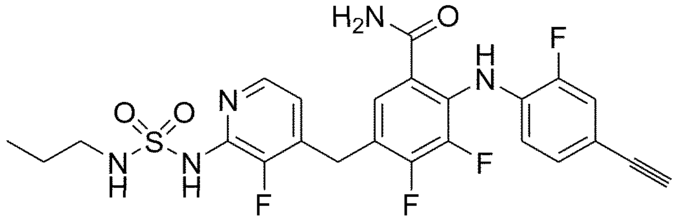

The title compound was synthesized from 5-[(2-amino-3-fluoropyridin-4-yl)methyl]-2-(4-ethynyl-2-fluoroanilino) -3,4-difluorobenzamide (compound a19) and the corresponding 4-nitrophenyl sulfamate under the same conditions as the production example for compound A-1.

LCMS m/z: 536 $[M+H]^+$
HPLC retention time: 1.18 min (analysis conditions E)

Compound A-25:

3,4-Difluoro-2-(2-fluoro-4-iodoanilino)-5-[[3-fluoro-2-(methylsulfamoy lamino)pyridin-4-yl]methyl]benzamide

[Chemical Formula 56]

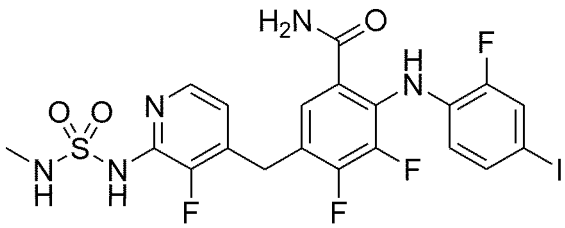

After dissolving 5-[(2-amino-3-fluoropyridin-4-yl)methyl]-3,4-difluoro-2-(2-fluoro-4-io doanilino)benzamide (compound a8, 10.0 mg, 0.019 mmol) in anhydrous DMA (0.1 mL), pyridine (2.3 μL, 0.029 mmol) and methylsulfamoyl chloride (2.5 μL, 0.029 mmol) were added at 0°C, and the mixture was stirred for 1 hour at room temperature. The reaction mixture was purified by reversed-phase column chromatography (0.1% formic acid aqueous solution/ 0.1% formic acid acetonitrile solution) to give the title compound (10.2 mg, 86%) as a colorless solid.

LCMS m/z: 610 $[M+H]^+$
HPLC retention time: 1.15 min (analysis conditions E)

Compound A-33:

3,4-difluoro-2-(2-fluoro-4-iodoanilino)-5-[[3-fluoro-2-(2-methoxyethyls ulfamoylamino)pyridin-4-yl]methyl]benzamide

[Chemical Formula 57]

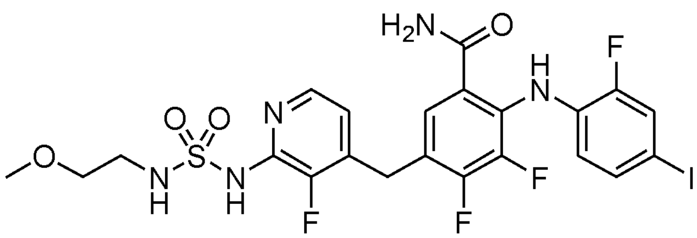

The title compound was synthesized from 5-[(2-amino-3-fluoropyridin-4-yl)methyl]-3,4-difluoro-2-(2-fluoro-4-io doanilino)benzamide (compound a8) and the corresponding sulfamoyl chloride under the same conditions as the production example for compound A-25.

LCMS m/z: 654 $[M+H]^+$
HPLC retention time: 1.17 min (analysis conditions E)

Compound a21:

5-[(2-Amino-3-fluoropyridin-4-yl)methyl]-2-(4-bromo-2-fluoroanilino)-3,4-difluorobenzamide

[Chemical Formula 58]

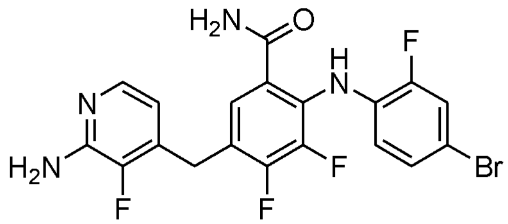

After dissolving 5-[(2-amino-3-fluoropyridin-4-yl)methyl]-3,4-difluoro-2-(2-fluoro-4-io doanilino)benzamide (compound a8, 60.0 mg, 0.116 mmol) in anhydrous DMF (1.2 mL), copper(I) bromide (83.0 mg, 0.581 mmol) was added and the mixture was stirred for 24 hours at 100°C. The reaction mixture was purified by preparative HPLC (5 μm TSK-gel ODS 80TS, 20 × 250 mm column (TOSOH), 0.1% formic acid aqueous solution/ 0.1% formic acid acetonitrile solution) to give the title compound (35.6 mg) as a solid.

LCMS m/z: 469 $[M+H]^+$
HPLC retention time: 0.61 min (analysis conditions B)

Compound A-27:

2-(4-Bromo-2-fluoroanilino)-3,4-difluoro-5-[[3-fluoro-2-(methylsulfam oylamino)pyridin-4-yl]methyl]benzamide

[Chemical Formula 59]

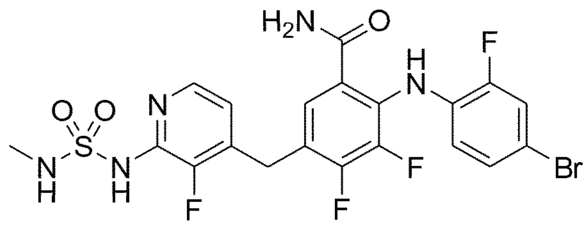

The title compound was synthesized from 5-[(2-amino-3-fluoropyridin-4-yl)methyl]-2-(4-bromo-2-fluoroanilino)-3,4-difluorobenzamide (compound a21) under the same conditions as the production example for compound A-25.

LCMS m/z: 562 $[M+H]^+$
HPLC retention time: 1.13 min (analysis conditions E)

Compound b1:

Methyl

3,4-difluoro-2-(2-fluoro-4-iodoanilino)-5-[[3-fluoro-2-(methylsulfamoyl amino)pyridin-4-yl]methyl]benzoate

[Chemical Formula 60]

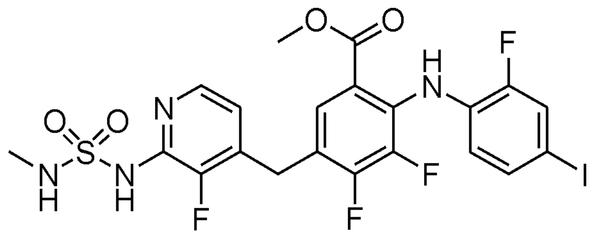

The title compound was synthesized from methyl 5-[(2-amino-3-fluoropyridin-4-yl)methyl]-3,4-difluoro-2-(2-fluoro-4-io doanilino)benzoate (compound a6) under the same conditions as the production example for compound A-25. However, anhydrous NMP was used instead of anhydrous DMA.

LCMS m/z: 436 $[M+H]^+$

HPLC retention time: 1.00 min (analysis conditions I)

Compound b2:

3,4-Difluoro-2-(2-fluoro-4-iodoanilino)-5-[[3-fluoro-2-(methylsulfamoy lamino)pyridin-4-yl]methyl]benzoic acid

[Chemical Formula 61]

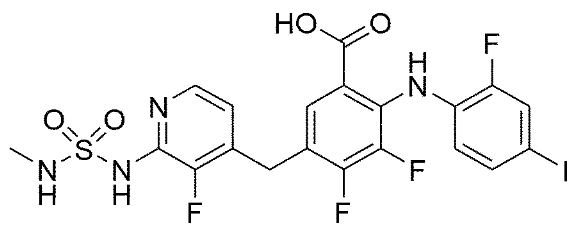

A mixed solution of methyl 3,4-difluoro-2-(2-fluoro-4-iodoanilino)-5-[[3-fluoro-2-(methylsulfamoyl amino)pyridin-4-yl]methyl]benzoate (compound b1, 158 mg, 0.253 mmol) in THF (4.8 mL) and water (2.4 mL) was cooled to 0°C, lithium hydroxide monohydrate (60.6 mg, 2.53 mmol) was added, and the mixture was stirred at room temperature for 2 hours. After then adding 2 M hydrochloric acid to the reaction mixture, extraction was performed with ethyl acetate. The organic layer was washed with saturated brine and dried over anhydrous sodium sulfate. After filtering off the drying agent, it was concentrated under reduced pressure to give the title compound (161 mg) as a foam.

LCMS m/z: 611 $[M+H]^+$

HPLC retention time: 0.67 min (analysis conditions I)

Compound B-1:

3,4-Difluoro-2-(2-fluoro-4-iodoanilino)-5-[[3-fluoro-2-(methylsulfamoy lamino)pyridin-4-yl]methyl]-N-(2-hydroxyethoxy)benzamide

[Chemical Formula 62]

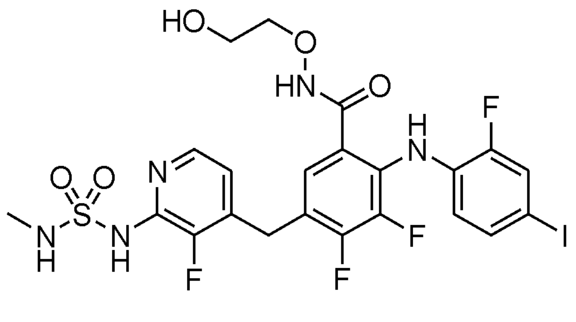

The title compound was synthesized from 3,4-difluoro-2-(2-fluoro-4-iodoanilino)-5-[[3-fluoro-2-(methylsulfamoyl amino)pyridin-4-yl]methyl]benzoic acid (compound b2) and the corresponding amine under the same conditions as the production example for compound a8.

LCMS m/z: 670 $[M+H]^+$

HPLC retention time: 1.07 min (analysis conditions E)

Compound c1:

3,4-Difluoro-2-(2-fluoro-4-iodoanilino)-5-[(E)-[(4-methylphenyl)sulfon ylhydrazinylidene]methyl]benzamide

[Chemical Formula 63]

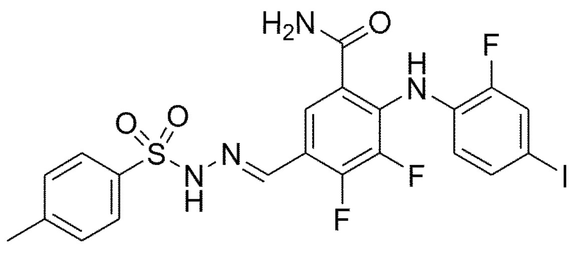

After adding 4-methylbenzenesulfonyl hydrazide (2.21 g, 11.9 mmol) to an anhydrous DMF solution (59 mL) of 3,4-difluoro-2-((2-fluoro-4-iodophenyl)amino)-5-formylbenzoic acid (5.00 g, 11.9 mmol), the mixture was stirred for 30 minutes at room temperature. Next, HOOBt (1.94 g, 11.9 mmol) and EDC·HCl (2.28 g, 11.9 mmol) were added and the mixture was stirred for 1.5 hours at room temperature. A 7 M ammonia MeOH solution (3.39 mL, 23.8 mmol) was added to the reaction mixture and stirring was continued for 30 minutes at room temperature, after which the solid was filtered off and washed with DMF (30 mL). Acetonitrile (90 mL) and 0.1 M hydrochloric acid (90 mL) were added to the filtrate, and the resulting solid was washed with a liquid mixture of acetonitrile/water to give the title compound (6.27 g, 90%) as a colorless solid.

LCMS m/z: 589 $[M+H]^+$

HPLC retention time: 0.90 min (analysis conditions B)

Compound c5:

5-Ethenyl-3,4-difluoro-2-(4-iodo-2-methylanilino)benzoic acid

[Chemical Formula 64]

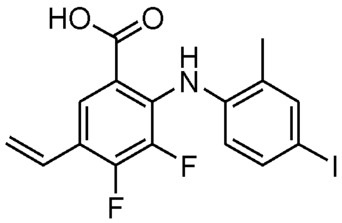

An anhydrous THF solution (1.8 ml) of 4-iodo-2-methylaniline 636 mg, 2.73 mmol was cooled to -78°C, and then a 1.3 M lithium bis(trimethylsilyl)amide THF solution (5.08 mL, 6.60 mmol) was added over 1 hour and the mixture was stirred for 1 hour. An anhydrous THF solution (3.9 mL) of 2,3,4-trifluoro-5-vinylbenzoic acid (460 mg, 2.28 mmol) was then added, and the mixture was stirred for 2 hours at 0°C. Water and 2 M hydrochloric acid were added to the reaction mixture and extraction was performed twice with ethyl acetate. The organic layer was washed with saturated brine, dried over anhydrous sodium sulfate and, after filtering off the drying agent, concentrated under reduced pressure. The resulting residue was suspended and washed in DCM to give the title compound (631 mg, 67%) as a brown solid.

LCMS m/z: 416 $[M+H]^+$

HPLC retention time: 0.99 min (analysis conditions D)

Compound c6:

3,4-Difluoro-5-formyl-2-(4-iodo-2-methylanilino)benzoic acid

[Chemical Formula 65]

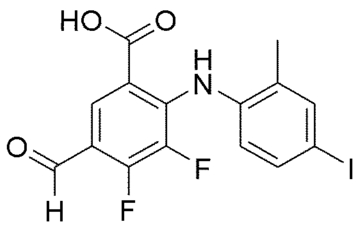

After adding a 1 M sodium hydrogen carbonate aqueous solution (3.02 mL, 3.02 mmol), sodium periodate (1.29 g, 6.03 mmol) and microcapsulated osmium(VIII) oxide (38.3 mg, 0.015 mmol) to an anhydrous THF solution (6.3 mL) of 5-ethenyl-3,4-difluoro-2-(4-iodo-2-methylanilino)benzoic acid (compound c5, 626 mg, 1.51 mmol), the mixture was stirred at room temperature for 6 hours. After then adding ethyl acetate to the reaction mixture, this was washed with 1 M hydrochloric acid and 0.2 M aqueous sodium thiosulfate solution. The organic layer was dried over anhydrous sodium sulfate and, after filtering off the drying agent, concentrated under reduced pressure. The resulting residue was suspended and washed in ethyl acetate/ hexane (1/25, 42 mL), and the solid was filtered off. The resulting solid was washed with hexane to give the title compound (558 mg, 89%) as a colorless solid.

LCMS m/z: 418 $[M+H]^+$

HPLC retention time: 0.86 min (analysis conditions B)

Compound d1:

Methyl

5-[(3-amino-2-fluorophenyl)methyl]-3,4-difluoro-2-(2-fluoro-4-iodoanil ino)benzoate

[Chemical Formula 66]

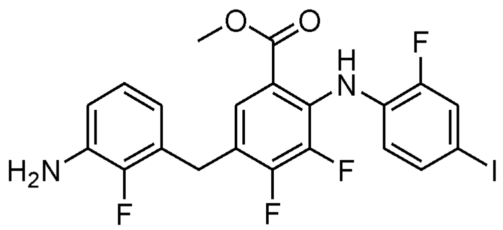

The title compound was synthesized from methyl 3,4-difluoro-2-(2-fluoro-4-iodoanilino)-5-[(E)-[(4-methylphenyl)sulfon ylhydrazinylidene]methyl]benzoate (compound a2) under the same conditions as the production example for compound a5. However, (3-amino-2-fluorophenyl)boronic acid hydrochloride was used instead of [2-[(2,4-dimethoxyphenyl)methylamino]-3-fluoropyridin-4-yl]boronic acid (compound a4).
LCMS m/z: 531 $[M+H]^+$
HPLC retention time: 0.96 min (analysis conditions I)

Compound E-1:

5-[[3-(Ethylsulfonylamino)-2-fluorophenyl]methyl]-3,4-difluoro-2-(2-fl uoro-4-iodoanilino)-N-methoxybenzamide

[Chemical Formula 67]

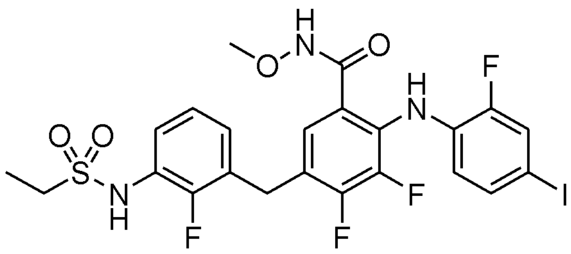

The title compound was synthesized from methyl 5-[(3-amino-2-fluorophenyl)methyl]-3,4-difluoro-2-(2-fluoro-4-iodoanil ino)benzoate (compound d1) and the corresponding sulfonyl chloride under the same conditions as the production examples for compound A-25, compound b2 and compound a12. However, pyridine was used as the solvent in the sulfonamidation step. Also, the corresponding amine was used instead of *tert*-butoxyamine hydrochloride, which was used in the production example for compound a12.
LCMS m/z: 638 $[M+H]^+$
HPLC retention time: 1.68 min (analysis conditions H)

Compound E-7:

3,4-Difluoro-2-(2-fluoro-4-iodoanilino)-5-[[2-fluoro-3-(methylsulfamoy lamino)phenyl]methyl]benzamide

[Chemical Formula 68]

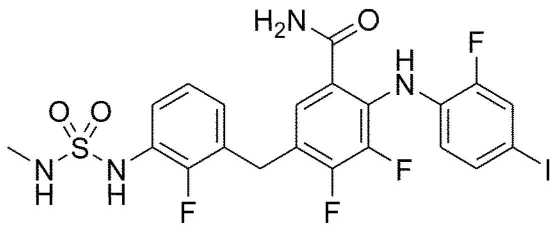

The title compound was synthesized from methyl 5-[(3-amino-2-fluorophenyl)methyl]-3,4-difluoro-2-(2-fluoro-4-iodoanil ino)benzoate (compound d1) under the same conditions as the production examples for compound A-25, compound b2 and compound a12. However, pyridine was used as the solvent in the sulfamidation step. Also, the corresponding amine was used instead of *tert*-butoxyamine hydrochloride, which was used in the production example for compound a12.

LCMS m/z: 609 $[M+H]^+$
HPLC retention time: 1.23 min (analysis conditions E)

Compound e20:

Methyl

3,4-difluoro-2-(2-fluoro-4-iodoanilino)-5-[[2-fluoro-3-(methanesulfona mide)phenyl]methyl]benzoate

[Chemical Formula 69]

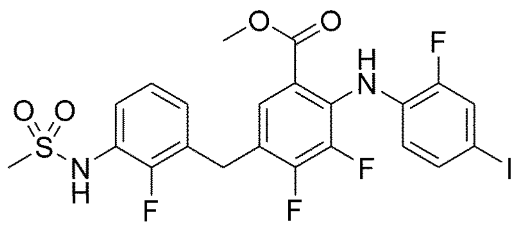

The title compound was synthesized from methyl 5-[(3-amino-2-fluorophenyl)methyl]-3,4-difluoro-2-(2-fluoro-4-iodoanil ino)benzoate (compound d1) and the corresponding sulfonyl chloride under the same conditions as the production example for compound A-25. However, pyridine was used as the solvent.

LCMS m/z: 609 $[M+H]^+$
HPLC retention time: 1.01 min (analysis conditions B)

Compound E-13:

3,4-Difluoro-2-(2-fluoro-4-iodoanilino)-5-[[2-fluoro-3-(methanesulfona mide)phenyl]methyl]benzamide

[Chemical Formula 70]

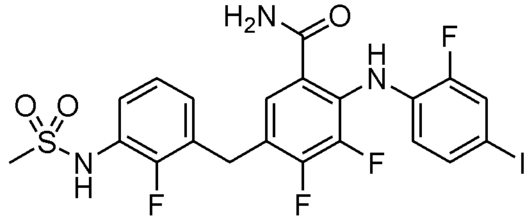

Lithium hydroxide monohydrate (7.9 mg, 0.19 mmol) was added to a mixed solution of methyl 3,4-difluoro-2-(2-fluoro-4-iodoanilino)-5-[[2-fluoro-3-(methanesulfona mide)phenyl]methyl]benzoate (compound e20, 23.0 mg, 0.038 mmol) in THF (0.7 mL) and water (0.3 mL), and the mixture was stirred at room temperature for 2 hours. The reaction mixture was concentrated under reduced pressure, and then 1 M hydrochloric acid (0.76 mL) was added and the mixture was further concentrated under reduced pressure. After then adding HOOBt (9.3 mg, 0.057 mmol) and EDC·HCl (10.9 mg, 0.057 mmol) to an anhydrous DMF solution (0.3 mL) of the resulting mixture, the resulting mixture was stirred at room temperature for 3 hours. After then adding a 7 M ammonia MeOH solution (22 μL, 0.15 mmol) at 0°C, stirring was continued for 30 minutes. A 10% trifluoroacetic acid aqueous solution (1 mL) was added to the reaction mixture, which was then purified by reversed-phase column chromatography (0.1% formic acid aqueous solution/ 0.1% formic acid acetonitrile solution) to give the title compound (19.7 mg, 97%) as a colorless solid.

LCMS m/z: 594 $[M+H]^+$

HPLC retention time: 1.61 min (analysis conditions H)

Compound h1:

Methyl

5-[(2-amino-3-fluoropyridin-4-yl)methyl]-2-[4-[3-(2-ethylhexaoxy)-3-o xopropyl]sulfanyl-2-fluoroanilino]-3,4-difluorobenzoate

[Chemical Formula 71]

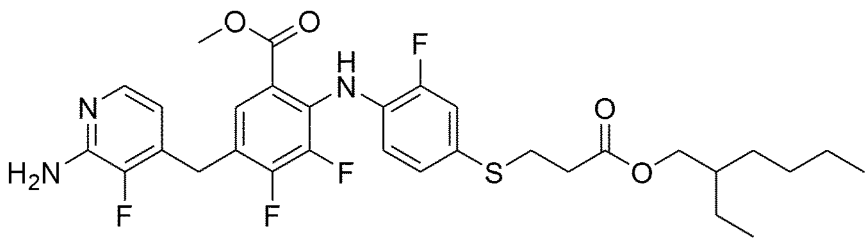

A suspension of methyl 5-((2-amino-3-fluoropyridin-4-yl)methyl)-3,4-difluoro-2-((2-fluoro-4-io dophenyl)amino)benzoate (compound a6, 500 mg, 0.941 mmol), 2-ethylhexyl 3-mercaptopropionate (226 mg, 1.04 mmol), Xantphos (109 mg, 0.188 mmol), tris(dibenzylideneacetone)dipalladium(0) (86 mg, 0.094 mmol) and DIPEA (0.492 mL, 2.82 mmol) in 1,4-dioxane (17 mL) was stirred for 1 hour at 110°C. After adding acetonitrile to the reaction mixture, this was filtered with Celite and the filtrate was concentrated under reduced pressure. The resulting residue was purified by reversed-phase column chromatography (0.1% formic acid aqueous solution/ 0.1% formic acid acetonitrile solution) to give the title compound (584 mg, quant.) as a yellow viscous oil.

LCMS m/z: 622 $[M+H]^+$

HPLC retention time: 1.14 min (analysis conditions G)

Compound h2:

Methyl

5-[(2-amino-3-fluorolpyridin-4-yl)methyl]-2-[4-(difluoromethylsulfanyl) -2-fluoroanilino]-3,4-difluorobenzoate

[Chemical Formula 72]

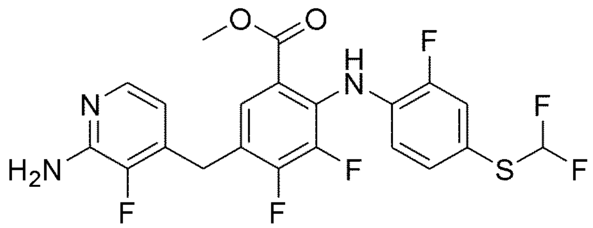

A methanol solution (9 mL) of methyl 5-[(2-amino-3-fluoropyridin-4-yl)methyl]-2-[4-[3-(2-ethylhexaoxy)-3-o xopropyl]sulfanyl-2-fluoroanilino]-3,4-difluorobenzoate (compound h1, 584 mg, 0.939 mmol) was cooled to 0°C, and then a 25% sodium methoxide methanol solution (1.29 mL, 5.64 mmol) was added and the mixture was stirred for 3 hours at room temperature. Diethyl (bromodifluoromethyl)phosphonate (1.00 g, 3.76 mmol) was then added at 0°C, and the mixture was stirred for 10 minutes at room temperature. The reaction mixture was cooled to 0°C, a 25% sodium methoxide methanol solution (1.29 mL, 5.64 mmol) and diethyl (bromodifluoromethyl)phosphonate (1.51 g, 5.64 mmol) were added, and the mixture was stirred for 20 minutes at room temperature. The reaction mixture was cooled to 0°C, formic acid (0.213 mL, 5.64 mmol) was added, and the mixture was concentrated under reduced pressure. The resulting residue was purified by reversed-phase column chromatography (0.1% formic acid aqueous solution/ 0.1% formic acid acetonitrile solution) to give the title compound (195 mg, 43%) as a colorless solid.
LCMS m/z: 488 $[M+H]^+$
HPLC retention time: 0.81 min (analysis conditions G)

Compound h3:

5-[(2-Amino-3-fluorolpyridin-4-yl)methyl]-2-[4-(difluoromethylsulfanyl )-2-fluoroanilino]-3,4-difluorobenzamide

[Chemical Formula 73]

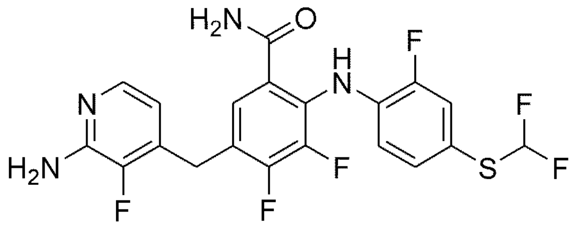

A mixture of methyl 5-[(2-amino-3-fluoropyridin-4-yl)methyl]-2-[4-(difluoromethylsulfanyl) -2-fluoroanilino]-3,4-difluorobenzoate (compound h2, 60.0 mg, 0.123 mmol) and a 7 M ammonia MeOH solution (1.80 mL, 12.6 mmol) was stirred for 6 hours at 85°C in a sealed tube using a microwave reactor. The reaction mixture was concentrated under reduced pressure, and the resulting residue was purified by reversed-phase column chromatography (0.05% trifluoroacetic acid aqueous solution/ 0.05% trifluoroacetic acid acetonitrile solution) to give the title compound (53.2 g, 91%) as a yellow oil.
LCMS m/z: 473 $[M+H]^+$
HPLC retention time: 0.63 min (analysis conditions B)

Compound H-1:

2-[4-(Difluoromethylsulfanyl)-2-fluoroanilino]-3,4-difluoro-5-[[3-fluor o-2-(methylsulfamoylamino)pyridin-4-yl]methyl]benzamide

[Chemical Formula 74]

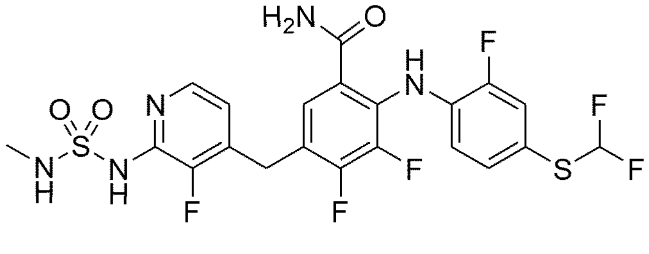

The title compound was synthesized from 5-[(2-amino-3-fluoropyridin-4-yl)methyl]-2-[4-(difluoromethylsulfanyl)-2-fluoroanilino]-3,4-difluorobenzamide (compound h3) under the same conditions as the production example for compound A-1.

LCMS m/z: 566 $[M+H]^+$

HPLC retention time: 1.49 min (analysis conditions H)

Compound h4:

2-(1-Benzothiophen-5-ylamino)-3,4-difluoro-5-formylbenzoic acid

[Chemical Formula 75]

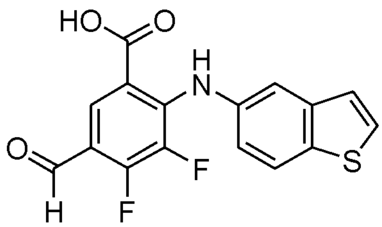

An anhydrous THF solution (30 mL) of 2,2,6,6-tetramethylpiperidine (2.53 g, 17.9 mmol) was cooled to -78°C, a 1.6 M n-butyllithium hexane solution (11.2 mL, 17.9 mmol) was added under a nitrogen atmosphere, and the mixture was stirred for 5 minutes. The reaction mixture was added to a THF solution (9.0 mL) of 2,3,4-trifluorobenzoic acid (1.50 g, 8.52 mmol) at -78°C and the mixture was stirred for 10 minutes, and then anhydrous DMF (0.759 mL, 9.80 mmol) was added and stirring was continued for 2 hours at 0°C. In a separate flask, a THF solution (30 mL) of benzo[b]thiophenone-5-amine (1.65 g, 11.1 mmol) was cooled to -78°C, and then a 1.3 M lithium bis(trimethylsilyl)amide THF solution (15.1 mL, 19.6 mmol) and the previous reaction mixture were added and the resulting mixture was stirred for 24 hours at room temperature. After adding 2 M hydrochloric acid to the reaction mixture and stirring for 24 hours, water and 2 M hydrochloric acid were added and extraction was performed with ethyl acetate. The organic layer was washed with saturated brine, dried over anhydrous sodium sulfate and, after filtering off the drying agent, concentrated under reduced pressure. The resulting residue was purified by reversed-phase column chromatography (0.1% formic acid aqueous solution/ 0.1% formic acid acetonitrile solution) to give the title compound (609 mg, 21%) as a gray solid.

LCMS m/z: 334 $[M+H]^+$

HPLC retention time: 0.80 min (analysis conditions B)

Compound h5:

2-(1-Benzothiophen-5-ylamino)-3,4-difluoro-5-[(E)-[(4-methylphenyl)s ulfonylhydrazinylidene]methyl]benzamide

[Chemical Formula 76]

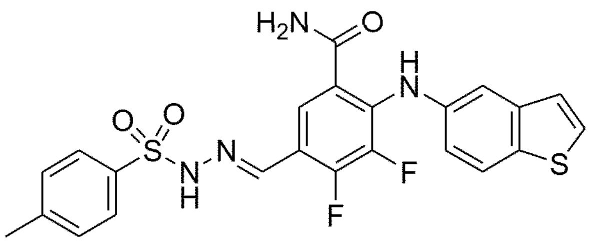

After adding HOOBt (595 mg, 3.65 mmol) and EDC·HCl (699 mg, 3.65 mmol) to an anhydrous DMF suspension (9.1 mL) of 2-(1-benzothiophen-5-ylamino)-3,4-difluoro-5-formylbenzoic acid (compound h4, 608 mg, 1.82 mmol), the mixture was stirred for 1.5 hours at room temperature. A 7 M ammonia MeOH solution (0.912 mL, 6.38 mmol) was then added at 0°C and stirring was continued for 30 minutes, 4-methylbenzenesulfonyl hydrazide (340 mg, 1.82 mmol) was further added at 0°C, and stirring was continued for 16 hours at room temperature. After filtering the reaction mixture, acetonitrile (14 mL) and 0.1 M hydrochloric acid (100 mL) were added to the filtrate. The solid was filtered and then washed with water to give the title compound (412 mg, 45%) as a light brown solid.

LCMS m/z: 501 $[M+H]^+$

HPLC retention time: 0.83 min (analysis conditions B)

Compound h7:

5-[(2-Amino-3-fluoropyridin-4-yl)methyl]-2-(1-benzothiophen-5-ylami no)-3,4-difluorobenzamide

[Chemical Formula 77]

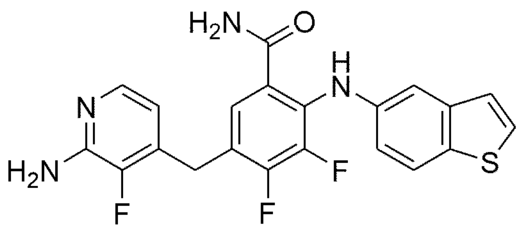

The title compound was synthesized from 2-(1-benzothiophen-5-ylamino)-3,4-difluoro-5-[(E)-[(4-methylphenyl)s ulfonylhydrazinylidene]methyl]benzamide (compound h5) under the same conditions as the production examples for compound a5 and compound a6.

LCMS m/z: 429 $[M+H]^+$

HPLC retention time: 0.57 min (analysis conditions B)

Compound h8:

5-[(2-Amino-3-fluoropyridin-4-yl)methyl]-3,4-difluoro-2-[(4-fluoro-1-b enzothiophen-5-yl)amino]benzamide

[Chemical Formula 78]

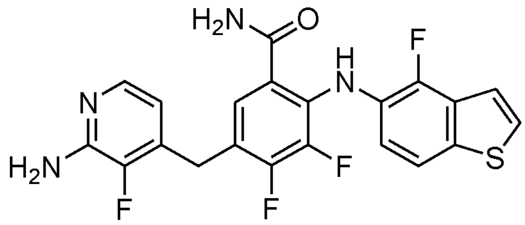

An anhydrous acetonitrile solution (0.3 mL) of 5-[(2-amino-3-fluoropyridin-4-yl)methyl]-2-(1-benzothiophen-5-ylamin o)-3,4-difluorobenzamide (compound h7, 22 mg, 0.051 mmol) was cooled to 0°C, and then N-fluoro-N'-(chloromethyl)triethylenediamine bis(tetrafluoroborate) (9.5 mg, 0.027 mmol) was added and the mixture was stirred for 2.5 hours. Next, N-fluoro-N'-(chloromethyl)triethylenediamine bis(tetrafluoroborate) (8.0 mg, 0.023 mmol) was added and the mixture was stirred for 1 hours. The reaction mixture was concentrated under reduced pressure, and the resulting residue was purified by reversed-phase column chromatography (0.05% trifluoroacetic acid aqueous solution/ 0.05% trifluoroacetic acid acetonitrile solution) to give the title compound (8.0 mg, 35%) as a brown solid.

LCMS m/z: 447 $[M+H]^+$
HPLC retention time: 0.61 min (analysis conditions B)

Compound H-3:

3,4-Difluoro-2-[(4-fluoro-1-benzothiophen-5-yl)amino]-5-[[3-fluoro-2-( methylsulfamoylamino)pyiridin-4-yl]methyl]benzamide

[Chemical Formula 79]

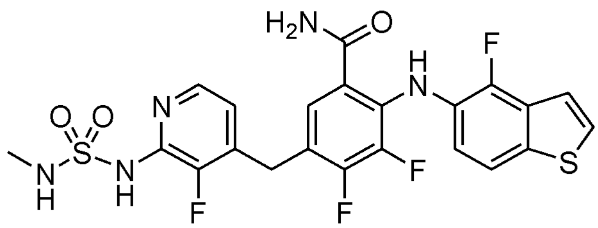

The title compound was synthesized from 5-[(2-amino-3-fluoropyridin-4-yl)methyl]-3,4-difluoro-2-[(4-fluoro-1-b enzothiophen-5-yl)amino]benzamide (compound h8) under the same conditions as the production example for compound A-1.

LCMS m/z: 540 $[M+H]^+$
HPLC retention time: 1.10 min (analysis conditions E)

Compound h9:

1,2,3-Trifluoro-4-[(4-methoxy)phenyl)methoxy]benzene

[Chemical Formula 80]

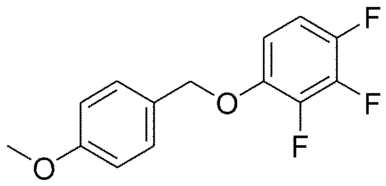

Potassium carbonate (9.90 g, 71.6 mmol) and 4-methoxybenzyl chloride (5.55 mL, 40.9 mmol) were added to an anhydrous acetone solution (101 mL) of 2,3,4-trifluorophenol (5.05 g, 34.1 mmol), and the mixture was stirred for 8 hours at 70°C. Water (150 mL) was added to the reaction mixture, and extraction was performed with ethyl acetate. The organic layer was dried over anhydrous sodium sulfate and, after filtering off the drying agent, concentrated under reduced pressure. DMSO (15 mL) and water (100 mL) were added to the resulting residue, and the resulting solid was washed to give the title compound (8.72 g, 95%) as a gray solid.

LCMS m/z: 267 $[M-H]^-$

HPLC retention time: 0.92 min (analysis conditions B)

Compound h10:

2,3,4-Trifluoro-5-[(4-methoxyphenyl)methoxy]benzoic acid

[Chemical Formula 81]

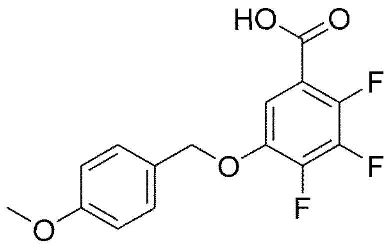

An anhydrous THF solution (15 mL) of 2,2,6,6-tetramethylpiperidine (4.15 mL, 24.6 mmol) was cooled to -78°C, and then a 1.6 M lithium bis(trimethylsilyl)amide hexane solution (15.4 mL, 24.6 mmol) was added under a nitrogen atmosphere and the mixture was stirred for 10 minutes. The reaction mixture was added to an anhydrous THF solution (15 mL) of 1,2,3-trifluoro-4-[(4-methoxyphenyl)methoxy]benzene (compound h9, 3.00 g, 11.2 mmol) at -78°C, and the mixture was stirred for 3 hours. Then the mixture was stirred for another 30 minutes while injecting carbon dioxide gas. After then adding 1 M hydrochloric acid (60 mL) to the reaction mixture, extraction was performed with ethyl acetate. The organic layer was washed with water, dried over anhydrous sodium sulfate and, after filtering off the drying agent, concentrated under reduced pressure. The resulting residue was purified by reversed-phase column chromatography (0.1% formic acid aqueous solution/ 0.1% formic acid acetonitrile solution) to give the title compound (1.32 g, 34%) as a gray solid.

LCMS m/z: 311 $[M-H]^-$

HPLC retention time: 0.80 min (analysis conditions B)

Compound h13:

3,4-Difluoro-2-(2-fluoro-4-iodoanilino)-5-methyl hydroxybenzoate

[Chemical Formula 82]

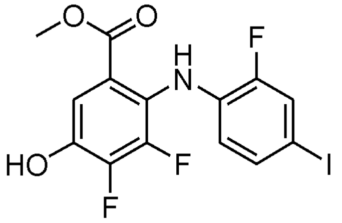

The title compound was synthesized from 2,3,4-trifluoro-5-[(4-methoxyphenyl)methoxy]benzoic acid (compound h10) under the same conditions as the production examples for compound c5, compound a1 and compound a6. However, 2-fluoro-4-iodoaniline was used instead of 4-iodo-2-methylaniline, which was used in the production example for compound c5, and anhydrous THF was used instead of toluene, which was used in the production example for compound a1.

LCMS m/z: 424 $[M+H]^+$

HPLC retention time: 0.91 min (analysis conditions B)

Compound h14:

Methyl

5-[2-[(2,4-dimethoxyphenyl)methylamino]-3-fluoropyridin-4-yl]oxy-3, 4-difluoro-2-(2-fluoro-4-iodoanilino)benzoate

[Chemical Formula 83]

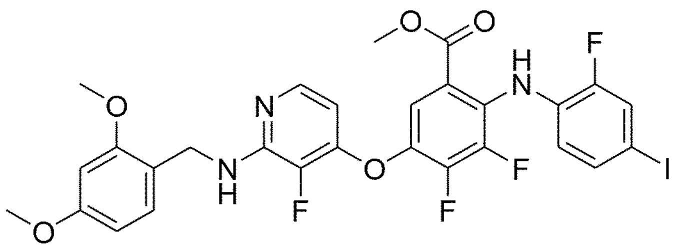

After adding [2-[(2,4-dimethoxyphenyl)methylamino]-3-fluoropyridin-4-yl]boronic acid (compound a4, 814 mg, 2.66 mmol), molecular sieves 4A (375 mg), tetrakis(acetonitrile)copper(I) hexafluorophosphate (495 mg, 1.33 mmol) and pyridine (0.287 mL, 3.55 mmol) to a DCM solution (15 mL) of 3,4-difluoro-2-(2-fluoro-4-iodoanilino)-5-methyl hydroxybenzoate (compound h13, 375 mg, 0.886 mmol), the mixture was stirred for 2.5 hours at room temperature. Next, [2-[(2,4-dimethoxyphenyl)methylamino]-3-fluoropyridin-4-yl]boronic acid (compound a4, 231 mg, 0.753 mmol) was added and the mixture was stirred for 4 hours. After then adding N-acetylcysteine (434 mg, 2.66 mmol) to the reaction mixture, stirring was continued for 3 hours. The solid portion was filtered off and washed with DCM (10 mL), and the filtrate was concentrated under reduced pressure. The resulting residue was purified by reversed-phase column chromatography (0.1% formic acid aqueous solution/ 0.1% formic acid acetonitrile solution) to give the title compound (168 mg, 28%) as a foam.

LCMS m/z: 684 $[M+H]^+$

HPLC retention time: 1.07 min (analysis conditions B)

Compound H-4:

3,4-Difluoro-2-(2-fluoro-4-iodoanilino)-5-[3-fluoro-2-(methylsulfamovl amino)pyridin-4-yl]oxybenzamide

[Chemical Formula 84]

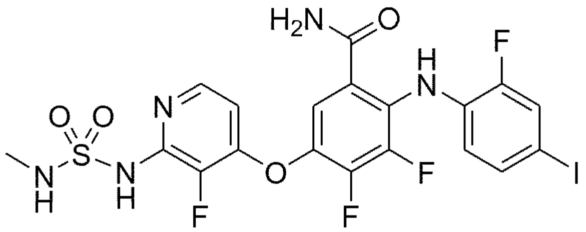

The title compound was synthesized from methyl 5-[2-[(2,4-dimethoxyphenyl)methylamino]-3-fluoropyridin-4-yl]oxy-3, 4-difluoro-2-(2-fluoro-4-iodoanilino)benzoate (compound h14) under the same conditions as the production examples for compound a6, compound E-13 and compound A-1.

LCMS m/z: 612 $[M+H]^+$
HPLC retention time: 1.55 min (analysis conditions H)

Compound I-1:

4-Fluoro-2-(2-fluoro-4-iodoanilino)-5-[[3-fluoro-2-(methylsulfamoylam ino)pyridin-4-yl]methyl]benzamide

[Chemical Formula 85]

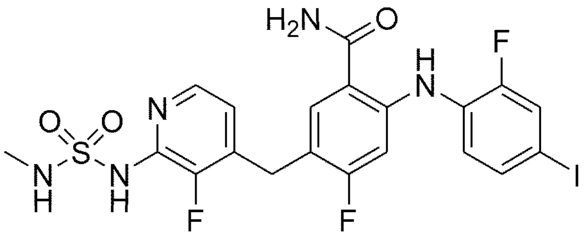

The title compound was synthesized from 2,4-difluoro-5-vinylbenzoic acid under the same conditions as the production examples for compound c5, compound c6, compound c1, compound a5, compound a6 and compound A-1. However, 2-fluoro-4-iodoaniline was used instead of 4-iodo-2-methylaniline, which was used in the production example for compound c5.

LCMS m/z: 592 $[M+H]^+$
HPLC retention time: 1.17 min (analysis conditions E)

Compound j1:

Methyl

5-[(2-amino-3-fluoropyridin-4-yl)methyl]-2-(2-fluoro-4-iodoanilino)-1-methyl-6-oxopyridine-3-carboxylate

[Chemical Formula 86]

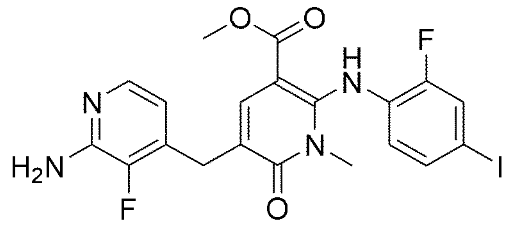

Thionyl chloride (10.6 mL, 145 mmol) was added to a DCM suspension (91 mL) of (2-amino-3-fluoropyridin-4-yl)methanol (10.3 g, 72.7 mmol) over 10 minutes, and the mixture was stirred for 65 minutes at room temperature. After filtering the reaction mixture, the resulting solid was dissolved in ethyl acetate and washed with sodium hydrogen carbonate aqueous solution. The organic layer was dried over anhydrous magnesium sulfate and, after filtering off the drying agent, concentrated under reduced pressure to give a crude product of 2-amino-4-(chloromethyl)-3-fluoropyridine (10.3 g).

The crude product of 2-amino-4-(chloromethyl)-3-fluoropyridine (3.47 g) and tripotassium phosphate (5.00 g, 23.6 mmol) were added to a 1,3-dimethyl-2-imidazolidinone solution (39 mL) of methyl 2-((2-fluoro-4-iodophenyl)amino)-1-methyl-6-oxo-1,6-dihydropyridine-3-carboxylate (7.90 g, 19.7 mmol) and tetrabutylammonium iodide (0.726 g, 1.97 mmol), and the mixture was stirred for 4 hours at 50°C. Water was added to the reaction mixture, and the resulting solid was filtered off and washed with a liquid mixture of acetonitrile/water to give the title compound (10.3 g, 60%).

LCMS m/z: 527 $[M+H]^+$

HPLC retention time: 0.63 min (analysis conditions B)

Compound j2:

5-[(2-Amino-3-fluoropyridin-4-yl)methyl]-2-(2-fluoro-4-iodoanilino)-1-methyl-6-oxopyridine-3-carboxylic acid hydrochloride

[Chemical Formula 87]

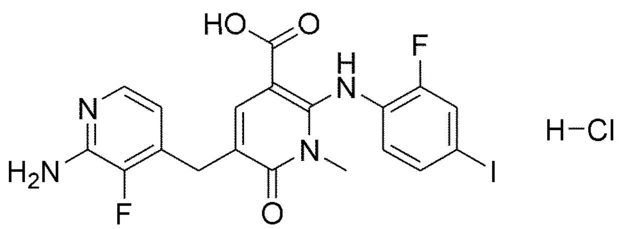

The title compound was synthesized from methyl 5-[(2-amino-3-fluoropyridin-4-yl)methyl]-2-(2-fluoro-4-iodoanilino)-1-methyl-6-oxopyridine-3-carboxylate (compound j1) under the same conditions as the production example for compound a7.

LCMS m/z: 513 $[M+H]^+$

HPLC retention time: 0.76 min (analysis conditions D)

Compound j3:

5-[(2-Amino-3-fluoropyridin-4-yl)methyl]-2-(2-fluoro-4-iodoanilino)-1-methyl-6-oxopyridine-3-carboxamide

[Chemical Formula 88]

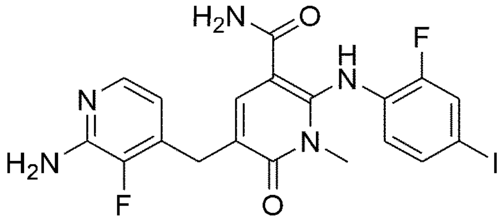

The title compound was synthesized from 5-[(2-amino-3-fluoropyridin-4-yl)methyl]-2-(2-fluoro-4-iodoanilino)-1-methyl-6-oxopyridine-3-carboxylic acid hydrochloride (compound j2) under the same conditions as the production example for compound a8.

LCMS m/z: 512 $[M+H]^+$

HPLC retention time: 0.84 min (analysis conditions D)

Compound J-1:

2-(2-Fluoro-4-iodoanilino)-5-[[3-fluoro-2-(methylsulfamoylamino)pyri din-4-yl]methyl-1-methyl-6-oxopyridine-3-carboxamide

[Chemical Formula 89]

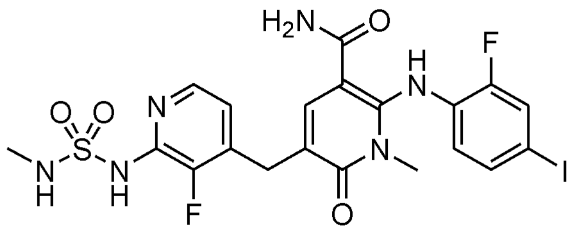

The title compound was synthesized from methyl 5-[(2-amino-3-fluoropyridin-4-yl)methyl]-2-(2-fluoro-4-iodoanilino)-1-methyl-6-oxopyridine-3-carboxamide (compound j3) under the same conditions as the production example for compound A-25.

LCMS m/z: 605 $[M+H]^+$

HPLC retention time: 0.95 min (analysis conditions E)

Compound k1:

Methyl

2-(2-fluoro-4-iodoanilino)-5-formyl-1-methyl-6-oxopyridine-3-carboxyl ate

[Chemical Formula 90]

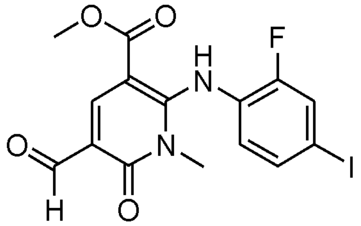

After adding (chloromethylene)dimethyliminium chloride (168 mg, 1.31 mmol) to an acetonitrile solution (2.7 mL) of methyl 2-((2-fluoro-4-iodophenyl)amino)-1-methyl-6-oxo-1,6-dihydropyridine-3-carboxylate (132 mg, 0.328 mmol), the mixture was stirred for 1.5 hours at room temperature. Water was added to the reaction mixture, stirring was continued for 30 minutes, and then the solid was filtered off to give the title compound (108 mg, 76%).
LCMS m/z: 431 $[M+H]^+$
HPLC retention time: 0.80 min (analysis conditions B)

Compound k4:

Methyl

5-[(3-amino-2-fluorophenyl)methyl]-2-(2-fluoro-4-iodoanilino)-1-meth yl-6-oxopyridine-3-carboxylate

[Chemical Formula 91]

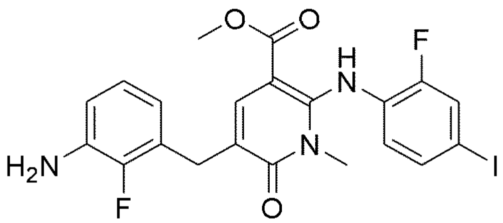

The title compound was synthesized from methyl 2-(2-fluoro-4-iodoanilino)-5-formyl-1-methyl-6-oxopyridine-3-carboxyl ate (compound k1) under the same conditions as the production examples for compound a2, compound a5 and compound a6. However, 2-nitrobenzene-1-sulfonohydrazide was used instead of 4-methylbenzenesulfonyl hydrazide, which was used in the production example for compound a2. Also, [2-fluoro-3-[(2-methylpropan-2-yl)oxycarbonylamino]phenyl]boronic acid and DIPEA were used instead of [2-[(2,4-dimethoxyphenyl)methylamino]-3-fluoropyridin-4-yl]boronic acid (compound a4) and potassium carbonate respectively, which were used in the production example for compound a5.
LCMS m/z: 526 $[M+H]^+$
HPLC retention time: 0.90 min (analysis conditions B)

Compound k11:

5-[[3-(Ethylsulfonylamino)-2-fluorophenyl]methyl]-2-(2-fluoro-4-iodoa nilino)-1-methyl-6-oxopyridine-3-carboxylic acid

[Chemical Formula 92]

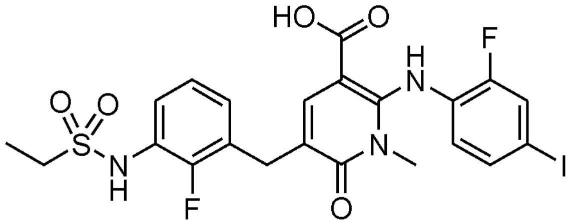

The title compound was synthesized from methyl 5-[(3-amino-2-fluorophenyl)methyl]-2-(2-fluoro-4-iodoanilino)-1-meth yl-6-oxopyridine-3-carboxylate (compound k4) and the corresponding sulfonyl chloride under the same conditions as the production examples for compound A-25 and compound b2. However, pyridine was used as the solvent in the sulfonamidation step.

LCMS m/z: 604 $[M+H]^+$

HPLC retention time: 0.77 min (analysis conditions B)

Compound K-10:

5-[[3-(ethylsulfonylamino)-2-fluorophenyl]methyl]-2-(2-fluoro-4-iodoa nilino)-1-methyl-6-oxopyridine-3-carboxamide

[Chemical Formula 93]

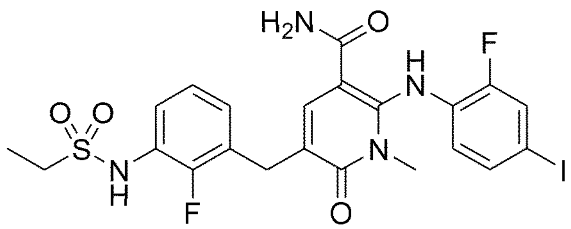

After adding HOOBt (8.92 mg, 0.055 mmol) and EDC·HCl (10.5 mg, 0.055 mmol) to an anhydrous DMF solution (0.264 mL) of 5-[[3-(ethylsulfonylamino)-2-fluorophenyl]methyl]-2-(2-fluoro-4-iodoa nilino)-1-methyl-6-oxopyridine-3-carboxylic acid (compound k11, 22.0 mg, 0.036 mmol), the mixture was stirred for 3 hours at room temperature. After then adding a 7 M ammonia MeOH solution (20.8 μL, 0.146 mmol) at 0°C, stirring was continued for 1 hour. The reaction mixture was purified by reversed-phase column chromatography (0.1% formic acid aqueous solution/ 0.1% formic acid acetonitrile solution) to give the title compound (15.8 mg, 72%) as a colorless solid.

LCMS m/z: 603 $[M+H]^+$

HPLC retention time: 1.42 min (analysis conditions H)

Compound 12:

Methyl

2-bromo-5-(2-fluoro-4-trimethylsilylanilino)pyridine-4-carbonlate

[Chemical Formula 94]

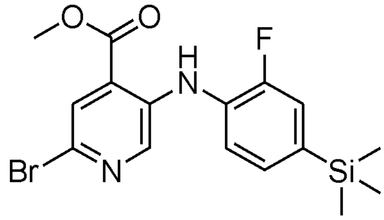

The title compound was synthesized from 2-bromo-5-fluoropyridine-4-carboxylic acid under the same conditions as the production examples for compound c5 and compound a1. However, 2-fluoro-4-trimethylsilylaniline was used instead of 4-iodo-2-methylaniline, which was used in the production example for compound c5.

LCMS m/z: 397 [M+H]$^+$
HPLC retention time: 1.17 min (analysis conditions G)

Compound 13a:

Methyl

5-(2-fluoro-4-trimethylsilylanilino)-2-formylpyridine-4-carboxylate

[Chemical Formula 95]

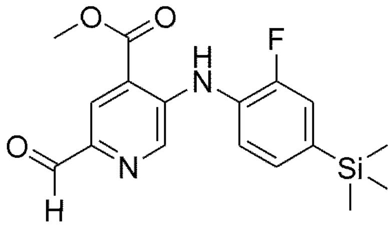

Compound 13b:

5-(2-Fluoro-4-trimethylsilylanilino)-4-methoxycarbonylpyridine-2-carb oxylic acid

[Chemical Formula 96]

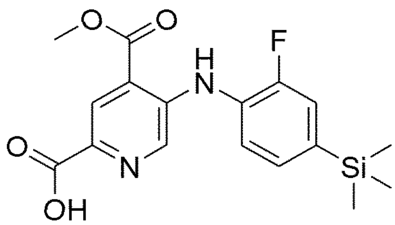

After adding an anhydrous DMF solution (63 mL) of triethylsilane (2.01 mL, 12.6 mmol) to an anhydrous DMF suspension (63 mL) of methyl 2-bromo-5-(2-fluoro-4-trimethylsilylanilino)pyridine-4-carboxylate (compound I2, 2.5 g, 6.29 mmol), 1,1,3-trioxo-1,2-benzothiazole-2-carbaldehyde (2.66 g, 612.6 mmol), Xantphos (728 mg, 1.26 mmol), palladium acetate (141 mg, 0.629 mmol) and sodium carbonate (1.67 g, 15.7 mmol), the mixture was stirred for 10 minutes at room temperature and then for 2.5 hours at 75°C. The reaction mixture was purified by reversed-phase column

chromatography (0.1% formic acid aqueous solution/ 0.1% formic acid acetonitrile solution) to give compound I3a (0.4 g, 18%) and compound I3b (1.4 g, 61%), each as a yellow solid.

Compound I3a

LCMS m/z: 347 $[M+H]^+$
HPLC retention time: 1.06 min (analysis conditions G)

Compound I3b

LCMS m/z: 363 $[M+H]^+$
HPLC retention time: 0.92 min (analysis conditions G)

Compound I4:

Methyl

5-(2-fluoro-4-trimethylsilylanilino)-2-(hydroxymethyl)pyridine-4-carbo xylate

[Chemical Formula 97]

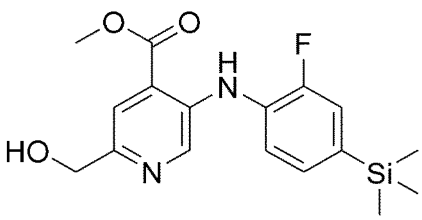

After adding a 1 M borane-tetrahydrofuran complex THF solution (4.33 mL, 4.33 mmol) to an anhydrous THF solution (14 mL) of methyl 5-(2-fluoro-4-trimethylsilylanilino)-2-formylpyridine-4-carboxylate (compound I3a, 500 mg, 1.44 mmol), the mixture was stirred for 1 hour at room temperature. Acetic acid (0.496 mL, 8.66 mmol) was added to the reaction mixture, which was then concentrated under reduced pressure. The resulting residue was purified by reversed-phase column chromatography (0.1% formic acid aqueous solution/ 0.1% formic acid acetonitrile solution) to give the title compound (360 mg, 72%) as a light yellow solid.
LCMS m/z: 349 $[M+H]^+$
HPLC retention time: 0.91 min (analysis conditions G)

Compound I4:

Methyl

5-(2-fluoro-4-trimethylsilylanilino)-2-(hydroxymethyl)pyridine-4-carbo xylate

[Chemical Formula 98]

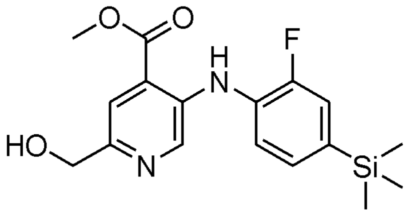

After adding a borane-dimethyl sulfide complex (0.747 mL, 7.86 mmol) to an anhydrous THF solution (16 mL) of 5-(2-fluoro-4-trimethylsilylanilino)-4-methoxycarbonylpyridine-2-carbo xylic acid (compound I3b, 570 mg, 1.57 mmol), the mixture was stirred for 2 hours at room temperature. Acetic acid (1.58 mL, 27.6 mmol) was added to the reaction mixture, which was then concentrated under reduced pressure. The resulting residue was purified by reversed-phase column chromatography (0.1% formic acid aqueous solution/ 0.1% formic acid acetonitrile solution) to give the title compound (350 mg, 64%) as a light yellow solid.

LCMS m/z: 349 $[M+H]^+$

HPLC retention time: 0.91 min (analysis conditions G)

Compound I5:

Methyl

2-(chloromethyl)-5-(2-fluoro-4-trimethylsilylanilino)pyridine-4-carboxy late

[Chemical Formula 99]

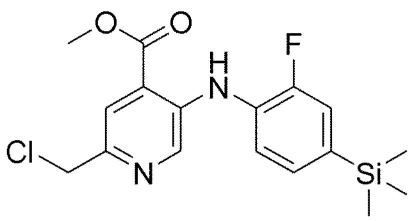

After adding thionyl chloride (0.168 mL, 2.30 mmol) to a DCM solution (12 mL) of methyl 5-(2-fluoro-4-trimethylsilylanilino)-2-(hydroxymethyl)pyridine-4-carbo xylate (compound I4, 400 mg, 1.15 mmol), the mixture was stirred for 50 minutes at room temperature. The reaction mixture was concentrated under reduced pressure to give a crude product of the title compound (400 mg).

LCMS m/z: 367 $[M+H]^+$

HPLC retention time: 1.11 min (analysis conditions G)

Compound I6:

Methyl

2-[[2-[(2,4-dimethoxyphenyl)methylamino]-3-fluoropyridin-4-yl]methy 1]-5-(2-fluoro-4-trimethylsilylanilino)pyridine-4-carboxylate

[Chemical Formula 100]

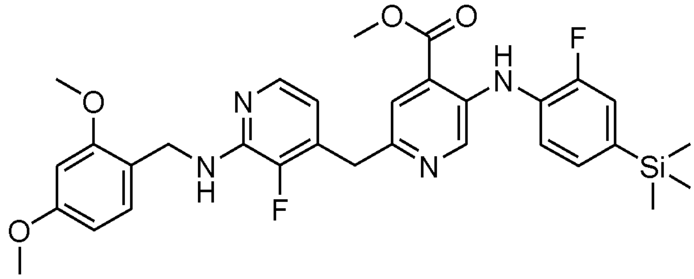

A 1,4-dioxane suspension (17 mL) of methyl 2-(chloromethyl)-5-(2-fluoro-4-trimethylsilylanilino)pyridine-4-carboxy late (compound l5, 584 mg, 1.59 mmol), [2-[(2,4-dimethoxyphenyl)methylamino]-3-fluoropyridin-4-yl]boronic acid (compound a4, 731 mg, 2.39 mmol), tetrakistriphenylphosphine palladium (184 mg, 0.159 mmol) and potassium carbonate (660 mg, 4.78 mmol) was stirred for 2 hours at 110°C. Water was added to the reaction mixture, and extraction was performed with ethyl acetate. The organic layer was washed with saturated brine, dried over anhydrous sodium sulfate and, after filtering off the drying agent, concentrated under reduced pressure. The resulting residue was purified by column chromatography (hexane/ ethyl acetate) to give the title compound (583 mg, 62%) as a yellow solid.

LCMS m/z: 593 $[M+H]^+$
HPLC retention time: 1.13 min (analysis conditions G)

Compound l7:

Methyl

2-[(2-amino-3-fluoropyridin-4-yl)methyl]-5-(2-fluoro-4-trimethylsilylan ilino)pyridine-4-carboxylate

[Chemical Formula 101]

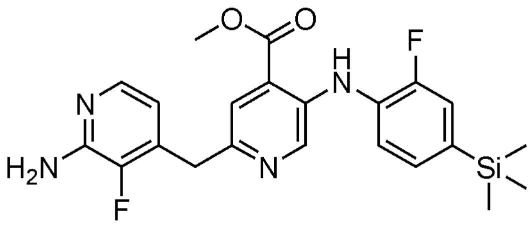

The title compound was synthesized from methyl 2-[[2-[(2,4-dimethoxyphenyl)methylamino]-3-fluoropyridin-4-yl]methy 1]-5-(2-fluoro-4-trimethylsilylanilino)pyridine-4-carboxylate (compound l6) under the same conditions as the production example for compound a6.

LCMS m/z: 443 $[M+H]^+$
HPLC retention time: 0.83 min (analysis conditions G)

Compound l8:

Methyl

2-[(2-amino-3-fluoropyridin-4-yl)methyl]-5-(2-fluoro-4-iodoanilino)pyr idine-4-carboxylate

[Chemical Formula 102]

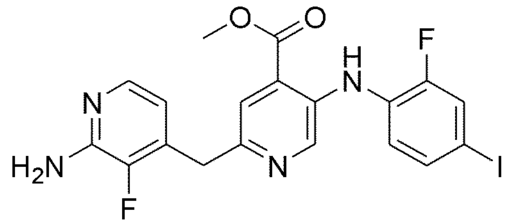

An anhydrous DCM solution (14 mL) of methyl 2-[(2-amino-3-fluoropyridin-4-yl)methyl]-5-(2-fluoro-4-trimethylsilylan ilino)pyridine-4-carboxylate (compound I7, 300 mg, 0.678 mmol) was cooled to 0°C, iodine monochloride (220 mg, 1.36 mmol) was added, and the mixture was stirred for 30 minutes at 0°C and then for 3 hours at room temperature. The reaction mixture was concentrated under reduced pressure, and the resulting residue was purified by reversed-phase column chromatography to give the title compound (320 mg, 95%) as a yellow solid.

LCMS m/z: 497 $[M+H]^+$

HPLC retention time: 0.69 min (analysis conditions G)

Compound L-1:

5-(2-Fluoro-4-iodoanilino)-2-[[3-fluoro-2-(methylsulfamoylamino)pyrl din-4-yl]methyl]pyridine-4-carboxamide

[Chemical Formula 103]

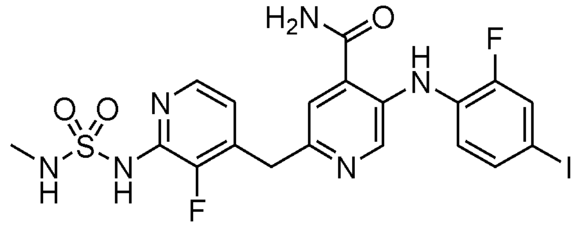

The title compound was synthesized from methyl 2-[(2-amino-3-fluoropyridin-4-yl)methyl]-5-(2-fluoro-4-iodoanilino)pyr idine-4-carboxylate (compound I8) under the same conditions as the production examples for compound a7, compound K-10 and compound A-1.

LCMS m/z: 575 $[M+H]^+$

HPLC retention time: 1.36 min (analysis conditions H)

Compound m1:

Methyl 2-amino-6-(aminomethyl)pyridine-3-carboxylate diacetate

[Chemical Formula 104]

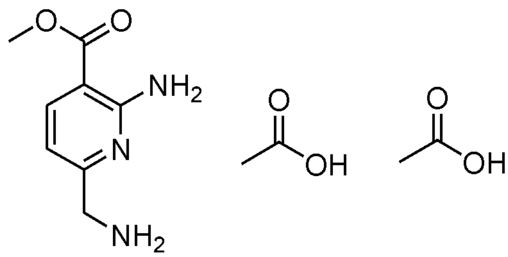

After adding a palladium on activated carbon powder catalyst (10% palladium) (933 mg, 0.877 mmol) to a mixed solution of methyl 2-amino-6-cyanopyridine-3-carboxylate (9.14 g, 51.6 mmol) in acetic acid (100 mL) and methanol (100 mL), the mixture was stirred for 4 hours at room temperature under a hydrogen atmosphere. The reaction mixture was filtered with Celite, and the filtrate was concentrated under reduced pressure to give a crude product of the title compound (15.3 g).

LCMS m/z: 182 $[M+H]^+$

HPLC retention time: 0.26 min (analysis conditions G)

Compound m2:

Methyl 2-amino-6-(formamidemethyl)pyridine-3-carboxylate

[Chemical Formula 105]

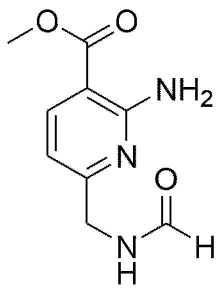

Acetic anhydride (115 mL, 1.22 mol) was added to a formic acid solution (230 mL) of methyl 2-amino-6-(aminomethyl)pyridine-3-carboxylate diacetate (compound m1, 14.7 g, 48.8 mmol) over 30 minutes, and the mixture was stirred overnight at 70°C. The reaction mixture was concentrated under reduced pressure, a saturated sodium hydrogen carbonate aqueous solution was added to the resulting residue, and extraction was performed with ethyl acetate. The organic layer was washed with saturated brine, dried over anhydrous magnesium sulfate and, after filtering off the drying agent, concentrated under reduced pressure. The resulting residue was purified by amino-silica gel column chromatography (hexane/DCM) to give the title compound (8.16 g, 80%) as a yellow solid.

LCMS m/z: 210 $[M+H]^+$

HPLC retention time: 0.29 min (analysis conditions G)

Compound m3:

Methyl 2-amino-5-bromo-6-(formamidemethyl)pyridine-3-carboxylate

[Chemical Formula 106]

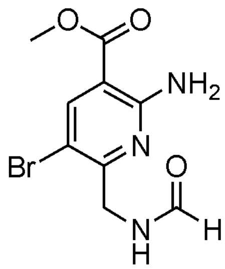

After adding N-bromosuccinimide (7.87 g, 44.2 mmol) to an anhydrous acetonitrile solution (400 mL) of methyl 2-amino-6-(formamidemethyl)pyridine-3-carboxylate (compound m2, 9.25 g, 44.2 mmol) in several portions, the mixture was stirred for 1 hour at room temperature. The reaction mixture was concentrated under reduced pressure and water was added to the resulting residue. The resulting solid was washed with water to give the title compound (12.0 g, 94%) as a yellow solid.

LCMS m/z: 288 $[M+H]^+$

HPLC retention time: 0.52 min (analysis conditions G)

Compound m4:

Methyl 5-amino-8-bromoimidazo[1,5-a]pyridine-6-carboxylate

[Chemical Formula 107]

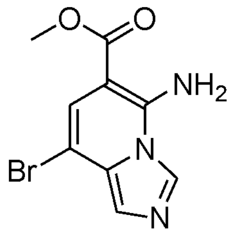

After adding phosphoryl chloride (17.5 mL, 187 mmol) to an anhydrous toluene suspension (200 mL) of methyl 2-amino-5-bromo-6-(formamidemethyl)pyridine-3-carboxylate (compound m3, 12.0 g, 41.7 mmol), the mixture was stirred for 1 hour at 95°C. The reaction mixture was concentrated under reduced pressure, and a saturated sodium hydrogen carbonate aqueous solution and water were added to the resulting residue. The resulting solid was washed with water and dissolved in DCM, and then dried over anhydrous magnesium sulfate. After filtering off the drying agent, the filtrate was concentrated under reduced pressure to give the title compound (10.5 g, 93%) as a light brown solid.

LCMS m/z: 270 $[M+H]^+$

HPLC retention time: 0.65 min (analysis conditions G)

Compound m5:

Methyl

5-[bis[(2-methylpropan-2-yl)oxycarbonyl]amino]-8-bromoimidazo[1,5-alpyridine-6-carboxylate

[Chemical Formula 108]

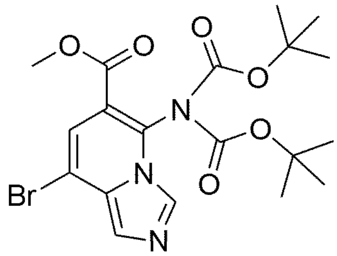

After adding 4-dimethylaminopyridine (143 mg, 1.17 mmol) to an anhydrous DCM solution (50 mL) of methyl 5-amino-8-bromoimidazo[1,5-a]pyridine-6-carboxylate (compound m4, 1.58 g, 5.85 mmol) and di-tert-butyl dicarbonate (3.19 g, 14.6 mmol), the mixture was stirred for 2 hours at room temperature. The reaction mixture was concentrated under reduced pressure, and the resulting residue was purified by silica gel column chromatography to give the title compound (2.5 g, 91%) as a yellow solid.

LCMS m/z: 470 $[M+H]^+$

HPLC retention time: 0.95 min (analysis conditions G)

Compound m6:

Methyl

5-[bis[(2-methylpropan-2-yl)oxycarbonyl]amino]-8-ethenylimidazo[1,5 -a]pyridine-6-carboxylate

[Chemical Formula 109]

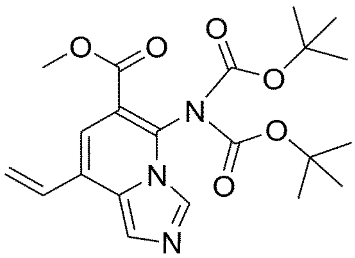

A mixed suspension of methyl 5-[bis[(2-methylpropan-2-yl)oxycarbonyl]amino]-8-bromoimidazo[1,5-a]pyridine-6-carboxylate (compound m5, 2.5 g, 5.32 mmol), potassium vinyl trifluoroborate (1.07 g, 7.97 mmol), tetrakistriphenylphosphine palladium (614 mg, 0.532 mmol) and cesium carbonate (5.20 g, 16.0 mmol) in 1,4-dioxane (40 mL) and water (10 mL) was stirred for 2 hours at 100°C. The reaction mixture was extracted with ethyl acetate and the organic layer was washed with saturated brine and dried over anhydrous magnesium sulfate. After filtering off the drying agent, the mixture was concentrated under reduced pressure and the resulting residue was purified by silica gel column chromatography to give the title compound (1.68 g, 76%) as a yellow solid.

LCMS m/z: 418 $[M+H]^+$

HPLC retention time: 0.89 min (analysis conditions G)

Compound m8:

Methyl 5-amino-8-formylimidazo[1,5-a]pyridine-6-carboxylate trifluoroacetate

[Chemical Formula 110]

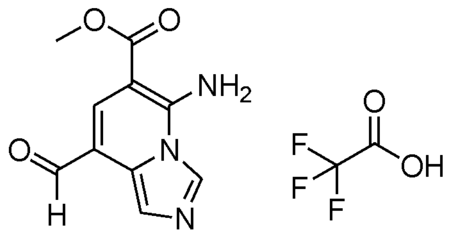

The title compound was synthesized from methyl 5-[bis[(2-methylpropan-2-yl)oxycarbonyl]amino]-8-ethenylimidazo[1,5 -a]pyridine-6-carboxylate (compound m6) under the same conditions as the production examples for compound c6 and compound a6.

LCMS m/z: 220 [M+H]+
HPLC retention time: 0.48 min (analysis conditions G)

Compound m9:

Methyl

5-amino-8-(5,5-dimethyl-1,3-dioxan-2-yl)imidazo[1,5-a]pyridine-6-carb oxylate

[Chemical Formula 111]

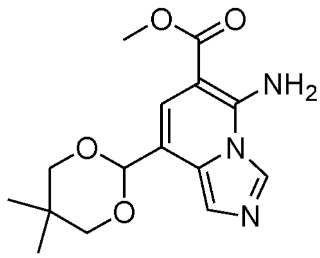

A toluene suspension (30 mL) of methyl 5-amino-8-formylimidazo[1,5-a]pyridine-6-carboxylate trifluoroacetate (compound m8, 475 mg, 1.43 mmol), p-toluenesulfonic acid monohydrate (54.2 mg, 0.285 mmol) and 2,2-dimethyl-1,3-propanediol (742 mg, 7.13 mmol) was stirred overnight at 110°C. DIPEA (1 mL) was added to the reaction mixture, which was then concentrated under reduced pressure. The resulting residue was purified by reversed-phase column chromatography to give the title compound (340 mg, 78%) as a red solid.

LCMS m/z: 306 [M+H]+
HPLC retention time: 0.66 min (analysis conditions G)

Compound m10:

Methyl

5-chloro-8-(5,5-dimethyl-1,3-dioxan-2-yl)imidazo[1,5-a]pyridine-6-car boxylate

[Chemical Formula 112]

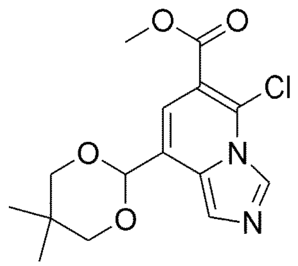

An acetonitrile suspension (15 mL) of methyl 5-amino-8-(5,5-dimethyl-1,3-dioxan-2-yl)imidazo[1,5-a]pyridine-6-carb oxylate (compound m9, 340 mg, 1.11 mmol) was cooled to 0°C, and then copper(I) chloride (165 mg, 1.67 mmol) and copper(II) chloride (225 mg, 1.67 mmol) were added. After then adding tert-butyl nitrite (172 mg, 1.67 mmol), the mixture was stirred for 30 minutes at room temperature. The reaction mixture was concentrated under reduced pressure, and the resulting residue was purified by reversed-phase column chromatography to give the title compound (237 mg, 66%) as a red solid.

LCMS m/z: 325 $[M+H]^+$

HPLC retention time: 0.77 min (analysis conditions G)

Compound m11:

Methyl

8-(5,5-dimethyl-1,3-dioxan-2-yl)-5-(2-fluoro-4-iodoanilino)imidazo[1,5 -alpyridine-6-carboxylate

[Chemical Formula 113]

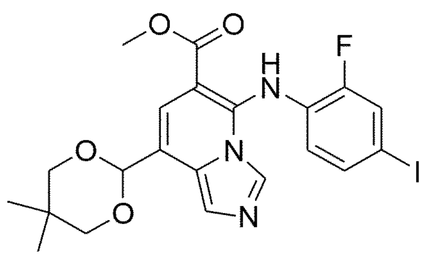

A DMA suspension (4 mL) of methyl 5-chloro-8-(5,5-dimethyl-1,3-dioxan-2-yl)imidazo[1,5-a]pyridine-6-car boxylate (compound m10, 237 mg, 0.730 mmol), cesium carbonate (713 mg, 2.19 mmol) and 2-fluoro-4-iodoaniline (346 mg, 1.46 mmol) was stirred overnight at 50°C. Water was added to the reaction mixture, and extraction was performed with ethyl acetate. The organic layer was washed with saturated brine, dried over anhydrous magnesium sulfate and, after filtering off the drying agent, concentrated under reduced pressure. The resulting residue was purified by reversed-phase column chromatography to give the title compound (210 mg, 55%) as a yellow solid.

LCMS m/z: 526 $[M+H]^+$

HPLC retention time: 1.02 min (analysis conditions G)

Compound m12:

Methyl

5-(2-fluoro-4-iodoanilino)-8-formylimidazo[1,5-a]pyridine-6-carboxylat e

[Chemical Formula 114]

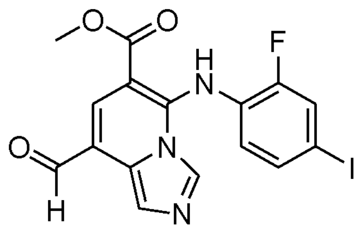

A mixed suspension of methyl 8-(5,5-dimethyl-1,3-dioxan-2-yl)-5-(2-fluoro-4-iodoanilino)imidazo[1,5 -a]pyridine-6-carboxylate (compound m11, 210 mg, 0.400 mmol) in water (2 mL) and TFA (2 mL) was stirred for 1 hour at room temperature. The reaction mixture was concentrated under reduced pressure, and the resulting residue was purified by reversed-phase column chromatography (0.1% formic acid aqueous solution/ 0.1% formic acid acetonitrile solution) to give the title compound (168 mg, 96%) as a yellow solid.
LCMS m/z: 440 $[M+H]^+$
HPLC retention time: 0.84 min (analysis conditions G)

Compound m15:

Methyl

8-[(2-amino-3-fluoropyridin-4-yl)methyl]-5-(2-fluoro-4-iodoanilino)imi dazo[1,5-a]pyridine-6-carboxylate trifluoroacetate

[Chemical Formula 115]

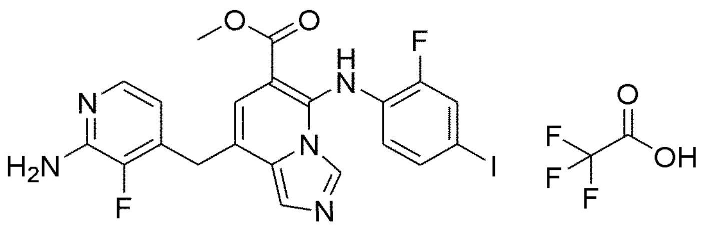

The title compound was synthesized from methyl 5-(2-fluoro-4-iodoanilino)-8-formylimidazo[1,5-a]pyridine-6-carboxylat e (compound m12) under the same conditions as the production examples for compound a2, compound a5 and compound a6. However, MeOH was used instead of EtOH, which was used in the production example for compound a2.
LCMS m/z: 536 $[M+H]^+$
HPLC retention time: 0.54 min (analysis conditions F)

Compound m16:

8-[(2-Amino-3-fluoropyridin-4-yl)methyl]-5-(2-fluoro-4-iodoanilino)im idazo[1,5-a]pvridine-6-carboxylic acid trifluoroacetate

[Chemical Formula 116]

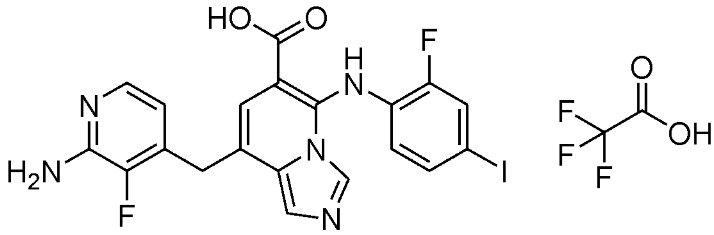

A mixed suspension of methyl 8-[(2-amino-3-fluoropyridin-4-yl)methyl]-5-(2-fluoro-4-iodoanilino)imi dazo[1,5-a]pyridine-6-carboxylate trifluoroacetate (compound m15, 60 mg, 0.092 mmol) and lithium hydroxide monohydrate (78 mg, 1.85 mmol) in THF (3 mL) and water (2 mL) was stirred overnight at 50°C. The reaction mixture was acidified by addition of formic acid, and concentrated under reduced pressure. The resulting residue was purified by reversed-phase column chromatography (0.1% TFA aqueous solution/ 0.1% TFA acetonitrile solution) to give the title compound (42 mg, 72%) as a yellow solid.
LCMS m/z: 522 $[M+H]^+$
HPLC retention time: 0.45 min (analysis conditions F)

Compound m17:

8-[(2-Amino-3-fluoropyridin-4-yl)methyl]-5-(2-fluoro-4-iodoanilino)im idazo[1,5-a]pyridine-6-carboxamide trifluoroacetate

[Chemical Formula 117]

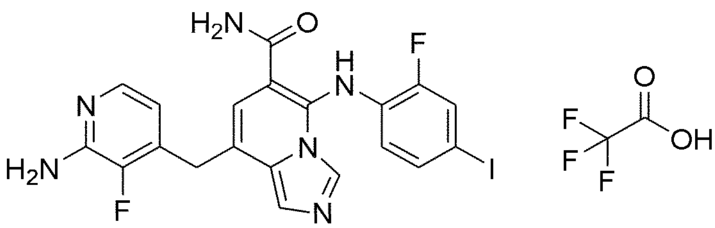

A DMF solution (1.6 mL) of 8-[(2-amino-3-fluoropyridin-4-yl)methyl]-5-(2-fluoro-4-iodoanilino)imi dazo[1,5-a]pyridine-6-carboxylic acid trifluoroacetate (compound m16, 42 mg, 0.066 mmol) was cooled to 0°C, and then HATU (390 mg, 1.03 mmol), ammonium chloride (67.2 mg, 1.26 mmol) and DIPEA (0.253 mL, 1.45 mmol) were added and the mixture was stirred for 5 hours at room temperature. The reaction mixture was concentrated under reduced pressure, and the resulting residue was purified by reversed-phase column chromatography (0.1% TFA aqueous solution/ 0.1% TFA acetonitrile solution) to give the title compound (25 mg, 60%) as a yellow solid.
LCMS m/z: 521 $[M+H]^+$
HPLC retention time: 0.41 min (analysis conditions F)

Compound M-1:

5-(2-Fluoro-4-iodoanilino)-8-[[3-fluoro-2-(methylsulfamoylamino)pyri din-4-yl]methyl]imidazo[1,5-a]pyridine-6-carboxamide

[Chemical Formula 118]

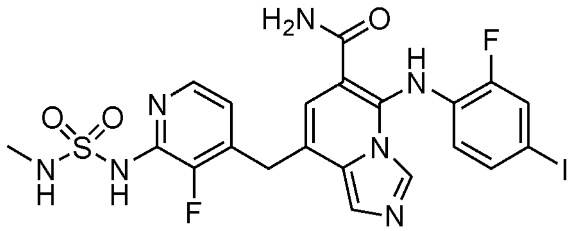

The title compound was synthesized from 8-[(2-amino-3-fluoropyridin-4-yl)methyl]-5-(2-fluoro-4-iodoanilino)imidazo[1,5-a]pyridine-6-carboxamide trifluoroacetate (compound m17) under the same conditions as the production example for compound A-1.

LCMS m/z: 614 $[M+H]^+$

HPLC retention time: 1.18 min (analysis conditions H)

Compound n1:

6-Chloro-5-fluoro-4-(2-fluoro-4-iodoanilino)pyridine-3-carboxylic acid

[Chemical Formula 119]

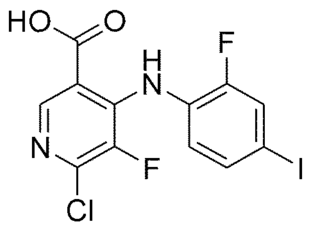

An anhydrous THF solution (8 ml) of 2-fluoro-4-iodoaniline (2.26 g, 9.52 mmol) was cooled to -78°C, and then a 2 M LDA THF/heptane/ethylbenzene solution (7.14 mL, 14.3 mmol) was added and the mixture was stirred for 30 minutes. An anhydrous THF solution (8 mL) of 4,6-dichloro-5-fluoropyridine-3-carboxylic acid (1.00 g, 4.76 mmol) was added, and the mixture was stirred for 30 minutes at -78°C. Water and 6 M hydrochloric acid were then added to the reaction mixture to adjust the pH to between 1 and 2, and extraction was performed with ethyl acetate. The organic layer was dried over anhydrous sodium sulfate and, after filtering off the drying agent, concentrated under reduced pressure. Recrystallization (DCM) was carried out from the resulting residue to give the title compound (850 mg, 44%) as a light brown solid.

LCMS m/z: 411 $[M+H]^+$

HPLC retention time: 0.85 min (analysis conditions G)

Compound n2:

Methyl

6-chloro-5-fluoro-4-(2-fluoro-4-iodoanilino)pyridine-3-carboxylate

[Chemical Formula 120]

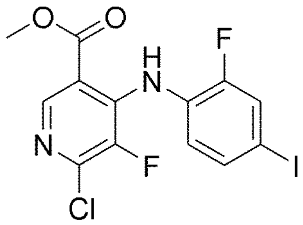

The title compound was synthesized from 6-chloro-5-fluoro-4-(2-fluoro-4-iodoanilino)pyridine-3-carboxylic acid (compound n1) under the same conditions as the production example for compound a1.

LCMS m/z: 425 [M+H]$^+$

HPLC retention time: 1.04 min (analysis conditions G)

Compound n3:

Methyl

5-fluoro-4-(2-fluoro-4-iodoanilino)-6-hydroxypyridine-3-carboxylate

[Chemical Formula 121]

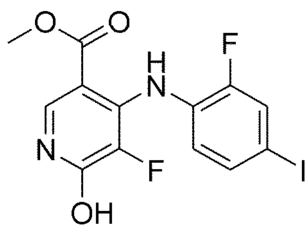

Potassium carbonate (570 mg, 4.12 mmol) and N-hydroxyacetamide (186 mg, 2.47 mmol) were added to a DMSO solution (2.75 mL) of methyl 6-chloro-5-fluoro-4-(2-fluoro-4-iodoanilino)pyridine-3-carboxylate (compound n2, 350 mg, 0.824 mmol), and the mixture was stirred for 1 hour at 100°C. Water was added to the reaction mixture, and the resulting solid was washed with water and DCM to give the title compound (281 mg, 84%) as a light brown solid.

LCMS m/z: 407 [M+H]$^+$

HPLC retention time: 0.72 min (analysis conditions G)

Compound n4:

N-[4-(Bromomethyl)-3-fluoropyridin-2-yl]-1,1-diphenylmethaneimine

[Chemical Formula 122]

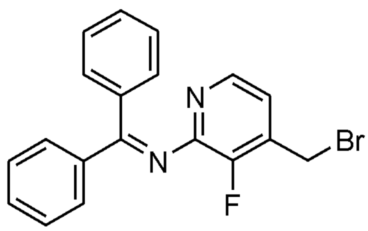

DIPEA (3.93 mL, 22.5 mmol) and methanesulfonic anhydride (2.07 g, 11.3 mmol) were added to an anhydrous DCM solution (37.5 mL) of [2-(benzhydrylideneamino)-3-fluoropyridin-4-yl]methanol (2.30 g, 7.51 mmol), and the mixture was stirred for 30 minutes at room temperature. An anhydrous THF solution (0.5 mL) of lithium bromide (3.26 g, 37.5 mmol) was then added, and the mixture was stirred for 2 hours at room temperature. Water was added to the reaction mixture and extraction was performed with DCM. The organic layer was dried over anhydrous sodium sulfate and, after filtering off the drying agent, concentrated under reduced pressure. The resulting residue was purified by reversed-phase column chromatography to give the title compound (990 mg, 34%) as a yellow semi-solid.
LCMS m/z: 369 $[M+H]^+$
HPLC retention time: 0.94 min (analysis conditions G)

Compound n5:

Methyl

1-[[2-(benzhydrylideneamino)-3-fluoropyridin-4-yl]methyl]-5-fluoro-4-(2-fluoro-4-iodoanilino)-6-oxopvridine-3-carboxylate

[Chemical Formula 123]

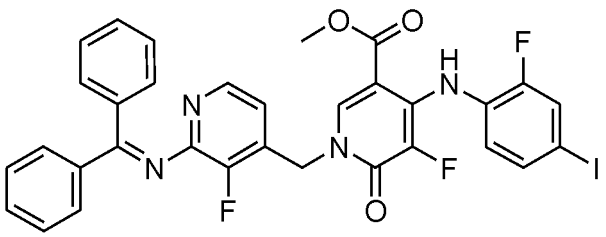

Lithium hydride (1.85 mg, 0.222 mmol) was added to an anhydrous DMF solution (0.739 mL) of methyl 5-fluoro-4-(2-fluoro-4-iodoanilino)-6-hydroxypyridine-3-carboxylate (compound n3, 30 mg, 0.074 mmol), and the mixture was stirred for 30 minutes at room temperature. An anhydrous THF solution (0.5 mL) of N-[4-(bromomethyl)-3-fluoropyridin-2-yl]-1,1-diphenylmethaneimine (compound n4, 82 mg, 0.222 mmol) was then added and the resulting mixture was stirred for 1 hour at room temperature. An anhydrous THF solution (0.5 mL) of lithium hydride (1 mg, 0.126 mmol) and compound n4 (25 mg, 0.068 mmol) was then further added, and stirring was continued for 1 hour at room temperature. The reaction mixture was cooled to 0°C, acetic acid (21.1 μL) and water were added, and extraction was performed with ethyl acetate. The organic layer was dried over anhydrous sodium sulfate and, after filtering off the drying agent, concentrated under reduced pressure. The resulting residue was purified by silica gel column chromatography (hexane/ethyl acetate) to give the title compound (19 mg, 37%) as a colorless solid.
LCMS m/z: 695 $[M+H]^+$
HPLC retention time: 1.05 min (analysis conditions G)

Compound n8:

1-[(2-Amino-3-fluoropyridin-4-yl)methyl]-5-fluoro-4-(2-fluoro-4-iodoa nilino)-6-oxopyridine-3-carboxamide

[Chemical Formula 124]

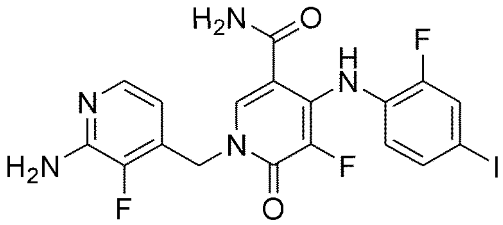

The title compound was synthesized from methyl 1-[[2-(benzhydrylideneamino)-3-fluoropyridin-4-yl]methyl]-5-fluoro-4-(2-fluoro-4-iodoanilino)-6-oxopyridine-3-carboxylate (compound n5) under the same conditions as the production examples for compound a6, compound a7 and compound K-10. However, 4 M hydrochloric acid was added during the initial step in which the reaction was carried out under the same conditions as the production example for compound a6.

LCMS m/z: 516 $[M+H]^+$

HPLC retention time: 0.52 min (analysis conditions B)

Compound N-1:

5-Fluoro-4-(2-fluoro-4-iodoanilino)-1-[[3-fluoro-2-(methylsulfamoylam ino)pyridin-4-yl]methyl]-6-oxopyridine-3-carboxamide

[Chemical Formula 125]

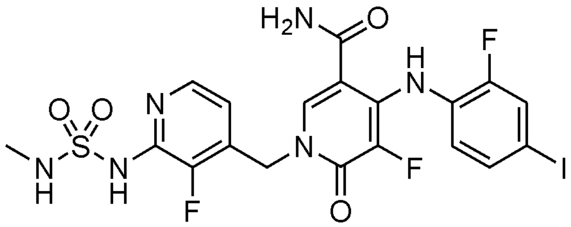

The title compound was synthesized from 1-[(2-amino-3-fluoropyridin-4-yl)methyl]-5-fluoro-4-(2-fluoro-4-iodoan ilino)-6-oxopyridine-3-carboxamide (compound n8) under the same conditions as the production example for compound A-1.

LCMS m/z: 609 $[M+H]^+$

HPLC retention time: 0.94 min (analysis conditions E)

Compound N-2:

5-Fluoro-4-(2-fluoro-4-iodoanilino)-1-[[3-fluoro-2-(propylsulfamoylami no)pyridin-4-yl]methyl]-6-oxopyridine-3-carboxamide

[Chemical Formula 126]

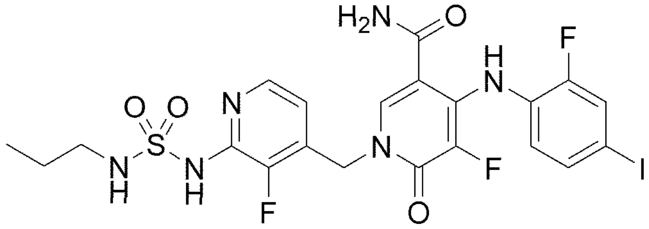

The title compound was synthesized from 1-[(2-amino-3-fluoropyridin-4-yl)methyl]-5-fluoro-4-(2-fluoro-4-iodoanilino)-6-oxopyridine-3-carboxamide (compound n8) and the corresponding 4-nitrophenyl sulfamate under the same conditions as the production example for compound A-1.

LCMS m/z: 637 $[M+H]^+$

HPLC retention time: 1.04 min (analysis conditions E)

Compound p3:

Methyl

4-(2-fluoro-4-iodoanilino)-6-hydroxy-5-methylpyridine-3-carboxylate

[Chemical Formula 127]

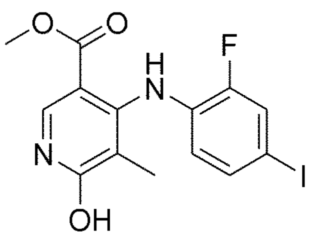

The title compound was synthesized from 4,6-dichloro-5-methylpyridine-3-carboxylic acid under the same conditions as the production examples for compound c5, compound a1 and compound n3. However, 2-fluoro-4-iodoaniline was used instead of 4-iodo-2-methylaniline, which was used in the production example for compound c5.

LCMS m/z: 403 $[M+H]^+$

HPLC retention time: 0.76 min (analysis conditions G)

Compound P-1:

4-(2-Fluoro-4-iodoanilino)-1-[[3-fluoro-2-(methylsulfamoylamino)pyri din-4-yl]methyl]-5-methyl-6-oxopyridine-3-carboxamide

[Chemical Formula 128]

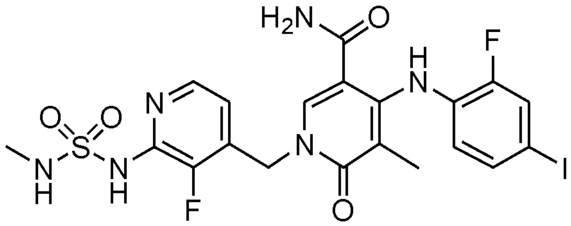

The title compound was synthesized from methyl 4-(2-fluoro-4-iodoanilino)-6-hydroxy-5-methylpyridine-3-carboxylate (compound p3) under the same conditions as the production examples for compound n4, compound b2, compound m17, compound a6 and compound A-1. However, a 2 M sodium hydroxide aqueous solution was used instead of lithium hydroxide monohydrate, which was used in the production example for compound b2, and a 4 M hydrochloric acid 1,4-dioxane solution was used instead of trifluoroacetic acid, which was used in the production example for compound a6.

LCMS m/z: 605 $[M+H]^+$

HPLC retention time: 0.66 min (analysis conditions B)

[Test Examples]

The compounds in the following test examples that were referred in the production examples are represented by the same compound numbers as in the production examples. The compound denoted as "ref-1" is the compound represented by formula (A) below, which is compound 34 in Bioorg. Med. Chem. Lett. 2008, vol. 18, no. 24, p. 6501-6504. The compound denoted as "ref-2" is the compound represented by formula (B) below, which is compound 27 in Bioorg. Med. Chem. Lett. 2013, vol. 23, no. 8, p. 2384-2390. The compounds denoted as "ref-3" and "ref-4" are the compounds represented by formulas (C) and (D) below respectively, which are compound 9 and compound 10 in Chem. Med. Chem. 2015, vol. 10, no. 12, p. 2004-2013. The compound denoted as "ref-5" is the compound represented by formula (E) below, which is compound 1 in ACS Medchem. Lett. 2014, vol. 5, no. 4, p. 309-314.

ref-1:

N-[(2R)-2,3-Dihydroxypropoxy]-3,4-difluoro-2-(2-fluoro-4-iodoanilino )benzamide (PD0325901)

[Chemical Formula 129]

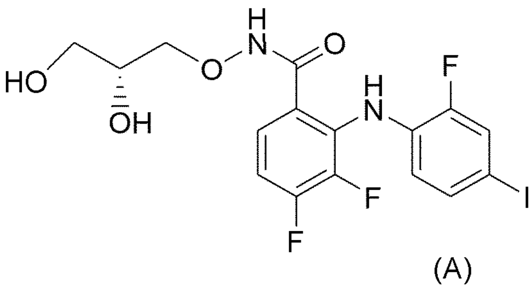

(A)

ref-2:

4-Fluoro-2-(2-fluoro-4-iodoanilino)-6-[3-(methylsulfamoylamino)phen oxy]benzamide

[Chemical Formula 130]

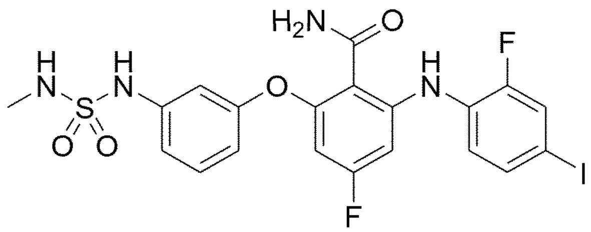

(B)

ref-3:

3-(2-Fluoro-4-iodoanilino)-5-[3-(propan-2-ylsulfonylamino)phenoxy]py ridine-4-carboxamide

[Chemical Formula 131]

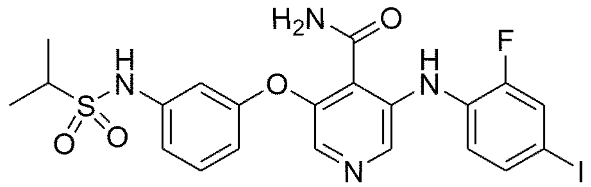

(C)

ref-4:

3-[3-(Cyclopropylsulfonylamino)phenoxyl-5-(2-fluoro-4-iodoanilino)p yridine-4-carboxamide

[Chemical Formula 132]

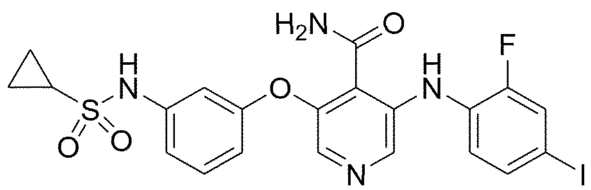

(D)

ref-5:

3-[[3-Fluoro-2-(methylsulfamoylamino)pyridin-4-yl]methyl]-4-methyl-7-pyrimidin-2-yloxychromen-2-one (CH5126766)

[Chemical Formula 133]

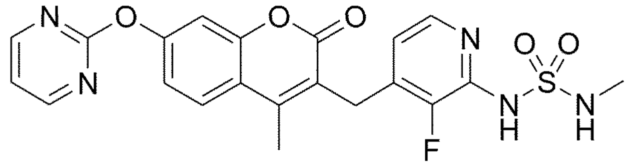

(E)

(Test Example 1)

Effect on interaction between RAF1 and MEK1

The effects of the compounds of Figs. 4 to 7 on interaction between RAF1 and MEK1 were investigated as described below using a Biacore 8K (GE Healthcare).

GST tag-fused RAF1 (Cama Biosciences) was immobilized on the surface of a Sensor Chip CM5 (GE Healthcare) using Anti-GST Antibody (GE Healthcare). Next, a running buffer (blank), a 40 nM MEK1 solution, or a mixed solution of 40 nM MEK1 and 3 $\mu$M test compound was flowed over the surface of the sensor chip for 120 seconds, and the running buffer was then flowed over it. The MEK1 used was MEK1 Recombinant Human protein, Inactive (Thermo Fisher Scientific). The running buffer used was PBS (Sigma-Aldrich) with the addition of 1 mM DTT (Wako), 10 mM $MgCl_2$ (Wako), 500 $\mu$M ATP (Wako), 0.01% Tween20 (Junsei-Kagaku) and 1% DMSO (Sigma-Aldrich), and this running buffer was also used for preparation of the sample solution. The measurement was carried out at 15°C. Both RAF1 and MEK1 were subjected to dephosphorylation treatment with Lambda Protein Phosphatase (New England Biolabs) before use, and MEK1 was purified by size exclusion chromatography.

The obtained sensorgrams (graphs representing change over time in the amount of MEK1 bound to immobilized RAF1) were double-referenced with Biacore Insight Evaluation Software, and normalization of the sensorgrams by the amount of immobilized RAF1 was performed using TIBCO Spotfire. The normalized sensorgrams are shown in Figs. 4 to 7. The experiment ID, the channel No. in the Biacore, and the compound No. are listed in order on each sensorgram (with "no compound" meaning that no test compound is present). For each sensorgram, the horizontal axis (X-axis) represents the time (sec) after the start of addition of the sample solution and the vertical axis (Y-axis) represents the normalized amount of binding of MEK1.

(Test Example 2)

Effect on phosphorylation of MEK and ERK

The effects of the compounds listed in Fig. 8 (ref-5 and compound A-1) on intracellular phosphorylation of MEK and ERK were investigated by Western blotting as described below.

A549 cells were seeded in a 12-well plate at 400,000 cells per well and cultured in a 5% carbon dioxide gas incubator at 37°C using Dulbecco's Modified Eagle's Medium with the addition of 10% fetal bovine serum (Sigma). On the following day, the test compound (0.3 $\mu$M ref-5 or 0.05 $\mu$M compound A-1) or DMSO was added to the culture medium and cultured for 30 minutes or 2 hours, and the cells were harvested with a cell scraper and solubilized. The extracted protein was separated by SDS-PAGE and transferred to a PVDF membrane. After blocking, the PVDF membrane was treated with Phospho-MEK1/2 (Ser217/221) antibody, MEK1/2 antibody, Phospho-ERK1/2 (Thr202/Tyr204) antibody, or ERK1/2 antibody (all by Cell Signaling Technology, Inc.). After washing the primary antibody, it was treated with HRP-labeled secondary antibody (Cell Signaling Technology, Inc.), and after washing, the signal was detected by chemiluminescence using a Chemi-Lumi One Super (Nacalai Tesque Inc.). Fig. 8 is electrophoresis images showing the results of the Western blotting. In Fig. 8, "p-MEK" and "p-ERK" represent phosphorylated MEK and phosphorylated ERK, respectively.

(Test Example 3)

MEK1-inhibiting activity

The MEK1-inhibiting activity of the compounds listed in Table 6 below were evaluated by the fluorescent polarization method as described below.

The test compound, CRAF (Thermo Fisher Scientific Inc.), MEK1 (Thermo Fisher Scientific Inc.) and ERK2 (Cama Biosciences, Inc.) were mixed in ATP-containing buffer and reacted for 60 minutes at 30°C. FAM-labeled ERKtide (Molecular Devices Corp.) was then added and reaction was continued for 45 minutes at 30°C. IMAP (registered trademark) Progressive Binding Reagent (Molecular Devices Corp.) was further added, and reaction was continued for 15 minutes at room temperature. Following the reaction, the fluorescent polarization was measured with a fluorescent plate reader and the 50% inhibition concentration ($IC_{50}$) was calculated based on the percent inhibition relative to a test compound-free control. The results are shown in Table 6.

(Test Example 4)

BRAF-inhibiting activity

The BRAF-inhibiting activity of the compounds listed in Table 6 below was evaluated by the time-resolved fluorescence-fluorescence resonance energy transfer assay as described below.

The test compound, BRAF (Eurofins Genomics KK.) and MEK1 (Thermo Fisher Scientific Inc.) were mixed in ATP-containing buffer and reacted for 90 minutes at 30°C. LANCE (registered trademark) Eu-Phospho-MEK1/2(Ser217/221) antibody (Perkin-Elmer) was then added, and reaction was continued for 60 minutes at room temperature. Following the reaction, the fluorescence resonance energy transfer was measured with a fluorescent plate reader and the 50% inhibition concentration ($IC_{50}$) was calculated based on the percent inhibition relative to a test compound-free control. The results are shown in Table 6.

(Test Example 5)

Cell proliferation-inhibiting activity

The cell proliferation-inhibiting activity of the compounds listed in Table 6 below was evaluated by measuring the amount of ATP in viable cells as described below.

The test compound was serially diluted with DMSO and then diluted 25-fold with $Ca^{2+}$, $Mg^{2+}$-free phosphate-buffered saline, and was then dispensed into a 96-well plate at 5 μL per well. A cell suspension containing the below indicated concentration of A549, Calu-6 or NCI-H2122 human lung cancer cells (all obtained from ATCC) was prepared using the below indicated medium with the addition of 10% fetal bovine serum (Sigma). The cell suspension was dispensed into the test compound-added plate at 95 μL per well and cultured in a 5% carbon dioxide gas incubator at 37°C. After 4 days, 80 μL of CellTiter-Glo (registered trademark) (Promega Corp.) was added to each well and the bioluminescence was measured with a fluorescent plate reader. The 50% inhibition concentration ($IC_{50}$) was calculated based on the percent inhibition relative to a test compound-free control. The results are shown in Table 6.

A549: Dulbecco's Modified Eagle's Medium (Sigma); 2000 cells/ 95 μL
Calu-6: Eagle's Minimum Essential Medium (Sigma); 4000 cells/ 95 μL
NCI-H2122: RPMI-1640 culture medium (Sigma); 2000 cells/ 95 μL

(Test Example 6)

Human liver microsome stability

The compounds listed in Table 6 below were tested for metabolic stability in human liver microsomes as described below using a Biomek3000 (Beckman Coulter).

There were dispensed 400 μL of 1 mg/mL human liver microsomes (XENOTECH)/ 0.1 M potassium phosphate buffer (pH 7.4) into each well of a 96-well plate. A DMSO solution (4 μL) of 200 μM test compound was then added, and incubation was conducted until reaching 37°C. To this reaction mixture (200 μL) there was added a solution (200 μL) containing 2 mM NADPH (ORIENTAL YEAST)/ 0.1 M potassium phosphate buffer (pH 7.4) that had been incubated at 37°C. At 0 min, 5 min, 15 min or 30 min after addition, the reaction mixture (50 μL) was added to acetonitrile (100 μL) to stop the metabolic reaction. After the metabolic reaction was stopped, a 1 μM warfarin aqueous solution (50 μL) was added to each reaction mixture as an internal standard. The reaction mixture was filtered and analyzed by LC/MS/MS (LC: NEXERA by Shimadzu; MS: 4000Q trap by ABSciex; column: Ascentis Express C18 HPLC column (5 cm × 2.1 mm, 2.7 μm); ionization method: electrospray ionization). The percent remaining relative to the amount of test compound at 0 min was calculated from the obtained peak area ratio of the test compound to internal standard. The elimination rate constant (ke) was calculated from the incubation time and percent remaining using the primary elimination rate equation, and the hepatic intrinsic clearance (CLint) was calculated using the formula shown below. The results are

shown in Table 6.

CLint (μL/min/mg) = ke ($min^{-1}$)/human liver microsome concentration (mg protein/ μL)

[Table 6]

| Table 6 | | | | | | |
|---|---|---|---|---|---|---|
| Compound No. | MEK1 $IC_{50}$ (nM) | BRAF $IC_{50}$ (nM) | A549 $IC_{50}$ (nM) | Calu-6 $IC_{50}$ (nM) | NCI-H2122 $IC_{50}$ (nM) | CLint (μL/min/mg) |
| A-1 | 17 | 1 | 26 | 64 | 5 | 1 |
| A-2 | 5 | 3 | 1 | 2 | 0.1 | 25 |
| A-8 | 3 | 4 | 0.4 | 2 | 0.1 | 33 |
| A-18 | 8 | 4 | 5 | 7 | 2 | 53 |
| A-20 | 23 | 2 | 7 | ND | 6 | 14 |
| A-25 | 5 | 2 | 0.5 | 2 | 0.2 | 7 |
| A-27 | 32 | 7 | 24 | ND | 10 | 4 |
| A-33 | 10 | 2 | 27 | 68 | 8 | 6 |
| B-1 | 2 | 5 | 6 | 11 | 2 | 15 |
| E-1 | 40 | 12 | 2 | 6 | 0.4 | 17 |
| E-7 | 334 | 6 | 1 | 8 | 0.4 | 5 |
| E-13 | 290 | 1 | 30 | 84 | 6 | 25 |
| H-1 | 7 | 2 | 9 | 21 | 5 | 39 |
| H-3 | 2 | 4 | 5 | ND | 1 | 149 |
| H-4 | 8 | 12 | 9 | 6 | 1 | 11 |
| I-1 | 25 | 3 | 5 | 15 | 3 | 19 |
| J-1 | 58 | 4 | 38 | 29 | 8 | 11 |
| K-10 | 128 | 8 | 25 | 81 | 16 | 69 |
| L-1 | 6 | 2 | 21 | 41 | 17 | 10 |
| M-1 | 5 | 4 | 27 | 28 | 13 | 59 |
| N-1 | 1 | 2 | 20 | ND | 28 | 8 |
| N-2 | 1 | 1 | 193 | ND | 60 | 11 |
| P-1 | 2 | ND | ND | 22 | 6 | 15 |
| ref 1 | 7 | 17 | 7 | 91 | 7 | 20 |
| ief-2 | 364 | 12 | 8 | 35 | 3 | 12 |
| ref-3 | 5 | 12 | 2 | 10 | 1 | 11 |
| ref-4 | 11 | 9 | 2 | 18 | 2 | 32 |
| ref-5 | 292 | 11 | 113 | 418 | 27 | <1 |
| ND: Not determined | | | | | | |

(Test Example 7)

*In vivo* antitumor effect

The effect of compound A-1 against KRAS-mutant cancer cells was evaluated as described below using tumor-bearing mice.

Calu-6 KRAS-mutant human lung cancer cells were transplanted into nude mice (CAnN.Cg-Foxn1nu/CrlCrlj, female, 5-week-old, Charles River) by subcutaneous injection of a cell suspension into the lateral abdomen using a 26G needle. The mice were divided into 5 groups (8 mice per group) according to the dosage of the test compound and administration of the test compound was initiated at 17 days post transplantation, when the tumor volume reached approximately 200 $mm^3$. The mice in 4 groups (A-1 treatment groups) were orally administered 0.0625 mg/kg, 0.25 mg/kg, 1 mg/kg or 4 mg/kg of compound A-1 each time using 10% DMSO/ 10% Cremophor EL/ 15% PEG400/ 15% HPCD as the solvent (vehicle). The mice in the remaining group (vehicle control) were orally administered the solvent alone. Administration of the test compound or solvent was carried out once per day for 10 days.

The tumor volumes were measured at 20 days, 24 days and 27 days post transplantation. The tumor volumes were calculated by the formula shown below after measuring the tumor long diameters and short diameters using a caliper. The results are shown in Fig. 9. Fig. 9 is a graph showing change over time in tumor volume (mean ± SD). The horizontal axis (X-axis) represents the days post transplantation and the vertical axis (Y-axis) represents the tumor volume.

$$\text{Tumor volume (mm}^3\text{)} = 1/2 \times \text{long diameter (mm)} \times \text{short diameter (mm)} \times \text{short diameter (mm)}$$

**Claims**

1. A method for producing a compound represented by general formula (1) below or a pharmaceutically acceptable salt thereof, or a pharmaceutically acceptable solvate of said compound or salt, the method comprising:

   (I) reacting a compound represented by general formula (2) below with a compound represented by $X_1$-$R_9$ and a base in a solvent to give a compound represented by general formula (4) below:

[Chemical Formula 1]

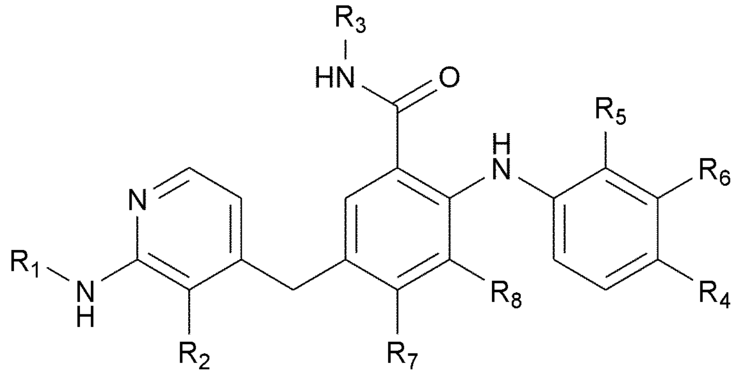

(1)

[Chemical Formula 2]

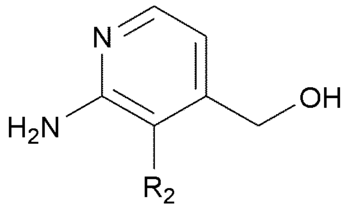

(2)

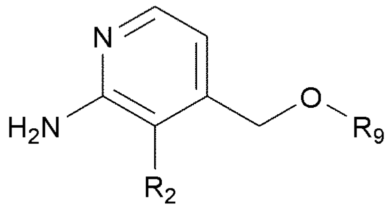

(4)

wherein:

$R_1$ is -S(=O)$_2$-NH-$R_{11}$ or -S(=O)$_2$-$R_{11}$;
$R_{11}$ is a hydrogen atom, a C1-6 alkyl group (the C1-6 alkyl group being optionally substituted with a halogen atom, a hydroxy group, or a C1-6 alkoxy group), or a C3-6 cycloalkyl group (the C3-6 cycloalkyl group being optionally substituted with a C1-6 alkyl group);
$R_2$ is a hydrogen atom, a halogen atom, or a C1-6 alkyl group;
$R_3$ is a hydrogen atom, a C1-6 alkyl group (the C1-6 alkyl group being optionally substituted with a halogen atom, a hydroxy group, or a C1-6 alkoxy group), a C3-6 cycloalkyl group (the C3-6 cycloalkyl group being optionally substituted with a halogen atom or a C1-6 alkyl group), or a C1-6 alkoxy group (the C1-6 alkoxy group being optionally substituted with a halogen atom, a hydroxy group, or a C1-6 alkoxy group);
$R_4$ is a hydrogen atom, a halogen atom, a C1-6 alkyl group, a C2-7 alkenyl group, a C2-7 alkynyl group, a C3-6 cycloalkyl group, or a C1-6 alkylthio group;
$R_5$ is a halogen atom or a C1-6 alkyl group;
$R_6$ is a hydrogen atom, a halogen atom, or a C1-6 alkyl group;
$R_7$ is a hydrogen atom, a halogen atom, or a C1-6 alkyl group;
$R_8$ is a hydrogen atom, a halogen atom, or a C1-6 alkyl group;
$X_1$ is a halogen atom or -O-$R_9$;
$R_9$ is -C(=O)-$R_{12}$, -C(=O)-O-$R_{12}$, or -P(=O)(-O-$R_{12}$)$_2$; and
$R_{12}$ is a C1-6 alkyl group or an aryl group.

**2.** The method according to claim 1, wherein the base used in step (I) is at least one selected from the group consisting of N,N-dimethylaminopyridine and 1-methylimidazole.

**3.** The method according to claim 1 or 2, wherein the solvent used in step (I) is at least one selected from the group consisting of acetonitrile, tetrahydrofuran, 2-methyltetrahydrofuran, 4-methyltetrahydropyran, cyclopentyl methyl ether, and tert-butyl methyl ether.

**4.** The method according to any one of claims 1 to 3, further comprising:
(II) reacting the compound represented by general formula (4) with a compound represented by general formula (10) below in a solvent in the presence of a catalyst to give a compound represented by general formula (5) below:

[Chemical Formula 3]

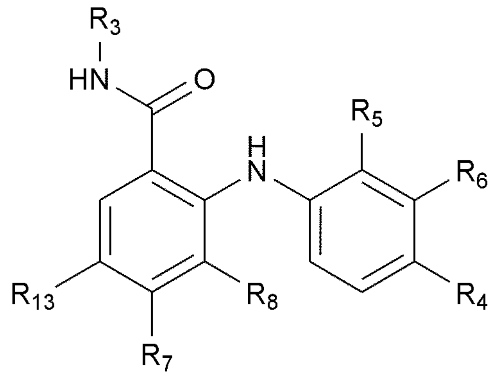

(10)

[Chemical Formula 4]

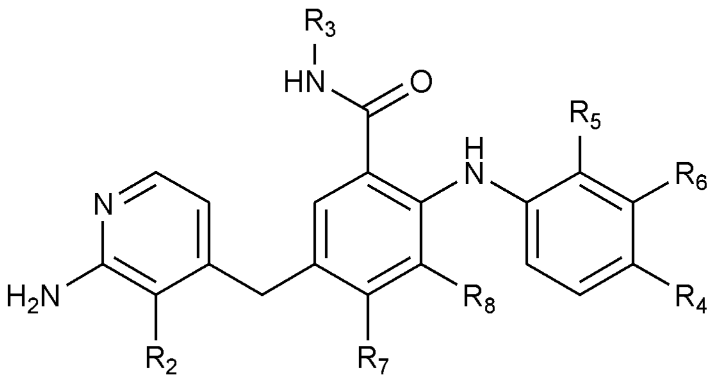

(5)

wherein:

$R_{13}$ is -B(-$OR_{14}$)(-$OR_{15}$) or -$BF_3K$;
$R_{14}$ and $R_{15}$ are each independently a hydrogen atom or a C1-6 alkyl group (the C1-6 alkyl group being optionally substituted with a C1-6 alkoxy group or an aryl group), or $R_{14}$ and $R_{15}$ form a 5- to 8-membered saturated or unsaturated ring together with the intervening oxygen and boron atoms (the ring being optionally substituted with a C1-6 alkyl group, a C1-6 alkoxy group, or an aryl group and being optionally fused with a benzene ring); and
$R_2$ to $R_9$ have the same definitions as above.

**5.** The method according to claim 4, wherein the catalyst used in step (II) is at least one selected from the group consisting of:

a combination of bis(allylchloropalladium) and 2',6'-dimethoxy-2-(dicyclohexylphosphino)biphenyl;
a combination of bis(allylchloropalladium) and 2-dicyclohexylphosphino-2',6' -diisopropoxybiphenyl;
a combination of bis(allylchloropalladium) and 2-dicyclohexylphosphino-2'-(N,N-dimethylamino)biphenyl;
(2-dicyclohexylphosphino-2' ,6' -dimethoxybiphenyl) [2-(2'-amin o-1,1'-biphenyl)]palladium(II) methanesulfonate;
2-dicyclohexylphosphino-2-(N,N-dimethylamino)biphenyl(2'-a mino-1,1'-biphenyl-2-yl)palladium(II) methanesulfonate; and
(2-dicyclohexylphosphino-2',6'-diisopropoxy-1,1'-biphenyl)[2-( 2'-amino-1,1'-biphenyl)]palladium(II) methanesulfonate.

**6.** The method according to claim 4 or 5, wherein the solvent used in step (II) contains a C1-6 alcohol.

**7.** The method according to any one of claims 4 to 6, further comprising:

(III) reacting the compound represented by general formula (5) with a compound represented by $X_2$-S(=O)$_2$-NH-$R_{11}$ or $X_2$-S(=O)$_2$-$R_{11}$ to give a compound represented by general formula (1) or a salt thereof or a solvate of said compound or salt;
wherein $X_2$ is a halogen atom and $R_{11}$ has the same definition as above.

**8.** The method according to any one of claims 1 to 7, wherein:

$R_9$ is -C(=O)-O-$R_{12}$; and
$R_{12}$ is a C1-6 alkyl group or an aryl group.

**9.** The method according to any one of claims 1 to 8, wherein:

$R_2$ is a fluorine atom;
$R_1$ is -S(=O)$_2$-NH-$R_{11}$;
$R_{11}$ is a C1-4 alkyl group;
$R_3$ is a hydrogen atom or a cyclopropyl group;
$R_5$ is a fluorine atom;
$R_6$ is a hydrogen atom;
$R_4$ is an iodine atom or a cyclopropyl group;
$R_7$ is a fluorine atom;
$R_8$ is a fluorine atom; and
$X_1$ is a chlorine atom.

**10.** The method according to any one of claims 1 to 9, wherein the compound represented by general formula (1) is 2-(4-cyclopropyl-2-fluoroanilino)-3,4-difluoro-5-[[3-fluoro-2-(methylsu lfamoylamino)pyridin-4-yl]methyl]benzamide.

**11.** A composition comprising a sodium salt of 2-(4-cyclopropyl-2-fluoroanilino)-3,4-difluoro-5-[[3-fluoro-2-(methylsu lfamoylamino)pyridin-4-yl]methyl]benzamide and a compound represented by formula (X) below or a sodium salt thereof, wherein the amount of the compound of formula (X) or sodium salt thereof that is contained in the composition is 3.0% w/w or less with respect to the weight of the sodium salt of 2-(4-cyclopropyl-2-fluoroanilino)-3,4-difluoro-5-[[3-fluoro-2-(methylsu lfamoylamino)pyridin-4-yl]methyl]benzamide that is contained in the composition.

[Chemical Formula 5]

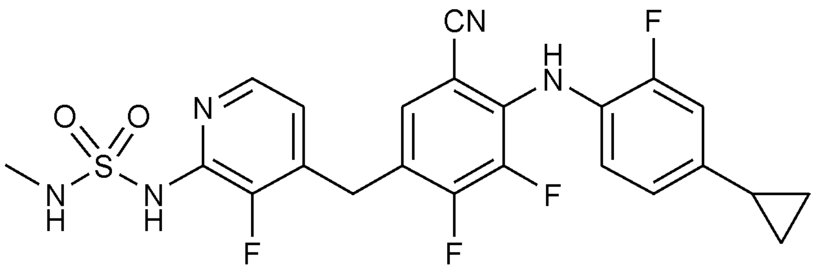

(X)

**12.** A method for producing a compound represented by general formula (4) below, the method comprising:

(I) reacting a compound represented by general formula (2) below with a compound represented by $X_1$-$R_9$ and a base in a solvent to give a compound represented by general formula (4):

[Chemical Formula 6]

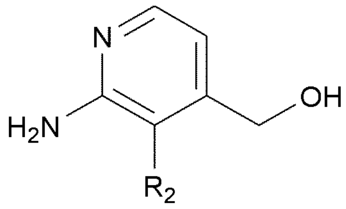

(2)

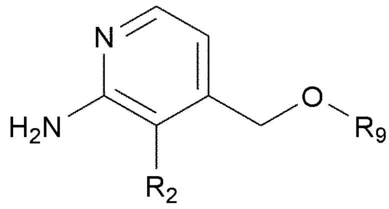

(4)

wherein:

$R_2$ is a hydrogen atom, a halogen atom, or a C1-6 alkyl group;
$X_1$ is a halogen atom or -O-$R_9$;
$R_9$ is -C(=O)-$R_{12}$, -C(=O)-O-$R_{12}$, or -P(=O)(-O-$R_{12}$)$_2$; and
$R_{12}$ is a C1-6 alkyl group or an aryl group.

**13.** The method according to claim 12, wherein the base used in step (I) is at least one selected from the group consisting of N,N-dimethylaminopyridine and 1-methylimidazole.

**14.** The method according to claim 12 or 13, wherein the solvent used in step (I) is at least one selected from the group consisting of acetonitrile, tetrahydrofuran, 2-methyltetrahydrofuran, 4-methyltetrahydropyran, cyclopentyl methyl ether, and tert-butyl methyl ether.

**15.** The method according to any one of claims 12 to 14, wherein:

$R_9$ is -C(=O)-O-$R_{12}$; and
$R_{12}$ is a C1-6 alkyl group or an aryl group.

**16.** (2-Amino-3-fluoropyridin-4-yl)methyl methyl carbonate.

Fig.1

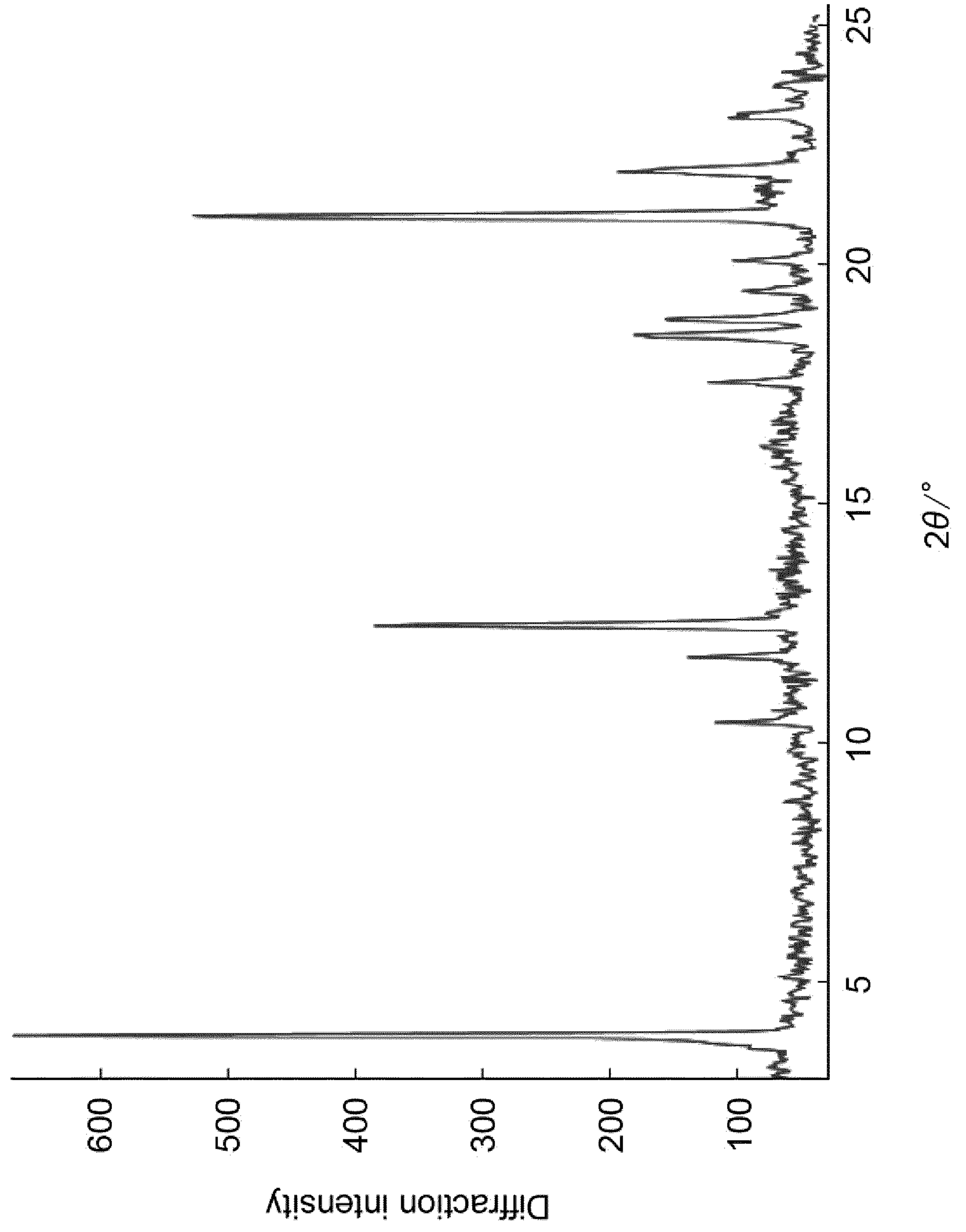

Fig.2

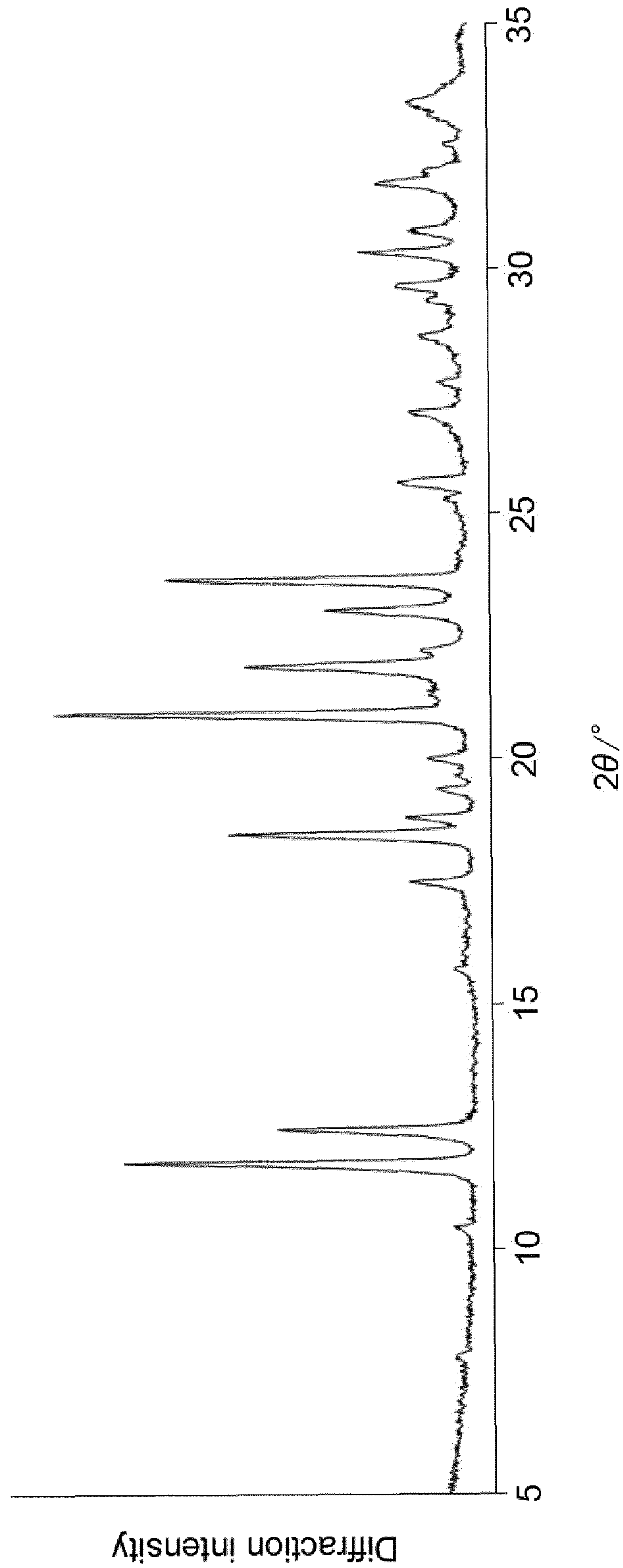

Fig.3

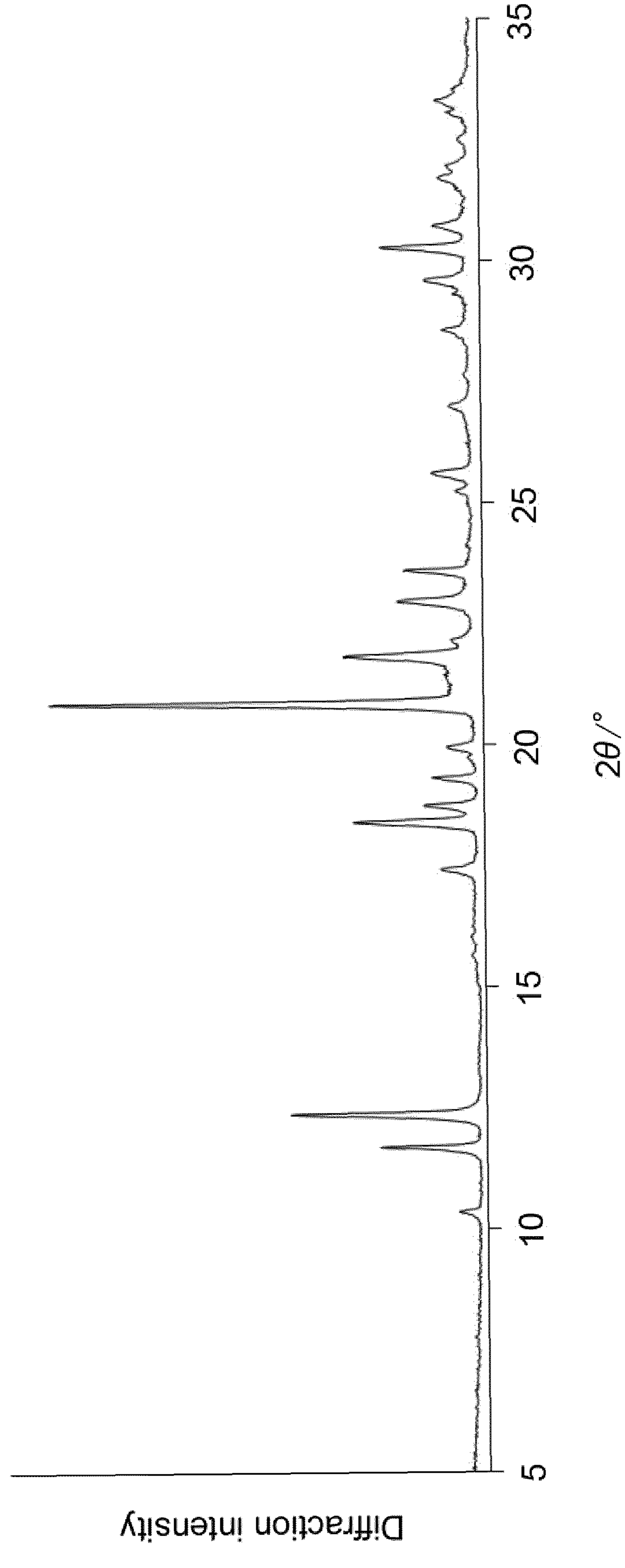

Fig.4

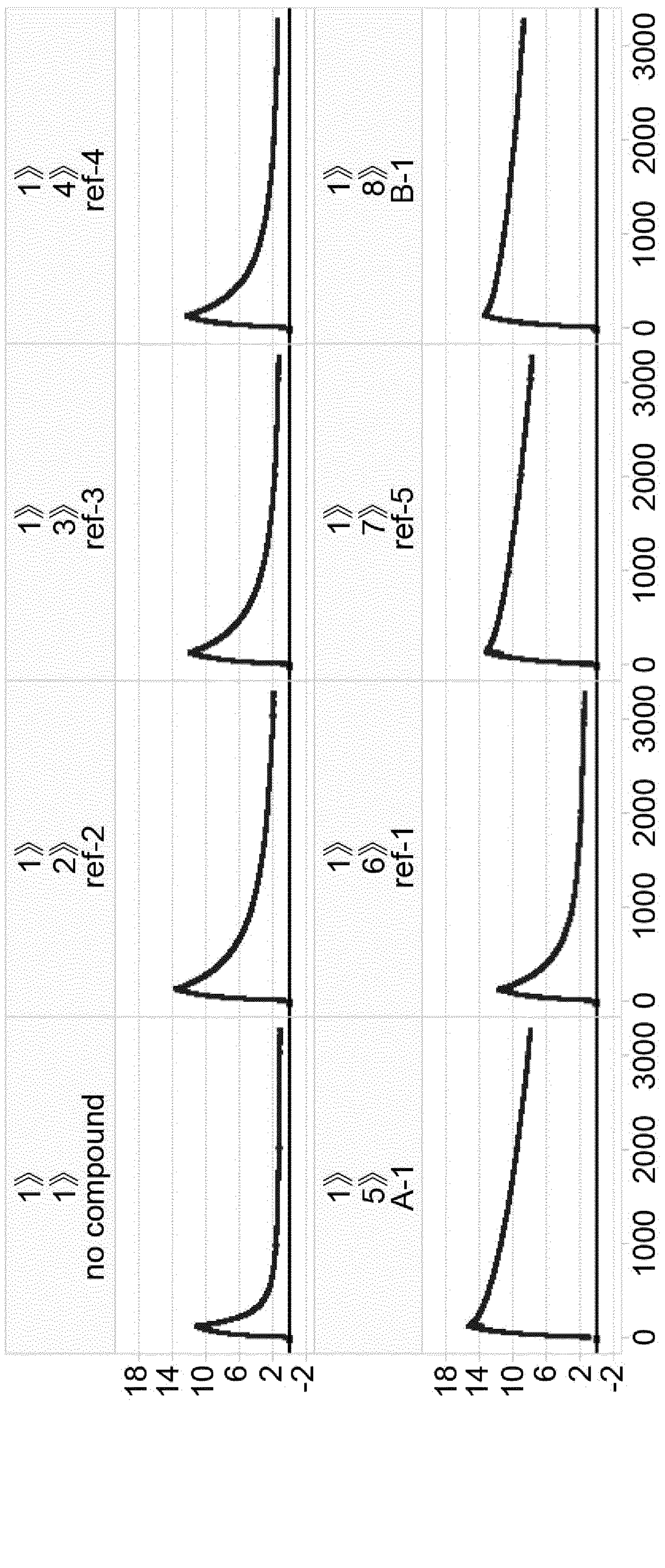
1》 1》 no compound
1》 2》 ref-2
1》 3》 ref-3
1》 4》 ref-4
1》 5》 A-1
1》 6》 ref-1
1》 7》 ref-5
1》 8》 B-1
18
14
10
6
2
-2
0
1000
2000
3000

*Fig.5*

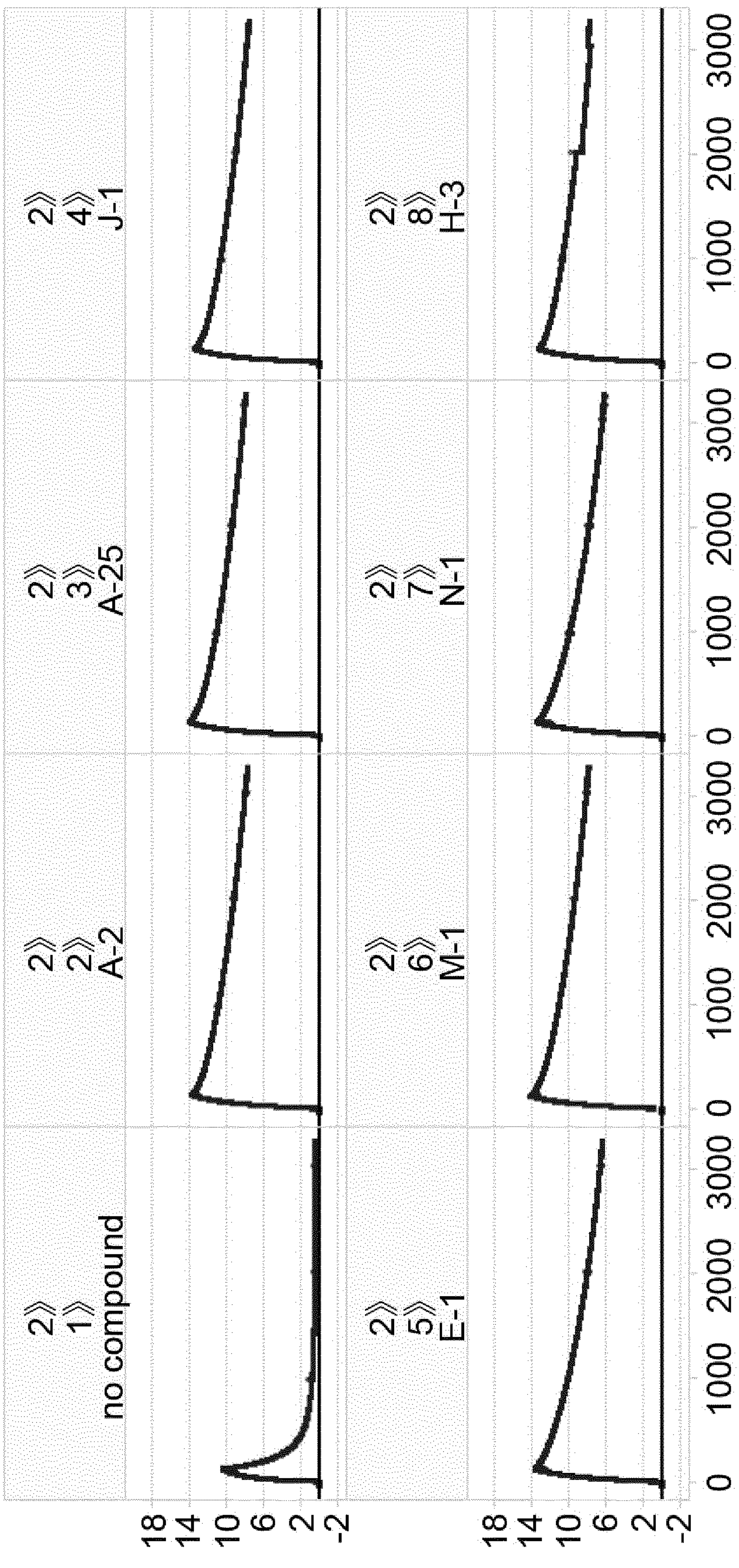
2》
1》
no compound
2》
2》
A-2
2》
3》
A-25
2》
4》
J-1
2》
5》
E-1
2》
6》
M-1
2》
7》
N-1
2》
8》
H-3
18
14
10
6
2
-2
0
1000
2000
3000

**Fig.6**

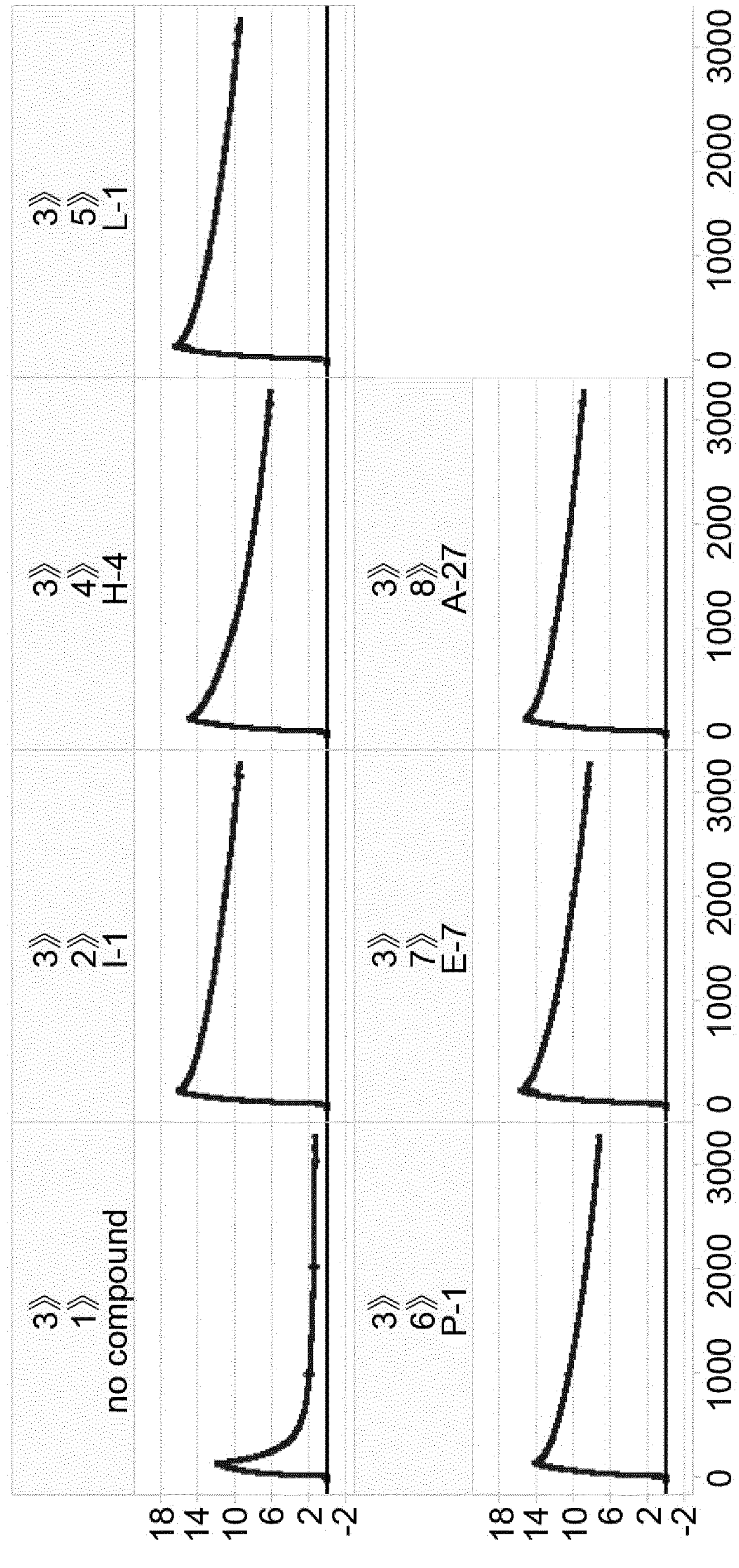
3》 1》 no compound
3》 2》 I-1
3》 4》 H-4
3》 5》 L-1
3》 6》 P-1
3》 7》 E-7
3》 8》 A-27
18 14 10 6 2 -2
0 1000 2000 3000

Fig.7

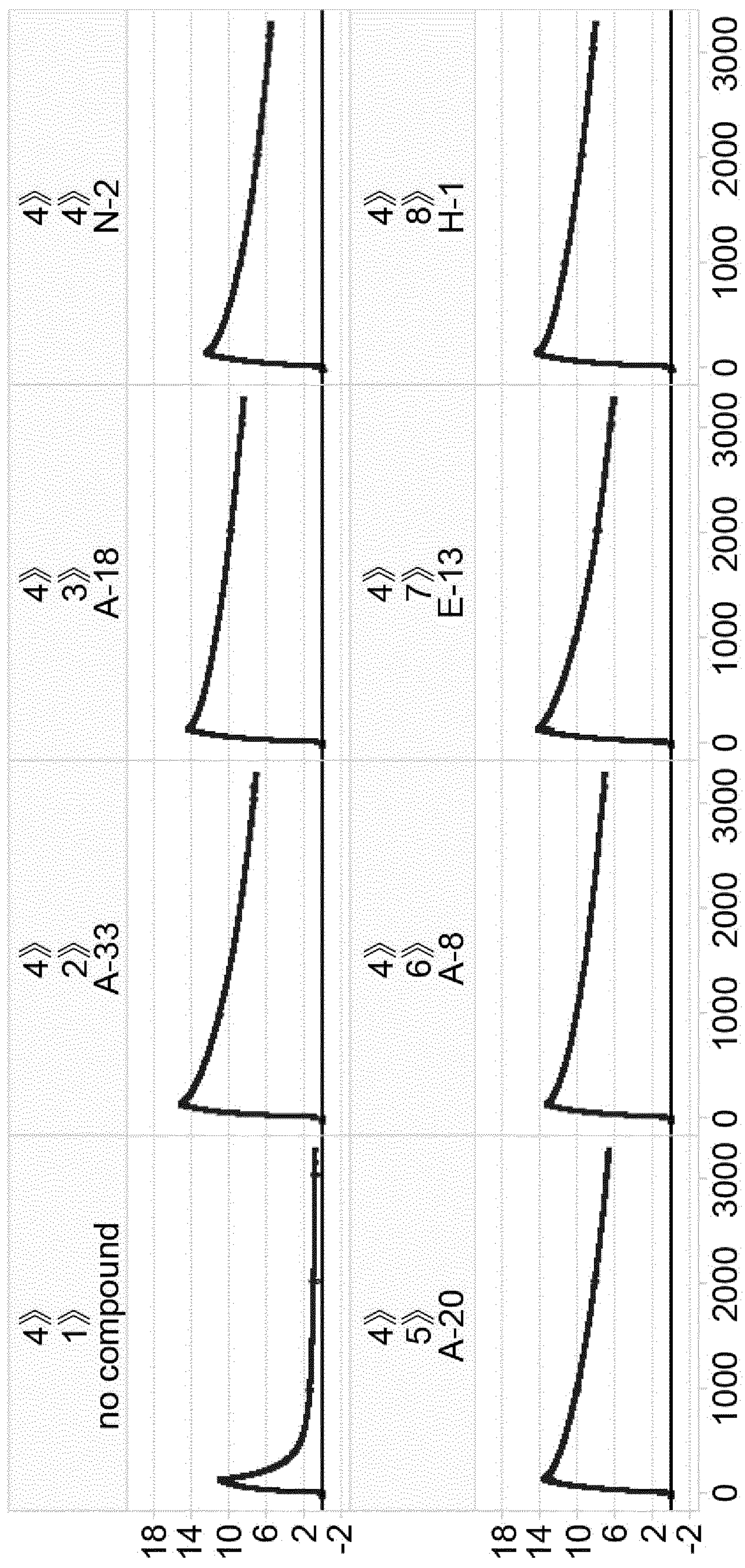
4》 1》 no compound
4》 2》 A-33
4》 3》 A-18
4》 4》 N-2
4》 5》 A-20
4》 6》 A-8
4》 7》 E-13
4》 8》 H-1
18 14 10 6 2 -2
0 1000 2000 3000

# Fig.8

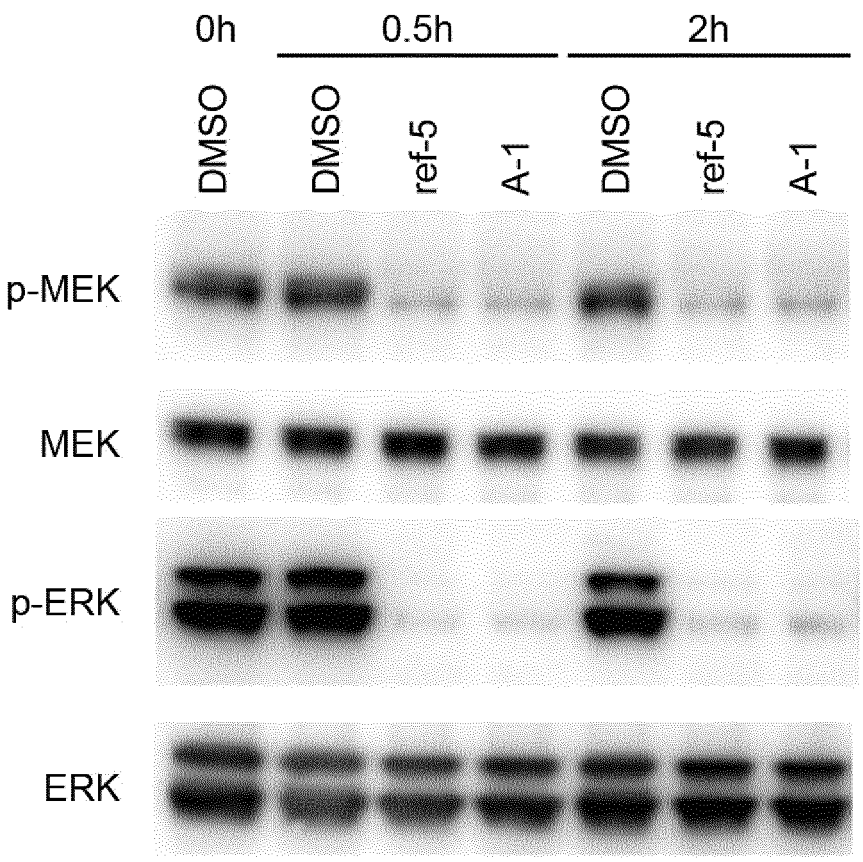

Fig.9

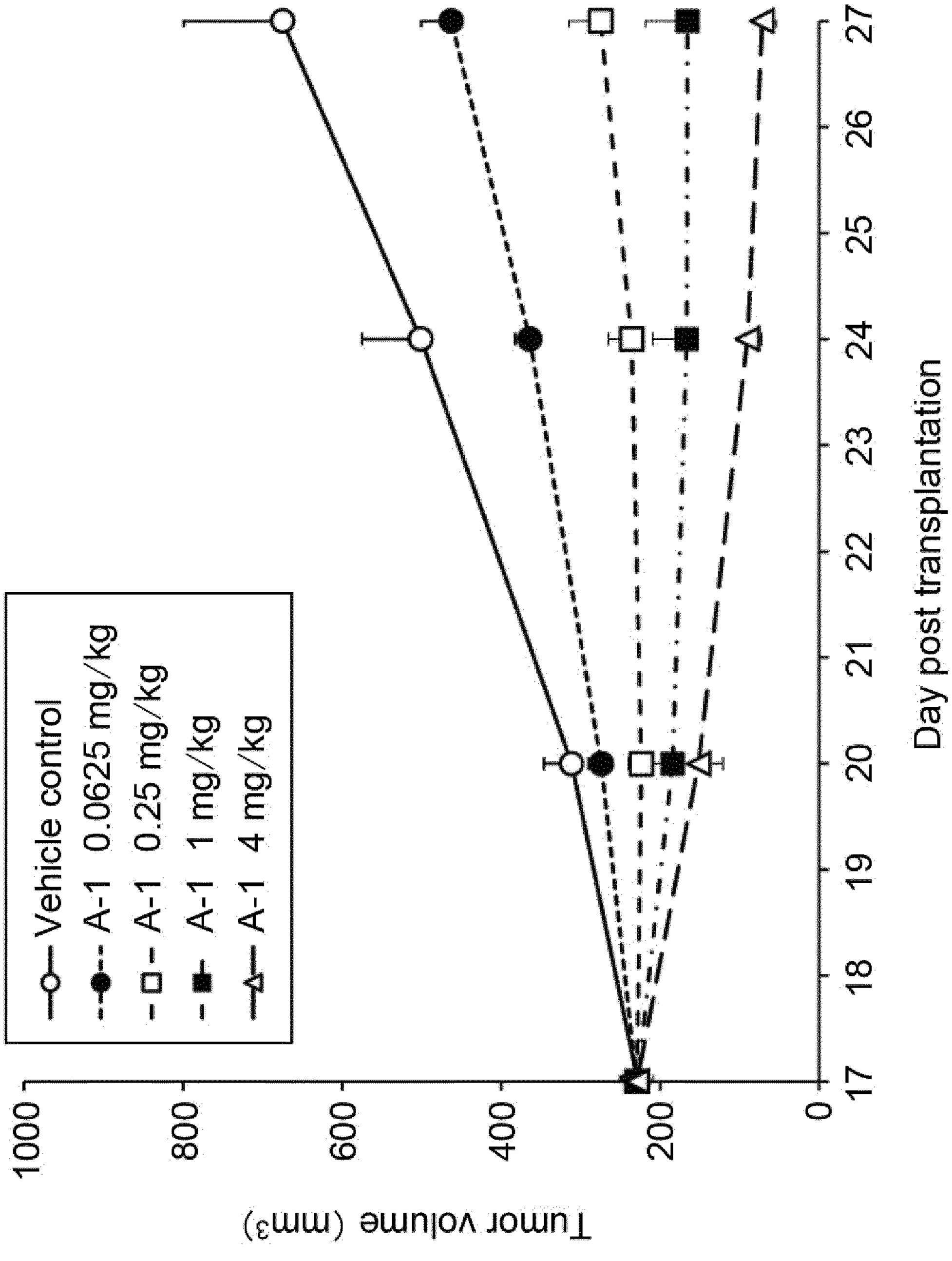
Vehicle control
A-1 0.0625 mg/kg
A-1 0.25 mg/kg
A-1 1 mg/kg
A-1 4 mg/kg
Tumor volume (mm³)
0
200
400
600
800
1000
17
18
19
20
21
22
23
24
25
26
27
Day post transplantation

## INTERNATIONAL SEARCH REPORT

International application No.
**PCT/JP2022/028187**

**A. CLASSIFICATION OF SUBJECT MATTER**

***C07D 213/76***(2006.01)i; ***A61K 31/44***(2006.01)i; ***A61K 31/4436***(2006.01)i; ***A61P 35/00***(2006.01)i; ***B01J 31/22***(2006.01)i; ***B01J 31/24***(2006.01)i; ***C07B 61/00***(2006.01)i; ***C07D 213/73***(2006.01)i; ***C07D 333/66***(2006.01)i; ***C07D 409/12***(2006.01)i
FI: C07D213/76; A61K31/44; A61K31/4436; A61P35/00; B01J31/22 Z; B01J31/24 Z; C07B61/00 300; C07D213/73 CSP; C07D333/66; C07D409/12

According to International Patent Classification (IPC) or to both national classification and IPC

**B. FIELDS SEARCHED**

Minimum documentation searched (classification system followed by classification symbols)

C07D213/76; A61K31/44; A61K31/4436; A61P35/00; B01J31/22; B01J31/24; C07B61/00; C07D213/73; C07D333/66; C07D409/12

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | JP 2016-034900 A (CHUGAI PHARMACEUTICAL CO LTD) 17 March 2016 (2016-03-17) entire text | 1-16 |
| A | WO 2006/011466 A1 (CHUGAI PHARMACEUTICAL CO LTD) 02 February 2006 (2006-02-02) entire text | 1-16 |
| A | WO 2005/028426 A1 (CHUGAI PHARMACEUTICAL CO LTD) 31 March 2005 (2005-03-31) entire text | 1-16 |
| P, A | WO 2021/149776 A1 (CHUGAI PHARMACEUTICAL CO LTD) 29 July 2021 (2021-07-29) entire text | 1-16 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 August 2022** | **30 August 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/028187**

| Patent document cited in search report | Publication date (day/month/year) | Patent family member(s) | Publication date (day/month/year) |
|---|---|---|---|
| JP 2016-034900 A | 17 March 2016 | WO 2014/081024 A1<br>entire text | |
| WO 2006/011466 A1 | 02 February 2006 | US 2009/0233915 A1<br>entire text<br>US 2010/0197676 A1<br>EP 1780197 A1<br>CN 101124199 A<br>KR 10-2007-0041752 A | |
| WO 2005/028426 A1 | 31 March 2005 | US 2007/0105859 A1<br>entire text<br>EP 1674452 A1<br>TW 200520745 A | |
| WO 2021/149776 A1 | 29 July 2021 | JP 6971432 B1<br>entire text | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006011466 A
- WO 2007091736 A


### Non-patent literature cited in the description


- *Nat. Rev. Clin. Oncol.,* 2018, vol. 15, 709-720
- *Molecules.,* 2017, vol. 22, e1551
- *N. Engl. J. Med.,* 2012, vol. 367, 107-114
- *N. Engl. J. Med.,* 2012, vol. 367, 1694-1703
- *JAMA.,* 2017, vol. 317 (18), 1844-1853
- *Ann. Oncol.,* 2015, vol. 26 (5), 894-901
- *Cancer Res.,* 2013, vol. 73 (13), 4050-4060
- *Cancer Cell.,* 2014, vol. 25 (5), 697-710
- *J. Clin. Oncol.,* 2017, vol. 35 (15
- *Nat. Rev. Clin. Oncol.,* 2014, vol. 11, 385-400
- *Bioorg. Med. Chem. Lett.,* 2008, vol. 18 (24), 6501-6504
- *Bioorg. Med. Chem. Lett.,* 2013, vol. 23 (8), 2384-2390
- *Chem. Med. Chem.,* 2015, vol. 10 (12), 2004-2013
- *ACS Medchem. Lett.,* 2014, vol. 5 (4), 309-314